# EUROPEAN PATENT SPECIFICATION

(11) **EP 0 600 950 B1**
(45) Date of publication and mention of the grant of the patent: **23.10.1996**
(21) Application number: 92917230.2
(22) Date of filing: 03.08.1992
(51) Int. Cl.: C07D 257/04, C07D 403/12, C07D 471/04, C07D 401/12, C07D 401/14, A61K 31/41, A61K 31/435, A61K 31/495

(54) **AMIDE TETRAZOLE ACAT INHIBITORS**
TETRAZOLAMIDE ALS ACAT INHIBITOREN
INHIBITEURS D'ACAT A BASE DE TETRAZOLE D'AMIDE

(30) Priority: 22.08.1991 US 748568; 20.07.1992 US 913643
(43) Date of publication of application: 15.06.1994
(73) Proprietor: WARNER-LAMBERT COMPANY, Ann Arbor, Michigan 48105 (US)
(72) Inventor: O'BRIEN, Patrick, Michael, Stockbridge, MI 49285 (US); PICARD, Joseph, Armand, Ypsilanti, MI 48197 (US); PURCHASE, Claude Forsey Jr., Ann Arbor, MI 48105 (US); ROTH, Bruce, David, Ann Arbor, MI 48108 (US); SLISKOVIC, Drago, Robert, Ypsilanti, MI 48197 (US); WHITE, Andrew, David, Lakeland, MI 48143 (US)
(74) Representative: Mansmann, Ivo
(86) International application number: US9206388
(87) International publication number: WO9304052

(56) References cited:
- EP-A- 0 035 046
- WO-A-91/17150

## Description

The present invention describes a series of novel amide tetrazoles which inhibit acyl-CoA: cholesterol acyltransferase (ACAT), the enzyme responsible for the esterification of dietary cholesterol. Such agents may decrease the absorption of dietary cholesterol and therefore provide a therapy for individuals with hypercholesterolemia.

### SUMMARY OF THE INVENTION

The compounds of the present invention can be described by the following general formula 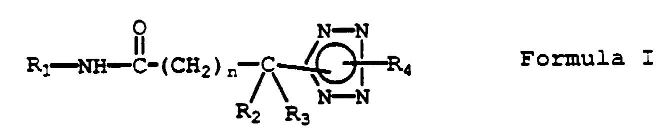 wherein n is zero, one or two;
wherein R₁ is selected from
(a) phenyl which is unsubstituted or is substituted with from one to three substituents selected from:
   alkyl having from 1 to 4 carbon atoms and which is straight or branched,
   alkoxy having from 1 to 3 carbon atoms and which is straight or branched,
   alkylthio having from 1 to 3 carbon atoms and which is straight or branched,
   phenyl,
   hydroxy,
   fluorine,
   chlorine,
   bromine,
   nitro,
   cyano,
   trifluoromethyl,
   -COOH,
   -COOalkyl wherein alkyl has from 1 to 4 carbon atoms and which is straight or branched,
   -(CH₂)ₘNR₅R₆ wherein m is zero or one, and each of R₅ and R₆ is hydrogen or a straight or branched alkyl group having 1 to 4 carbon atoms;
(b) 1- or 2-naphthyl which is unsubstituted or substituted with one to three substituents selected from:
   alkyl having from 1 to 4 carbon atoms and which is straight or branched,
   alkoxy having from 1 to 3 carbon atoms and which is straight or branched,
   hydroxy,
   fluorine,
   chlorine,
   bromine,
   nitro,
   cyano,
   trifluoromethyl,
   -COOH,
   -COOalkyl wherein alkyl has from 1 to 4 carbon atoms and is straight or branched,
   -(CH₂)ₘNR₅R₆ wherein m, R₅, and R₆ have the meanings defined above;
(c) the group 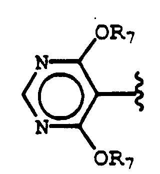 wherein R₇ is a lower alkyl group having from 1 to 3 carbon atoms and is straight or branched;
(d) the group 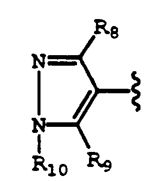 wherein R₈ and R₉ are straight or branched alkyl having from 1 to 4 carbon atoms or phenyl, and R₁₀ is a straight or branched hydrocarbon group having from 1 to 18 carbon atoms which is saturated or is unsaturated containing one double bond or two nonadjacent double bonds; phenyl; phenyl substituted with from one to three substituents selected from straight or branched alkyl having 1 to 4 carbon atoms, straight or branched alkoxy having from 1 to 3 carbon atoms, hydroxy, fluorine, chlorine, bromine, nitro, cyano, trifluoromethyl, -COOH, -COOalkyl wherein alkyl has from 1 to 4 carbon atoms and is straight or branched or (CH₂)ₘNR₅R₆ wherein m, R₅, and R₆ are as defined above; or a heterocyclic group selected from 2-, 3-, or 4-pyridyl, 2-, 4-, or 5-pyrimidinyl, 2- or 3-pyrazinyl, 2-, 3-, 4-, 5-, 6-, 7-, or 8-quinolinyl, or 3- or 4-pyridazinyl and the N-oxides thereof;
(e) the group 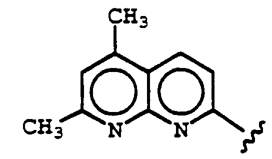
(f) the group 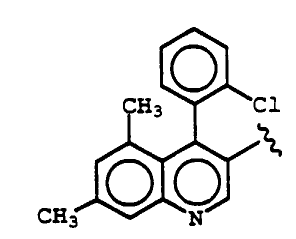
(g) a straight or branched hydrocarbon group having from 1 to 18 carbon atoms which is saturated or is unsaturated containing one double bond or two nonadjacent double bonds;
(h) a cycloalkyl group having from 3 to 8 carbon atoms;
(i) a heteroaromatic group selected from 2-, 3-, or 4-pyridyl which is unsubstituted or substituted with an alkyl group having from 1 to 4 carbon atoms or 2-, 4-, or 5-pyrimidinyl, and the N-oxides thereof;
(j) the group 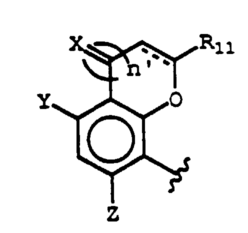 wherein --- denotes a single or double bond; Y and Z are each independently hydrogen, a straight or branched alkyl group of 1 to 4 carbon atoms, an alkoxy group of 1 to 3 carbon atoms, or halo; X is oxygen or two hydrogen atoms; R₁₁ is hydrogen or a straight or branched alkyl group of 1 to 4 carbon atoms, and n' is zero or one; or
(k) is selected from the group 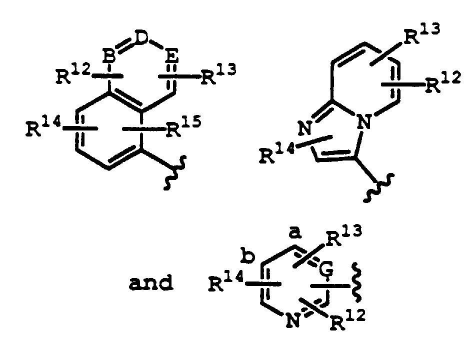 wherein R¹², R¹³, R¹⁴, and R¹⁵ are each independently hydrogen, halo, a straight or branched alkyl group of 1 to 4 carbon atoms, an alkoxy group of 1 to 3 carbon atoms, an alkylthio group of 1 to 3 carbon atoms, cycloalkylthio of 5 to 7 carbon atoms, phenylalkylthio in which alkyl is 1 to 4 carbon atoms, substituted phenylthio, heteroarylthio, or heteroaryloxy; and B, D, E, and G are nitrogen or carbon where one or more of B, D, and E is nitrogen; with the proviso that when G = N the group is attached to the nitrogen atom of Formula I at the 4 or 5 position of the pyrimidine ring (a and b),
wherein R₂ and R₃ are the same or different and are selected from:
(a) hydrogen, halo, or one of R₂ or R₃ is hydroxy;
(b) a straight or branched alkyl group having from 1 to 12 carbon atoms, or a cycloalkyl group having from 3 to 8 carbon atoms;
(c) a phenyl or phenylalkyl group where alkyl is from 1 to 4 carbon atoms and which the phenyl ring is unsubstituted or substituted with from 1 to 3 substituents selected from straight or branched alkyl having from 1 to 4 carbon atoms, straight or branched alkoxy having from 1 to 4 carbon atoms, alkythio (straight or branched) having 1 to 4 carbon atoms, hydroxy, fluorine, chlorine, bromine, trifluoromethyl, cyano, nitro, phenyl, or (CH₂)ₘNR₅R₆ wherein m, R₅, and R₆ have the meanings defined above;
(d) a straight or branched alkenyl group having from 2 to 6 carbon atoms; or
(e) R₂ and R₃ taken together with the carbon atom to which they are attached form an alkylidene group of 1 to 4 carbon atoms, a benzylidene group or a spiroalkyl group having from 3 to 7 carbon atoms;
(f) when R₂ is hydrogen, F, alkyl of C₁₋₁₂ atoms, R₃ can be heteroaryl selected from a 5- or 6-membered monocyclic or fused bicyclic heterocyclic group containing at least 1 to 4 heteroatoms in at least one ring, said heteroatoms being nitrogen, oxygen, or sulfur and combinations thereof, said heterocyclic group being unsubstituted or substituted with an alkyl group having from 1 to 4 carbon atoms and the N-oxides thereof; or
(g) 1- or 2-naphthyl which is unsubstituted or substituted with one to three substituents selected from:
   alkyl having from 1 to 4 carbon atoms and which is straight or branched, and
   alkoxy having from 1 to 3 carbon atoms and which is straight or branched,
   wherein R₄ is a straight or branched hydrocarbon chain having from 1 to 20 carbon atoms and is saturated or is unsaturated and has 1 double bond or has 2 nonadjacent double bonds; or is alkylthio having 1 to 20 carbon atoms and is saturated; pharmaceutically acceptable salts and individual enantiomeric isomers of the compounds.

### DETAILED DESCRIPTION

Pharmaceutically acceptable salts of the compounds of Formula I are also included as a part of the present invention. Suitable acids for forming acid salts of the compounds of Formula I containing a basic group include, but are not necessarily limited to acetic, benzoic, benzenesulfonic, hydrobromic, hydrochloric, citric, fumaric, gluconic, glucuronic, glutamic, lactic, malic, maleic, methanesulfonic, pamoic, salicylic, stearic, succinic, sulfuric, and tartaric acids. The acid addition salts are formed by procedures well known in the art.

Certain compounds of the present invention may also exist in different stereoisomeric forms by virtue of the presence of asymmetric centers in the compound. The present invention contemplates all stereoisomers may be obtained, if desired, by methods known in the art as, for example, the separation of stereoisomers by chiral chromatographic columns.

Further, the compounds of this invention may exist in unsolvated as well as solvated forms with pharmaceutically acceptable solvents such as water, ethanol, and the like. In general, the solvated forms are considered equivalent to the unsolvated forms for the purposes of this invention.

Illustrative examples of straight or branched saturated hydrocarbon chains having from 1 to 20 carbon atoms include methyl, ethyl, n-propyl, isopropyl, n-butyl, iso-butyl, tert-butyl, n-pentyl, isopentyl, n-hexyl, n-heptyl, n-octyl, n-undecyl, n-dodecyl, n-hexadecyl, 2,2-dimethyldodecyl, 2-tetradecyl, and n-octadecyl groups.

Illustrative examples of straight or branched hydrocarbon chains having from 1 to 20 carbon atoms and having 1 double bond or 2-nonadjacent double bonds include ethenyl, 2-propenyl, 2-butenyl, 3-pentenyl, 2-octenyl, 5-nonenyl, 4-undecenyl, 5-heptadecenyl, 3-octadecenyl, 9-octadecenyl, 2,2-dimethyl-11-eicosenyl, 9,12-octadecadienyl, and hexadecenyl.

Straight or branched alkoxy groups having 1 to 3 carbon atoms include methoxy, ethoxy, n-propoxy, and isopropoxy.

Straight or branched alkyl groups having from 1 to 4 carbon atoms include, for example, methyl, ethyl, n-propyl, isopropyl, and n-butyl.

Cycloalkyl groups having from 3 to 8 carbon atoms which R₁ may represent are cyclopropyl, cyclobutyl, cyclopentyl, cyclohexyl, cycloheptyl, and cyclooctyl.

Halo is fluoro, chloro, bromo, or iodo, but preferably fluoro.

A 5- or 6-membered monocyclic or fused bicyclic heterocycle is a monocyclic or fused bicyclic aromatic ring containing at least one to four heteroatoms in at least one ring, such as nitrogen, oxygen, or sulfur or a combination thereof. Such a heterocyclic group includes, for example, thienyl, benzothienyl, furanyl, benzofuranyl, pyridyl, pyrimidinyl, pyridazinyl, pyrazinyl, pyrrolyl, pyrazolyl, isothiazolyl, thiazolyl, oxazolyl, isoxazolyl, triazolyl, tetrazolyl, imidazolyl, benzothiazolyl, indolyl, quinolinyl, isoquinolinyl, or N-oxides of heterocycles containing a nitrogen atom.

More specifically, such a heterocycle may be a 2- or 3-thienyl; 2-, or 3-furanyl; 2-, or 3-, or 4-pyridyl or 2-, or 3-, or 4-pyridinyl-N-oxide; 2-, 4-, or 5-pyrimidinyl; 3- or 4-pyridazinyl; 2-pyrazinyl; 2-pyrazinyl-N-oxide; 2- or 3-pyrrolyl; 3-, 4-, or 5-pyrazolyl; 2-, 4-, or 5-thiazolyl; 3-, 4-, or 5-isoxazolyl; 2-, 4-, or 5-oxazolyl; 3-, 4-, or 5-isothiazolyl; 5-tetrazolyl; 3- or 5-(1,2,4)-triazolyl; 4- or 5-(1,2,3)-triazolyl; 2-, 4-, or 5-imidazolyl; 2-, 3-, 4-, 5-, 6-, or 7-indolyl; 2-, 3-, 4-, 5-, 6-, 7-, or 8-quinolinyl; 1-, 3-, 4-, 5-, 6-, 7-, or 8-isoquinolinyl; 2-, 4-, 5-, 6-, or 7-benzothiazolyl; or 2-, 3-, 4-, 5-, 6-, or 7-benzothienyl.

Preferred compounds of this invention are those wherein the R₄ substituent group is attached to the 2-position of the tetrazole moiety and the side chain or remainder of the molecule is attached to the carbon atom of the tetrazole moiety, the 5-position. Compounds wherein n is zero or one are also preferred with compounds wherein n is zero being more preferred. Compounds wherein R₁ is other than naphthyl or substituted naphthyl are also preferred. Compounds wherein n is zero, R₁ is substituted phenyl, and R₄ is in the 2-position and has from 8 to 18 carbon atoms are most preferred.

Most preferred are compounds of Formula I wherein R₁ is 2,6-(1-methylethyl)phenyl or 2,4,6-trimethoxyphenyl; n is zero; R₂ and R₃ are each independently hydrogen, methyl, fluoro, cyclohexyl, or phenyl, and R₄ is in the 2-position and has 12 carbon atoms.

As shown by the data presented below in Table 1, the compounds of the present invention are potent inhibitors of the enzyme acyl-CoA: cholesterol acyltransferase (ACAT), and are thus effective in inhibiting the esterification and transport of cholesterol across the intestinal cell wall. The compounds of the present invention are thus useful in pharmaceutical formulations for the treatment of hypercholesterolemia or atherosclerosis.

The ability of representative compounds of the present invention to inhibit ACAT was measured using an in vitro test more fully described in F. J. Field and R. G. Salone, Biochemica et Biophysica 712:557-570 (1982). The test assesses the ability of a test compound to inhibit the acylation of cholesterol by oleic acid by measuring the amount of radiolabeled cholesterol oleate formed from radiolabeled oleic acid in a tissue preparation containing rabbit intestinal microsomes (designated IAI) or from rat liver microsomes (designated LAI).

These data appear in Tables 1 and 3 where they are expressed as IC₅₀ values; i.e., the concentration of test compound required to inhibit the activity of the enzyme by 50%.

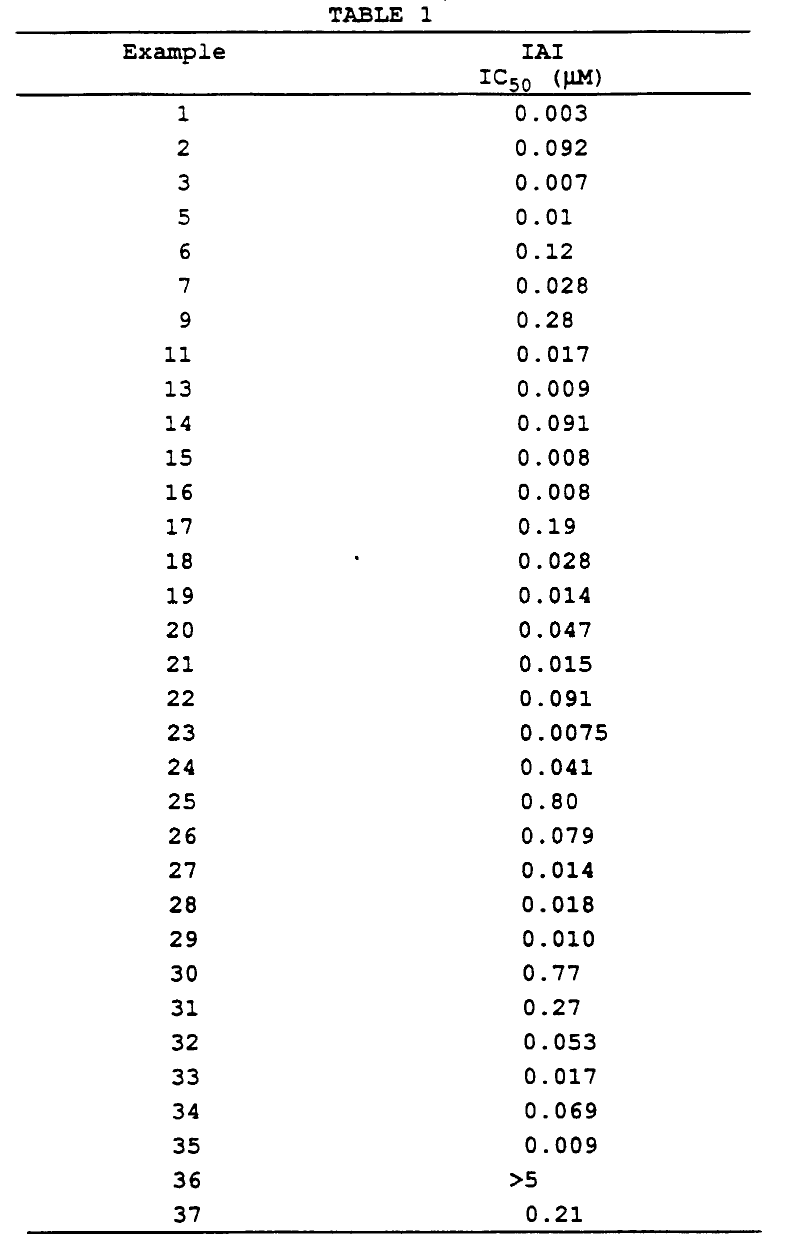

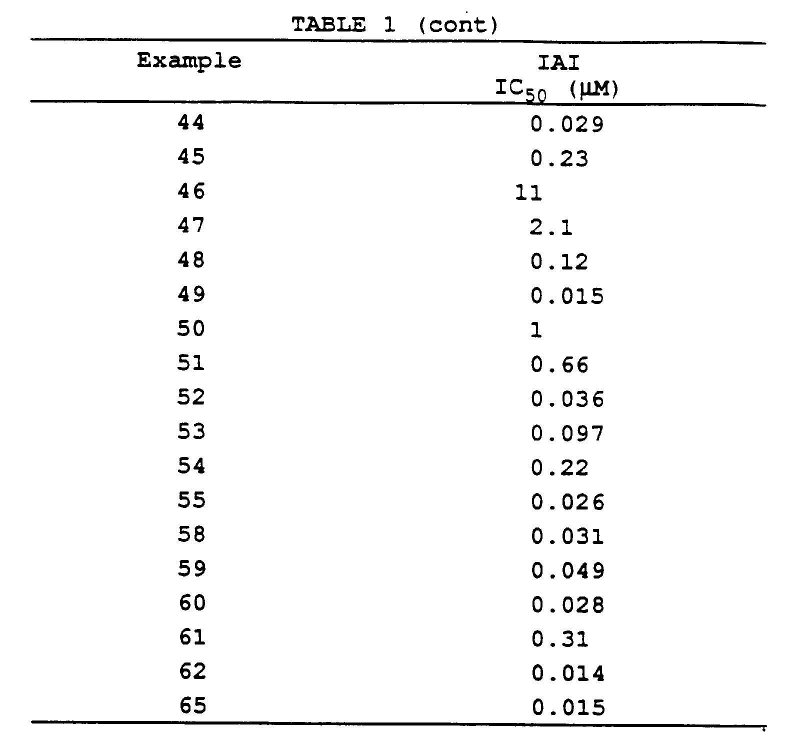

In one in vivo screen designated APCC, male Sprague-Dawley rats (200 to 225 g) were randomly divided into treatment groups and dosed at 4 PM with either vehicle (CMC/Tween) or suspensions of compounds in vehicle. The normal chow diet was then replaced with a high fat, high cholesterol diet with 0.5% cholic acid. The rats consumed this diet ad libitum during the night and were sacrificed at 8 AM to obtain blood samples for cholesterol analysis using standard procedures. Statistical differences between mean cholesterol values for the same vehicle were determined using analysis of variance followed by Fisher's least significant test. The results of this trial for representative compounds of the present invention appear in Table 2. The compounds were dosed at 30 mg/kg unless otherwise noted.

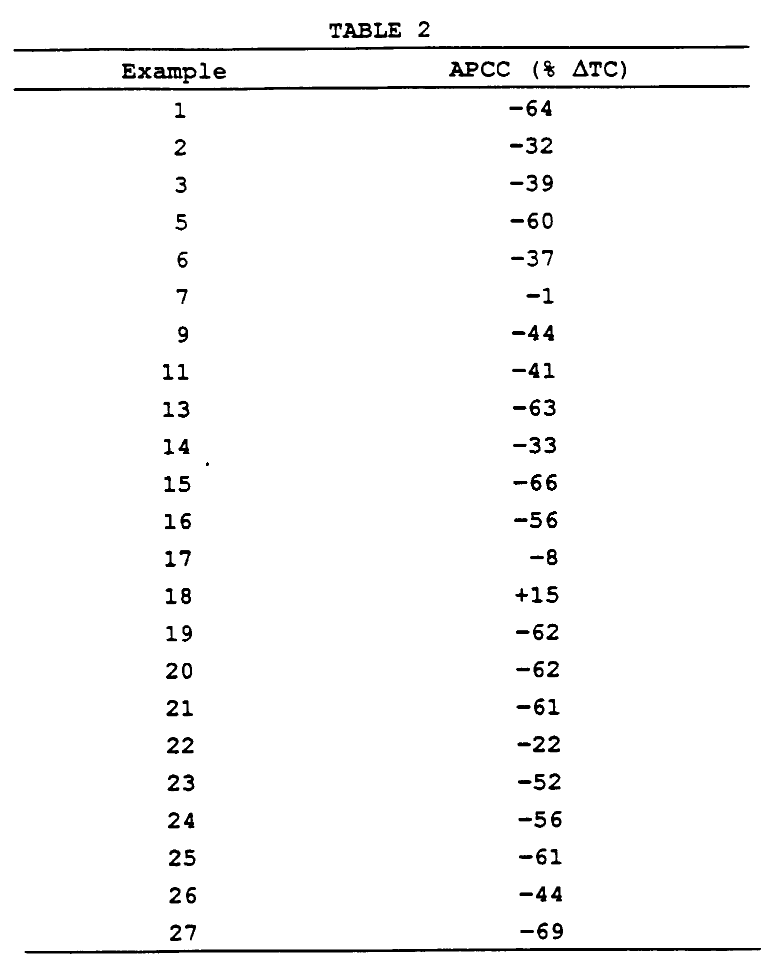

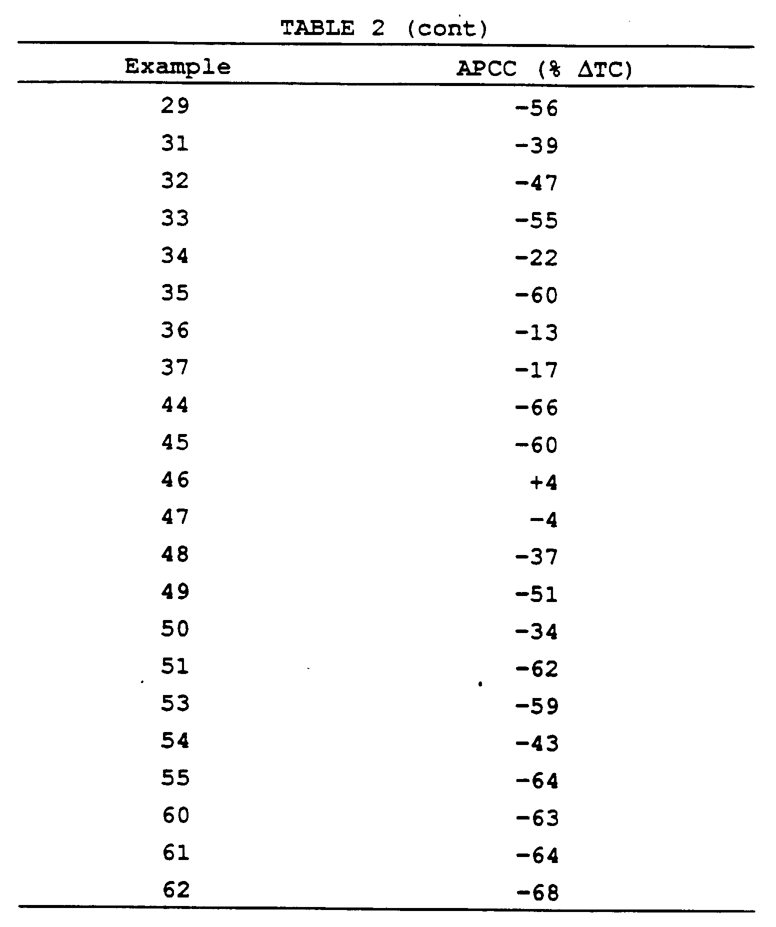

Compounds of Formula I where the side chain is attached directly to a nitrogen atom were also active in the above described tests and the results are shown in Table 3.

**TABLE 3**

| Example | LAI (IC₅₀) (µM) | APCC (% ΔTC) |
|---|---|---|
| 88 | 0.010 | -62 |
| 89 | 0.390 | -35 |
| 90 | 0.10 | +5 |
| 91 | 0.006 | -68 |
| 92 | 0.015 | -77 |
| 93 | 0.022 | -30 |
| 94 | 0.029 | -26 |
| 95 | 0.058 | -64 |
| 96 | 0.19 | -47 |
| 97 | 0.056 | -69 |
| 98 | 0.021 | -65 |
| 99 | 0.032 | -51 |
| 100 | 0.080 | -63 |
| 101 | >5.0 | +8 |
| 102 | 0.042 | -47 |
| 103 | 0.049 | -60 |
| 104 | 0.055 | -50 |

In therapeutic use as agents for treating hypercholesterolemia or atherosclerosis, the compounds of Formula I or pharmaceutically acceptable salts thereof are administered to the patient at dosage levels of from 250 to 3000 mg per day. For a normal human adult of approximately 70 kg of body weight, this translates into a dosage of from 5 to 40 mg/kg of body weight per day. The specific dosages employed, however, may be varied depending upon the requirements of the patient, the severity of the condition being treated, and the activity of the compound being employed. The determination of optimum dosages for a particular situation is within the skill of the art.

For preparing the pharmaceutical compositions from the compounds of this invention, inert, pharmaceutically acceptable carriers can be either solid or liquid. Solid form preparations include powders, tablets, dispersible granules, capsules, and cachets.

A solid carrier can be one or more substances which may also act as diluents, flavoring agents, solubilizers, lubricants, suspending agents, binders, or tablet disintegrating agents; it can also be an encapsulating material.

In powders, the carrier is a finely divided solid which is in a mixture with the finely divided active component. In tablets, the active component is mixed with the carrier having the necessary binding properties in suitable proportions and compacted in the shape and size desired.

Powders and tablets preferably contain between about 5% to about 70% by weight of the active ingredient. Suitable carriers are magnesium dicarbonate, magnesium stearate, talc, lactose, sugar, pectin, dextrin, starch, tragacanth, methyl cellulose, sodium carboxymethyl cellulose, a low-melting wax, cocoa butter, and the like.

The term "preparation" is intended to include the formulation of the active compound with encapsulating material as a carrier providing a capsule in which the active component (with or without carriers) is surrounded by a carrier, which is thus in association with it. In a similar manner cachets are also included.

Tablets, powders, cachets, and capsules can be used as solid dosage forms suitable for oral administration.

Liquid form preparations include solutions, suspensions, or emulsions suitable for oral administration. Aqueous solutions for oral administration can be prepared by dissolving the active compound in water and adding suitable flavorants, coloring agents, stabilizers, and thickening agents as desired. Aqueous suspensions for oral use can be made by dispersing the finely divided active component in water together with a viscous material such as natural or synthetic gums, resins, methyl cellulose, sodium carboxymethylcellulose, and other suspending agents known to the pharmaceutical formulation art.

Preferably, the pharmaceutical preparation is in unit dosage form. In such form, the preparation is divided into unit doses containing appropriate quantities of the active component. The unit dosage form can be a packaged preparation containing discrete quantities of the preparation, for example, packeted tablets, capsules, and powders in vials or ampoules. The unit dosage form can also be a capsule, cachet, or tablet itself, or it can be the appropriate number of these packaged forms.

The compounds of the present invention can be prepared by various routes all of which are generally known in the art. The compounds of Formula I wherein n is zero, each of R₂ and R₃ is hydrogen and R₁ and R₄ are as defined in Formula I can be prepared as set forth in Chart I hereof.

In Chart I, the tetrazole ester (2) is synthesized via treatment of ethyl cyanoacetate (1) with sodium azide. Alkylation of the tetrazole ester (2) with a halide of the formula R₄ halo (3) wherein R₄ has the meaning defined in Formula I and halo is, e.g., bromine or chlorine, provides a mixture of (4) and (7), i.e., the 2- and 1-regioisomers, respectively, isomers which are separable by chromatography. Esters (4) and (7) can then be independently hydrolyzed to the acids (5) and (8) which are coupled with an amine of the formula R₁NH₂ wherein R₁ has the meaning defined in Formula I using carbonyldiimidazole in THF to give the 2 and 1 substituted tetrazole amides (6) and (9), respectively.

Compounds of Formula I wherein n is zero and R₁, R₂, R₃, and R₄ are as defined in Formula I except that both R₂ and R₃ are not hydrogen and R₃ is other than heteroaryl or naphthyl are best synthesized employing the synthetic sequence presented in Chart II. In Chart II the ethyl cyanoacetate derivatives (11) are treated with tri-n-butyltin azide in dioxane at reflux to give compound (12) after acidic hydrolysis with HCl in ether or tetrahydrofuran. The tetrazole is then alkylated with a primary alkyl halide in acetonitrile at reflux using a base such as triethylamine or pyridine. The resulting 2- and 1-regioisomers compounds (13) and (14)] are separated by chromatography. Compound (13) is easily hydrolyzed to carboxylic acid (15) when treated with NaOH or KOH in an alcoholic solvent such as methanol or ethanol at room temperature. However, when R₂ is hydrogen and R₃ is alkyl, aryl, or alkenyl, regioisomer (14) decarboxylates to (17) when subjected to the previously described hydrolytic conditions. The desired acid (19) is obtained under these conditions, however, when R₂ = R₃ = H or R₂ and R₃ is alkyl, alkenyl, aryl, or spirocycloalkyl. The carboxylic acids (15, 19) are easily converted to the corresponding amides (16, 18) when treated with a coupling agent such as carbonyldiimidazole or dicyclohexylcarbodiimide in tetrahydrofuran or dichloromethane and an appropriate amine. Alternatively, regioisomer (18) is prepared by treating (17) with n-butyllithium in tetrahydrofuran at -20°C followed by the addition of an appropriate isocyanate.

Also when R₂ = H in Compound 15 (Chart II(a)), Compound 15 may be deprotonated using n-BuLi in THF at -78°C to give an anion which can then be treated with an electrophilic reagent (R₂X) to give the α,α'-disubstituted acid shown which can then be coupled with an appropriate amine (R₁NH₂) in a manner as previously described to yield the corresponding amides. Also in Compound 13 (Chart II) when R₂ = H, R₃ as defined in Formula I, this ester can also be deprotonated and the anion fluorinated using N-fluorobenzenesulfonimide to yield the α-fluoro ester which is then used as described in the text for Compound 13.

Compounds of formula (11) are either commercially available or can be synthesized employing the following conditions:

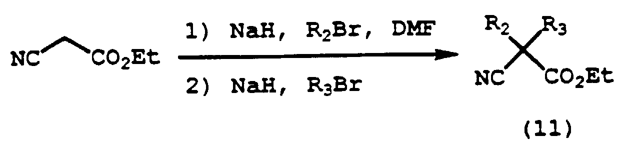

Ethyl cyanoacetate is treated with one equivalent of sodium hydride in dimethylformamide or tetrahydrofuran followed by the addition of an appropriate alkylating agent such as 1-bromopropane or benzyl bromide to give monoalkylated analogs of Compound 11. Similarly, a second equivalent of base may then be added followed by the addition of an appropriate alkylating agent to give disubstituted ethyl cyanoacetates of formula (11). The compounds of Formula I wherein n is zero, R₂ is hydrogen, R₃ is heteroaryl, 1- or 2-naphthyl, substituted phenyl, and R₁ and R₄ are as defined in Formula I are prepared as shown in Chart VI hereof wherein the reaction conditions are set forth. Specific Example 38 is illustrative of this synthetic route. The acetonitriles, R₃CH₂CN, are known in the art or are prepared from the alcohol, R₃CH₂OH, by procedures generally known in the art, e.g., J. Am. Chem. Soc. (71):3994, 1949. Spirocycloalkyl analogues are synthesized in a similar manner by employing dihalo alkyl halides of the formula halo-(CH₂)ₚ-halo wherein p is two to six and halo is chlorine or bromine as the alkylating agent. An illustrative alkylating agent is 1,4-dibromobutane. Compounds of Formula I wherein n = zero, R₂, R₃ = alkyl, aryl, R₁, R₄ as defined in Formula I can also be synthesized as shown in Chart XI. The commercially available acetonitriles are treated with tri-n-butyltin azide in dioxane at reflux to give the corresponding tetrazole which is then alkylated with a primary alkyl halide in acetonitrile at reflux using a base such as TEA or pyridine. The resulting 1- and 2-regioisomers are separated by chromatography. Treatment of these compounds with n-butyllithium in tetrahydrofuran at -78°C followed by the addition of an appropriate isocyanate (R₁NCO) gives the desired amides. Specific Example 46 is illustrative of this synthetic route.

Additionally compounds of Formula I wherein n = zero, R₂, and/or R₃ is F or OH, R₁, R₄ as defined in Formula I can be synthesized as shown in Chart XII. The alkylated tetrazole is treated with n-BuLi and TMEDA in THF at -78°C followed by ethyl phenyl glyoxylate. The resulting hydroxy compound was then treated with diethyl amino sulfur trifluoride (DAST) in dichloromethane at -78°C under N₂. The resulting fluoro ester was then hydrolyzed using NaOH in methanol/water. The resulting acid was converted to the acid chloride via treatment with oxalyl chloride in dichloromethane at room temperature. The crude acid chloride was treated with an appropriate amine in dichloromethane with Et₃N as base at 0°C to yield the desired amide. Specific Example 65 is illustrative of this synthetic route. Also the hydroxyester may be treated with t-butyldimethyl silyl trifluoromethane sulfonate in dichloromethane with Et₃N as base to yield the protected hydroxy ester, which can then be converted to the desired amide as shown in the scheme.

The compounds of Formula I wherein n is one or two, R₂ and R₃ are hydrogen and R₁ and R₄ are as defined in Formula I are prepared as set forth in Chart III hereof. In Chart III an appropriate nitrile ester (20) is heated with an alkali metal azide, such as LiN₃ or NaN₃, and NH₄Cl in dimethylformamide at temperatures ranging from 50° to 80°C to give after work-up the corresponding tetrazole ester (20-A). The tetrazole ester (20-A) is heated, typically at temperatures between 50° and 100°C, with a tertiary amine such as triethylamine, and an appropriate alkyl halide, including alkyl bromides, chlorides, and iodides, or an arylalkyl halide in a polar solvent, such as CH₃CN, to give after work-up and chromatographic separation both of the corresponding regioisomeric 1-alkylated and 2-alkylated tetrazole esters (22) and (21). The alkyl tetrazole esters (21 and 22) are stirred, typically at temperatures between 0° and 30°C, with alkali metal hydroxides, such as LiOH, NaOH, or KOH, in an alcoholic solvent such as methanol or ethanol for 1 to 24 hours to give after work-up the corresponding alkyltetrazole carboxylic acids (23 and 24). The alkyltetrazole carboxylic acids are coupled with primary amines, especially aryl amines of the formula R₁NH₂ wherein R₁ is as defined in Formula I such as 2,4,6-trimethoxyaniline, 2,6-diisopropylaniline, and 2,4-difluoroaniline, using a carboxylic acid activating reagent such as carbonyldiimidazole or dicyclohexylcarbodiimide in an aprotic solvent such as THF or CH₂Cl₂, at temperatures between -10° and +110°C to give after work-up the corresponding alkyltetrazole amides (25 and 26).

The compounds of general Formula I wherein n is one, R₂ is hydrogen, R₃ is phenyl, substituted phenyl, heteroaryl, alkyl, or alkenyl and R₁ and R₄ are as defined in Formula I are prepared as set forth in Chart IV. In Chart IV the group R₃(X) is phenyl, substituted phenyl or heteroaryl as defined in formula I or R₃(X) is a straight or branched alkyl having from 1 to 6 carbon atoms or a straight or branched alkenyl having from 2 to 6 carbon atoms. The β-substituted cyanopropionic acid compound (27) is prepared from the corresponding aldehyde of the formula XCHO using the procedure described in US 4,760,089. Compound (27) is treated with an appropriate amine, R₁NH₂ wherein R₁ has the meaning defined in general Formula I employing a coupling agent such as carbonyldiimidazole in tetrahydrofuran at room temperature or dicylohexylcarbodiimide in dichloromethane at 0°C to give the nitrile amide (28). The nitrile amide (28) is converted to the tetrazole (29) by treatment with (n-Bu)₃SnN₃ in refluxing dioxane and then is alkylated with an appropriate compound of the formula R₄halo wherein R₄ has the meaning defined in Formula I and halo is chlorine, or bromine employing triethylamine in acetonitrile. The products (30) and (31) are separated by chromatography. Specific Example 45 is illustrative of this synthetic route.

The compounds of Formula I wherein n is two, R₂ is hydrogen, and R₃ is phenyl or substituted phenyl are prepared as set forth in Chart V. Compound (32) is prepared according to the method of Paganelli (Tett. Lett. 32:2807-2810, 1991) by a transition metal catalyzed Michael addition of benzyl cyanide to methyl acrylate. Compound (32) is then treated with (n-Bu)₃SnN₃ in refluxing dioxane to give the tetrazole (33), which is then alkylated with an alkyl halide, R₄halo, e.g., R₄Br, wherein R₄ is as defined in Formula I, in acetonitrile employing Et₃N as base, giving a mixture of regioisomers (34) and (35) which are separated by flash chromatography. Hydrolysis of each ester with ethanolic NaOH at room temperature gives the respective acids (36) and (38). The acids are then coupled with an appropriate amine of the formula R₁NH₂ wherein R₁ is as defined in Formula I employing carbonyldiimidazole in tetrahydrofuran at room temperature or dicyclohexylcarbodiimide in CH₂Cl₂ at 0°C as coupling agent to give the amides (37) and (39).

The compounds of Formula I wherein n is one, R₃ is other than heteroaryl and R₁, R₂, and R₄ are as defined in Formula I are prepared as set forth in Chart VII.

Ethyl cyanoacetate is alkylated (or dialkylated) by treatment with NaH in an appropriate solvent such as dimethylformamide or tetrahydrofuran at from 0° to 25°C to give the alkylated nitrile (40). The nitrile is then treated with (n-Bu)₃SnN₃ in dioxane at reflux for 24 hours to give after acidic hydrolysis the tetrazole (41) which is then alkylated with an alkyl halide (R₄Br) in CH₃CN employing Et₃N as base to give a mixture of regioisomers (42) and (43). The regioisomers are separated by flash chromatography and each ester is reduced by DIBAL-H in CH₂Cl₂ or toluene at -78°C to give the corresponding alcohols (44) and (45). The alcohols are treated with methanesulfonyl chloride in CH₂Cl₂ using triethylamine as a base at 0°C to give the corresponding mesylates which are then treated with KCN in dimethylformamide or dimethyl sulfoxide at 100°C to give the corresponding nitriles (46) and (47). These are then hydrolyzed to the corresponding acids (48) and (49) by treatment with ethanolic NaOH (or KOH) at reflux. The acids are then coupled with an appropriate amine employing carbonyldiimidazole in tetrahydrofuran at room temperature or dicyclohexylcarbodiimide in CH₂Cl₂ at 0°C to give the amides (50) and (51).

The compounds of Formula I wherein n is one, R₃ is heteroaryl and R₁ and R₄ are as defined in Formula I are prepared in the same manner as set forth in Chart VII beginning with compounds which are the same as compounds (42) and (43) except that R₂ is hydrogen and R₃ is heteroaryl. These comparable tetrazole intermediates are prepared as set forth in Chart VIII hereof wherein R₃ is heteroaryl and R₄ has the meaning defined in Formula I. The reaction conditions are set forth in Chart VIII.

The compounds of Formula I wherein n is two, R₂ and R₃ are as defined in Formula I only at least one is other than hydrogen, and R₁ and R₄ are as defined in Formula I are prepared as set forth in Chart IX.

Malonitrile is alkylated (or dialkylated) by treatment with NaH in an appropriate solvent such as dimethylformamide or tetrahydrofuran at 0° to 25°C to give compounds (51). Treatment of the substituted nitrile with (n-Bu)₃SnN₃ in refluxing dioxane for 24 hours gives, after acidic hydrolysis, the tetrazole (52), which is then alkylated with an alkyl halide (R₄Br) in CH₃CN employing Et₃N as base to give a mixture of regioisomers (53) and (54). The regioisomers are then separated by flash chromatography and each nitrile is then reduced to the corresponding aldehydes (55) and (56) by treatment with Raney nickel in formic acid at 60°C. The resulting aldehydes are then treated with a stabilized ylide such as ethyl(triphenylphosphoranylidene)acetate in CH₂Cl₂ at room temperature to give (57) and (58) which are reduced catalytically using hydrogen gas, Pd/C as catalyst in methanol or ethanol at room temperature to give esters (59) and (60). These are then hydrolyzed to the corresponding acids (61) and (62) by treatment with alcoholic (MeOH/EtOH) alkali metal hydroxide (NaOH or KOH) at reflux. The acids are then coupled with an appropriate amine employing carbonyldiimidazole in tetrahydrofuran at room temperature or dicyclohexylcarbodiimide in CH₂Cl₂ at 0°C to give amides (63) and (64).

The N-oxides of compounds of this invention are prepared by standard procedures known in the art, for example, by treatment with m-perchlorobenzoic acid at reflux in chloroform or dichloromethane.

The isocyanates, R₁NCO, and the amines R₁NH₂ wherein R₁ has the meaning defined in Formula I, employed in preparing the compounds of this invention are known in the art or can be prepared by procedures generally known in the art. For example, the pyrazole amines are prepared as set forth in Chart X hereof wherein the reaction conditions are indicated in the chart.

In addition, compounds of Formula I having an asymmetric carbon atom can be synthesized in either enantiomeric form (R₂ does not equal R₃) by treating compounds (15) or (19) in Chart II, (27) in Chart IV, (36) or (38) in Chart V, (48) or (49) in Chart VII, and (61) or (62) in Chart IX with appropriate chiral amines such as R-(+)- or S-(-)-α-methylbenzyl amine, (1S, 2R) ephedrine, or brucine. The salts are prepared by dissolving the racemic acid enumerated above in ethyl acetate or a mixture of hexane/ethyl acetate containing the appropriate chiral amine. The chiral salt is collected by filtration and recrystallized several times from hexane/ethyl acetate. The chiral acid is then liberated through an acidic workup and its enantiomeric purity is determined by chiral HPLC. The chiral acids are then coupled with appropriate amines to give enantiomerically pure compounds designated as (16), (18), (28), (37), (39), (50), (51), (63), and (64), respectively. Similarly, to obtain the chiral products of the compounds of formulas (67) and (68) in Chart VI the intermediates (65) and (66) are treated with n-BuLi and ethyl chloroformate as shown in Chart VIII and the resulting esters are hydrolyzed to obtain acids corresponding to (48) and (49) only wherein R₄ is a heteroaryl group. These acids are then treated with chiral amines as described above.

For compounds of Formula I where the side chain is attached on a nitrogen atom of the tetrazole ring (Chart XIII), a nitrile (R₄CN) is converted to the corresponding 5-substituted tetrazole by cycloaddition with an azide (ammonium azide, tributyltin azide, etc) in an inert solvent such as dimethylformamide. The resulting 5-substituted tetrazole can be alkylated with an α-bromo ester using a base such as triethylamine in a neutral solvent such as acetonitrile. The resulting mixture of 1,5 and 2,5 regioisomers is separated by chromatography or recrystallization. The esters of the pure regioisomers are then individually saponified using an inorganic base (NaOH, KOH, etc) and acidified with a mineral acid such as HCl to give the corresponding carboxylic acids. The carboxylic acids are coupled with various amines using standard coupling reagents (CDI, DCC, mixed anhydride, etc) to give the final products.

### EXAMPLE 1

### N-[2,6-Bis(1-methylethyl)phenyl]-2-dodecyl-2H-tetrazole-5-acetamide (R₁ = 2,6-diisopropylphenyl; n is zero; R₂ and R₃ are hydrogen; and R₄ is 2-(CH₂)₁₁CH₃.

### (a) Tetrazoleacetic acid ethyl ester

To a solution of ethylcyanoacetate (20.0 g, 0.177 mol) in dimethylformamide (DMF) (180 mL) was added NH₄Cl (10.4 g, 0.19 mol) and sodium azide (12.6 g, 0.19 mol) sequentially. The mixture was heated for 5 hours at 100°C, allowed to cool, and the DMF removed in vacuo. The residue was taken up in water (150 mL), acidified to pH 2 with concentrated HCl, and filtered. The filtrate was cooled to 5°C and allowed to crystallize. The solid was filtered, dried in vacuo over self-indicating silica gel to give 10.61 g, 42%, mp 124-129°C.

### (b) 1-Dodecyltetrazoleacetic acid ethyl ester and 2-Dodecyltetrazole acetic acid ethyl ester

1-Bromododecane (8.78 g, 0.035 mol) was added to a refluxing solution of the tetrazole acetic acid ethyl ester (5.0 g, 0.032 mol) obtained in (1a) above, and triethylamine (3.56 g, 0.035 mol) in acetonitrile (150 mL). The mixture was refluxed for 18 hours, allowed to cool, and filtered. The filtrate was concentrated in vacuo and partitioned between ethyl acetate (150 mL) and water (150 mL). The organic layer was washed with brine (100 mL) and dried over MgSO₄, then filtered, concentrated, and chromatographed on silica gel, eluting with 10%, then 20% ethyl acetate in hexanes to give 5.40 g, 52% of the 2-isomer (Rf 0.66, 50% ethyl acetate/hexane) as an oil and 3.39 g, 33% of the 1-isomer (Rf 0.50, 50% ethyl acetate/hexane) as a solid, mp 59-62°C.

### (c) 2-Dodecyltetrazoleacetic acid

A solution of KOH (4.21 g, 0.075 mol) in water (10 mL) was added to a solution of the 2-dodecyltetrazole acetic acid ethyl ester (23.2 g, 0.0715 mol) in ethanol (250 mL). The mixture was stirred at room temperature for 3 hours, concentrated in vacuo to ∼50 mL, diluted with water (200 mL), and washed with ethyl acetate (100 mL). The aqueous layer was acidified with 1.0 M HCl and extracted with ethyl acetate. The organic layer was dried over MgSO₄, filtered, and concentrated to give 18.0 g, 85% of a white solid, mp 70-73°C.

### (d) N-[2,6-Bis(1-methylethyl)phenyl]-2-dodecyl-2H-tetrazole-5-acetamide

Carbonyldiimidazole (5.74 g, 0.035 mol) was added to a solution of the 2-dodecyltetrazole acetic acid (10.0 g, 0.034 mol) obtained in (c) above in dry THF (100 mL) under an inert atmosphere (N₂). The mixture was stirred at room temperature for 30 minutes, then 2,6-diisopropylaniline (6.7 mL, 0.038 mol) was added in one portion. The resulting solution was stirred for 3 days at room temperature, concentrated in vacuo, taken up in dichloromethane (200 mL), washed with water (100 mL) and brine (100 mL), and dried over Na₂SO₄. The dried solution was filtered, concentrated, and chromatographed on silica gel, eluting with 15% ethyl acetate in hexanes to give 10.6 g, 68% of the title compound as an off-white solid, mp 75-79°C.

### EXAMPLE 2

### N-[2,6-Bis(1-methylethyl)phenyl]-1-dodecyl-1H-tetrazole-5-acetamide (R = 2,6-diisopropylphenyl; n is zero; R₂ and R₃ are hydrogen; and R₄ is 1-(CH₂)₁₁CH₃.

Following the procedure set forth in steps (c) and (d) of Example 1, only substituting 1-dodecyl-tetrazoleacetic acid ethyl ester for 2-dodecyl-tetrazoleacetic acid ethyl ester, the title compound was obtained, mp 88-91°C.

Following the general procedure of Examples 1 and 2 only substituting an appropriate amount of the amine listed below for 2,6-diisopropylaniline, the respective products listed below were obtained.

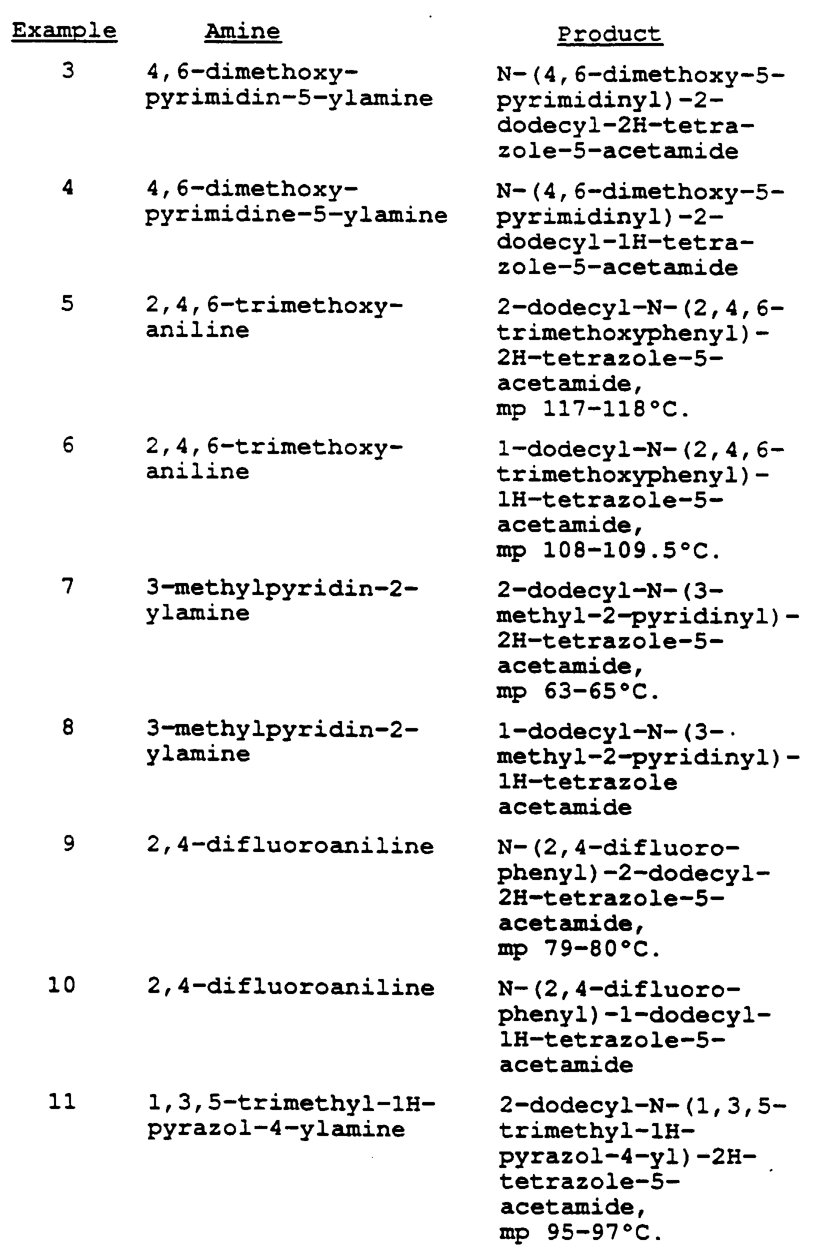

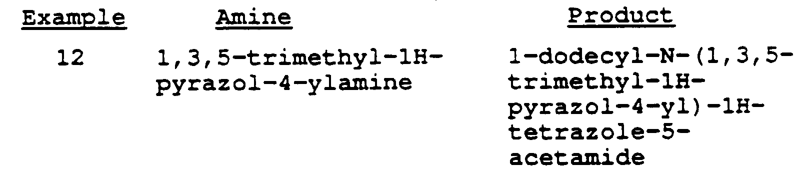

The compounds of Example 9 and 10 above were made as a mixture.

### EXAMPLE 13

### (±) 2-Dodecyl-α-phenyl-N-(2,4,6-trimethoxyphenyl)-2H-tetrazole-5-acetamide

### (a) (±) α-Phenyl tetrazole-5-acetic acid, ethyl ester

Ethyl phenylcyanoacetate (44.4 g; 0.23 moles) was dissolved in p-dioxane (900 mL) and treated with n-tributyltin azide (76.3 g; 0.23 moles) in one portion. The solution was heated to reflux for 16 hours, cooled to room temperature, and then concentrated in vacuo. The resulting liquid was dissolved in ethyl ether (500 mL) and treated with gaseous HCl for over 15 minutes. The ether was removed in vacuo leaving a viscous liquid which solidified when triturated with boiling hexanes. Yield: 47.29 (88%).
¹H NMR (DMSO-D₆) δ 7.3 (s, 5H), 5.7 (s, 1H), 4.2 (q, 2H), 1.1 (t, 3H) ppm.

### (b) (±) 2-Dodecyl-α-phenyl-2H-tetrazole-5-acetic acid, ethyl ester

The tetrazole ester (a) (47 g; 0.20 moles) was dissolved in acetonitrile (550 mL) containing one equivalent of triethylamine (20.2 g; 0.20 moles). The solution was heated to reflux and then 1-bromododecane (49.8 g; 0.20 moles) was added dropwise over 20 minutes. Upon completion, the solution was heated to reflux for 16 hours, cooled to room temperature, and concentrated in vacuo. The residue was triturated with ethyl acetate (1 L), filtered, and the filtrate was washed with aqueous HCl (1N), brine, and dried over magnesium sulfate. The drying agent was removed by filtration and the solvent concentrated in vacuo, leaving a viscous liquid containing both 1- and 2-isomers. The regioisomers were separated by silica gel chromatography using 75% hexane and 25% ethyl acetate as the eluent, obtaining the title compound as a colorless liquid. Yield: 33 g (41%).
¹H NMR (CDCl₃) δ 7.5 (d, 2H), 7.3 (m, 3H), 5.3 (s, 1H), 4.5 (t, 2H), 4.2 (m, 2H), 2.0 (m, 2H), 1.2 (s, 18H), 0.8 (t, 3H) ppm.

### (c) (±) 1-Dodecyl-α-phenyl-1H-tetrazole-5-acetic acid, ethyl ester

The 1-dodecyl compound was isolated from the silica gel column previously described in isolating compound (b) above. Yield: 14.3 g (18%).
¹H NMR (CDCl₃) δ 7.2-7.4 (m, 5H), 5.3 (s, 1H), 4.2 (q, 2H), 4.0 (t, 2H), 1.5 (m, 2H), 1.2 (s, 18H), 0.8 (t, 3H) ppm.

### (d) (±) 2-Dodecyl-α-phenyl-2H-tetrazole-5-acetic acid

Compound (c) (33.0 g; 0.082 moles) obtained above was dissolved in absolute ethanol (400 mL) and treated with sodium hydroxide pellets (6.5 g; 0.16 moles) in one portion. The solution was stirred for several hours at room temperature before concentrating the ethanol in vacuo, leaving a viscous syrup. The carboxylic acid sodium salt was dissolved in water (300 mL) and washed with one portion of ethyl ether (75 mL). The aqueous solution was then acidified to a pH of 1.0 with concentrated HCl, and the product was extracted with two portions of ethyl acetate. The combined organic solution was washed once with brine, dried over MgSO₄, and filtered. The filtrate was concentrated in vacuo, leaving a colorless liquid that solidified on standing, mp 55-57°C. Yield: 27.8 g (91%).
¹H NMR (DMSO-D₆) δ 7.4 (d, 2H), 7.3 (m, 3H), 5.4 (s, 1H), 4.6 (t, 2H), 1.8 (m, 2H), 1.2 (s, 18H), 0.8 (s, 3H) ppm.

### (e) (±) 2-Dodecyl-α-phenyl-N-(2,4,6-trimethoxyphenyl)-2H-tetrazole-5-acetamide

The compound obtained in (d) above (6.58 g; 17.6 mmoles) was dissolved in tetrahydrofuran (50 mL), treated with carbonyldiimidazole (3.1 g; 19.1 mmoles), and stirred for 1 hour at room temperature under an atmosphere of N₂. A solution of 2,4,6-trimethoxyaniline (3.2 g; 17.6 mmoles/50 mL THF) was then added in one portion and the solution was stirred at room temperature for overnight. Ethyl acetate (150 mL) was then added as well as aqueous HCl (1N). The layers were separated and the organic portion was washed with NaOH (1N), brine, and then dried over MgSO₄. The drying agent was filtered and the filtrate concentrated in vacuo leaving a lavender colored solid which was purified by silica gel chromatography using chloroform (95%)/methanol (5%) as the eluent. Yield: 6.7 g (70%), mp 119-120°C.

When in the procedure of Example 13(e) an appropriate amount of the amine listed below was substituted for 2,4,6-trimethoxyaniline and the general procedure of Example 13(e) was followed, the respective products listed below were obtained.

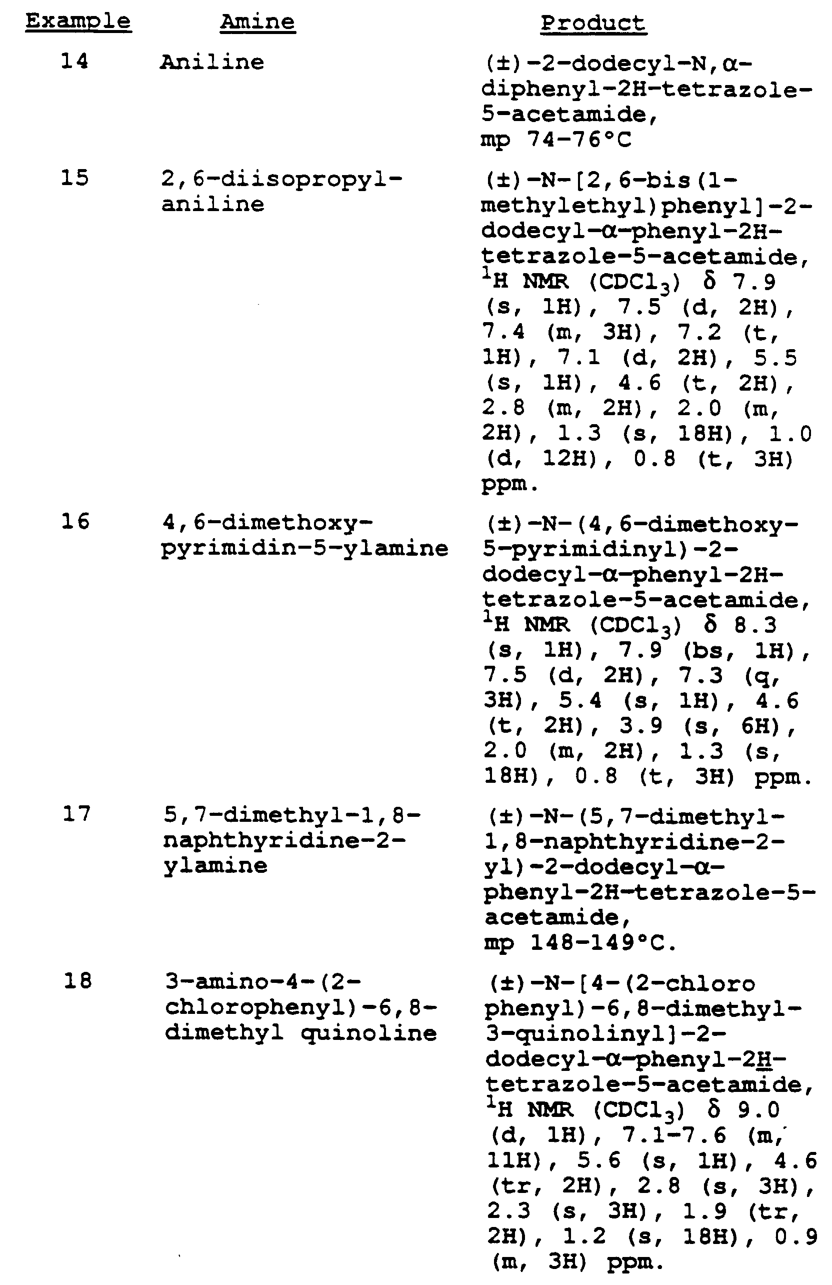

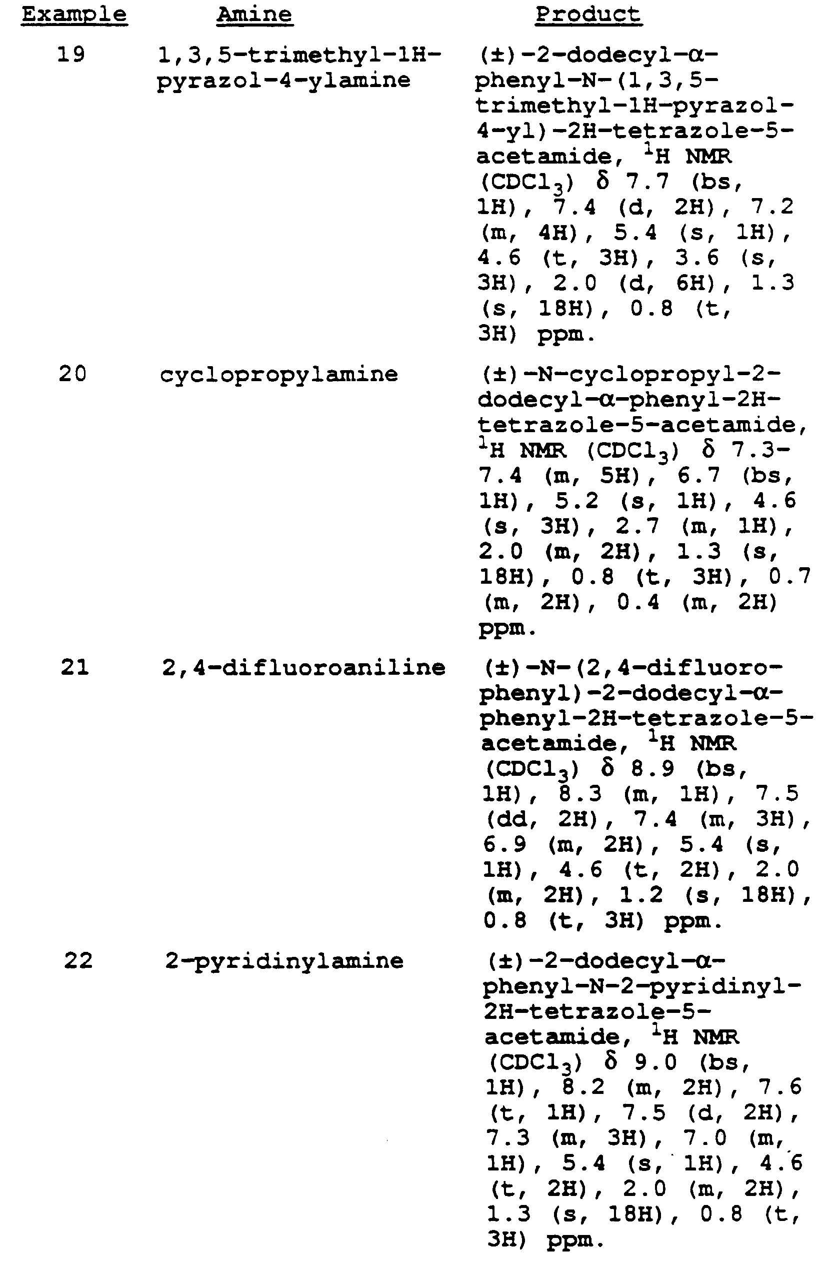

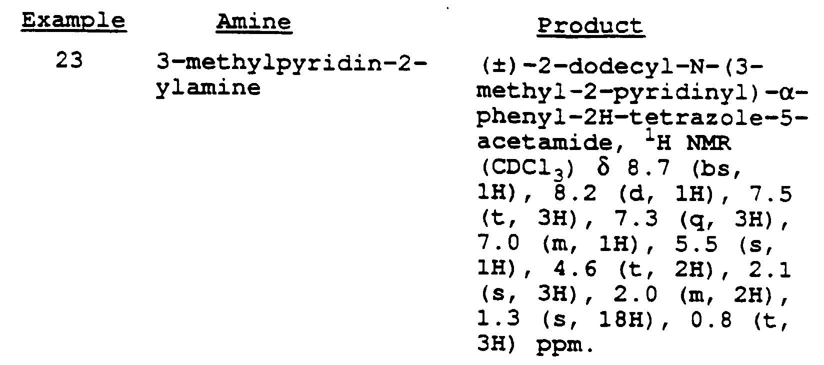

### EXAMPLE 24

### (±)-2-Dodecyl-N-(3-methyl-2-pyridinyl)-2-phenyl-2H-tetrazole-5-acetamide, N-oxide

The compound of Example 23 (0.50 g; 1.0 mmole) was dissolved in dichloromethane and then treated with MCPBA (0.22 g; 1.1 mmole) in one portion and stirred at room temperature for 12 hours. The resulting 3-chlorobenzoic acid byproduct was removed by washing the organic solution with aqueous potassium carbonate and then brine. The dichloromethane was dried over magnesium sulfate, filtered, and concentrated in vacuo, leaving a white precipitate. The crude product was triturated with ethyl ether and collected by filtration.
¹H NMR (CDCl₃) δ 9.7 (bs, 1H), 8.1 (d, 1H), 7.6 (d, 2H), 7.3 (q, 3H), 7.1 (d, 1H), 7.0 (t, 1H), 5.5 (s, 1H), 4.6 (t, 2H), 2.2 (s, 3H), 2.0 (m, 2H), 1.3 (s, 18H), 0.8 (t, 3H) ppm.

### EXAMPLE 25

### (±)-N-(2,4-Difluorophenyl)-1-dodecyl-α-phenyl-1H-tetrazole-5-acetamide

### (a) 5-Benzyl-1-dodecyl-1H-tetrazole

(±)-1-Dodecyl-α-phenyl-1H-tetrazole-5-acetic acid, ethyl ester, i.e., the compound of Example 13(c) (14 g; 0.034 mmoles) was dissolved in absolute ethanol (175 mL) and treated with sodium hydroxide pellets (2.7 g; 0.069 mmoles). The solution was stirred for 30 minutes forming a gelatinous precipitate. The solid was removed by filtration, dissolved in water, and then acidified to a pH of 1.0 using concentrated HCl. The precipitate was collected by filtration and washed with water. Yield: 8.5 g (76%), mp 50-51°C.

### (b) (±)-N-(2,4-Difluorophenyl)-1-dodecyl-α-phenyl-1H-tetrazole-5-acetamide

The compound from (a) above (1.5 g; 4.5 mmoles) was dissolved in tetrahydrofuran (20 mL), cooled to -20°C, and then treated dropwise with n-butyllithium (2.8 mL; 4.5 mmoles) for over 5 minutes. The solution was stirred for 5 minutes before adding 2,4-difluorophenyl isocyanate (0.7 g; 4.5 mmoles). The ice bath was removed and the solution gradually warmed to room temperature over 30 minutes, at which time the reaction was quenched with water (20 mL) and diluted with ethyl acetate. The layers were separated and the organic portion was washed with aqueous HCl (1N), aqueous sodium carbonate (10%), and brine. The solution was dried over magnesium sulfate, filtered, and stripped to dryness leaving a viscous liquid that was dissolved in 75% hexane/25% ethyl acetate and chromatographed using silica gel. Yield: 0.9 g (41%).
¹H NMR (CDCl₃) δ 10.1 (s, 1H), 8.1 (m, 1H), 7.3 (s, 5H), 6.8 (m, 2H), 5.2 (s, 1H), 4.2 (t, 2H), 1.6 (m, 2H), 1.2 (d, 18H), 0.8 (t, 3H) ppm.

### EXAMPLE 26

### (±)-N-[2,6-Bis(1-methylethyl)phenyl]-1-dodecyl-α-phenyl-1H-tetrazole-5-acetamide

When in the procedure of Example 25(b) an appropriate amount of 2,6-diisopropylphenylisocyanate was substituted for 2,4-difluorophenylisocyanate and the general procedure of Example 25(b) was followed, the title compound was obtained, mp 113-115°C.

### EXAMPLE 27

### 2-Dodecyl-α,α-dimethyl-N-(2,4,6-trimethoxyphenyl)-2H-tetrazole-5-acetamide

### (a) Ethyl 2,2-dimethylcyanoacetate

A solution of ethyl cyanoacetate (20 g; 0.17 moles) in tetrahydrofuran (350 mL) was cooled to -10°C followed by the addition of sodium hydride (7.25 g; 0.17 moles) in several portions. The suspension was stirred for 10 minutes at -10°C before adding iodomethane (23.3 g; 0.17 moles). The ice bath was removed and the solution gradually warmed to 20°C for over 45 minutes. The solution was then recooled to -10°C and a second equivalent of sodium hydride (7.25 g; 0.17 moles) was added, again, in small portions. Soon after, iodomethane (23.3 g; 0.17 moles) was added, the ice bath removed, and the solution stirred at room temperature for 2 hours before being quenched with H₂O. The product was extracted with ethyl ether (500 mL) and washed with brine, dried over MgSO₄, and the solution concentrated in vacuo, leaving a crude product that was purified by distillation. Yield: 16.9 g, b.p. 82-85°C; 15 mm Hg.

### (b) α,α'-Dimethyltetrazole-5-acetic acid, ethyl ester

Ethyl-2,2-dimethylcyanoacetate (a) (11.6 g; 0.082 moles) was dissolved in dioxane (240 mL) and treated with tri-n-butyltin azide (76.3 g; 0.23 moles) in one portion. The solution was refluxed for overnight, cooled to room temperature, and then concentrated in vacuo. The resulting liquid was dissolved in ethyl ether (500 mL) and treated with gaseous HCl continuously for 15 minutes. The ether was concentrated in vacuo, leaving a viscous liquid which gradually solidified on standing. Yield: 8.4 g.
¹H NMR (CDCl₃) δ 12.2 (bs, 1H), 4.2 (q, 2H), 1.8 (s, 6H), 1.3 (5, 3H) ppm.

### (c) 2-Dodecyl-α,α'-dimethyl-2H-tetrazole-5-acetic acid, ethyl ester

The compound obtained in (b) above (4.0 g; 0.021 moles) was dissolved in acetonitrile (50 mL) containing one equivalent of triethylamine (2.3 g; 0.021 moles). The solution was heated to reflux followed by the addition of 1-bromododecane (5.6 g; 0.022 moles). The solution was refluxed for 16 hours, cooled to room temperature, and then concentrated in vacuo. The residue was triturated with ethyl acetate (250 mL), filtered, and the filtrate was washed with aqueous HCl (1N), brine, and dried over magnesium sulfate. Concentration of the solution after filtration afforded a viscous liquid containing both the 1- and 2-regioisomers. The latter isomer was obtained by silica gel chromatography using 75% hexane and 25% ethyl acetate as the eluant. The product was isolated as a colorless liquid (4.5 g).
¹H NMR (CDCl₃) δ 4.5 (t, 2H), 4.1 (q, 2H), 1.9 (m, 2H), 1.7 (s, 6H), 1.2 (s, 18H), 0.9 (t, 3H) ppm.

### (d) 2-Dodecyl-α,α'-dimethyl-2H-tetrazole-5-acetic acid

The compound obtained in (c) above (3.2 g; 0.009 moles) was dissolved in absolute ethanol (40 mL) and treated with sodium hydroxide pellets (.38 g; 0.0095 moles) in one portion. The solution was stirred at room temperature for overnight before concentrating the ethanol in vacuo. The residue was dissolved in H₂O and acidified to a pH of 1.0. The product was extracted with ethyl acetate in two portions. The combined organic solution was washed with brine, dried over magnesium sulfate, and filtered. The filtrate was concentrated in vacuo leaving a colorless liquid that solidified on standing. Yield: 2.05 g.
¹H NMR (CDCl₃) δ 4.5 (t, 2H), 2.0 (m, 2H), 1.7 (s, 6H), 1.2 (s, 18H), 0.9 (t, 3H) ppm.

### (e) 2-Dodecyl-α,α'-dimethyl-N-(2,4,6-trimethoxyphenyl)-2H-tetrazole-5-acetamide

The carboxylic acid obtained in (d) above (2.0 g; 0.006 moles) was dissolved in dry THF (50 mL) and then treated with carbonyldiimidazole (1.0 g; 0.006 moles) in one portion. The solution was stirred for 1 hour under nitrogen before adding 2,4,6-trimethoxyaniline (1.0 g; 0.006 moles), also in one portion. The solution was stirred for 5 days under nitrogen and at room temperature. The solution was diluted with ethyl acetate and washed with aqueous HCl (1N), NaOH (1N), and brine. Magnesium sulfate was added as the drying agent and the solution filtered. The filtrate was concentrated in vacuo leaving a maroon-colored liquid. The crude product was purified by silica gel chromatography employing 75% hexane and 25% ethyl acetate as the eluant. Yield: 1.5 g colorless liquid.
¹H NMR (CDCl₃) δ 7.2 (bs, 1H), 6.1 (s, 2H), 4.6 (t, 2H), 3.7 (d, 9H), 2.1 (m, 2H), 1.7 (s, 6H), 1.3 (s, 18H), 0.9 (t, 3H) ppm.

### EXAMPLE 28

### 2-Dodecyl-α,α'-(2-propenyl)-N-(2,4,6-trimethoxyphenyl)-2H-tetrazole-5-acetamide

Following the general procedure of Example 27, only substituting ethyl-2,2-bis(allyl)cyanoacetate for ethyl-2,2-dimethylcyanoacetate in 27(a) and following the general procedure of 13(a) through 13(e) the title compound was obtained.
¹H NMR (CDCl₃) δ 8.5 (bs, 1H), 6.1 (s, 2H), 5.7 (m, 2H), 5.0 (m, 4H), 4.6 (t, 2H), 3.7 (d, 9H), 3.0 (dd, 2H), 2.9 (dd, 2H), 1.9 (m, 2H), 1.2 (s, 18H), 0.8 (t, 3H) ppm.

### EXAMPLE 29

### 1-(2-Dodecyl-2H-tetrazol-5-yl)-N-(2,4,6-trimethoxyphenyl)cyclopentanecarboxamide

### (a) 1,1-Dicyanocyclopentane

Sodium hydride (37.8 g; 0.94 moles) was suspended in dimethylformamide (250 mL) under an atmosphere of N₂. A solution of malononitrile (30 g; 0.45 moles) and 1,4-dibromobutane 99.7 g; 0.45 moles) in dimethylformamide (150 mL) was added dropwise at such a rate so as not to exceed 30°C. The mixture was stirred for overnight, poured into H₂O (500 mL), and then washed with two portions of ethyl ether. The organics were combined, washed with brine, and dried over magnesium sulfate. The drying agent was removed by filtration and the filtrate was concentrated in vacuo, leaving a bilayered liquid. The lower portion was separated (28.8 g) and identified as the desired product.
¹H NMR (CDCl₃) δ 2.4 (m, 4H), 2.0 (m, 4H) ppm.

### (b) 5-Cyanocyclopentyl tetrazole

The compound obtained in (a) above (9.8 g; 0.082 moles) was dissolved in dioxane (240 mL) and treated with tri-n-butyltin azide (27.3 g; 0.082 moles) in one portion. The solution was refluxed overnight, cooled, and the dioxane removed in vacuo. The resulting liquid was taken up in ethyl ether and continuously treated with gaseous HCl for over 15 minutes. The ethereal solution was concentrated in vacuo leaving a viscous orange syrup. Yield: 11.0 g.

### (c) 5-Cyanocyclopentyl-2-dodecyl-2H-tetrazole

The tetrazole (b) obtained above (11.0 g; 0.067 moles) was dissolved in acetonitrile (150 mL) containing one equivalent of triethylamine (6.8 g; 0.067 moles). The solution was heated to reflux followed by the addition of 1-bromo dodecane (16.8 g; 0.067 moles). Isolation of the 2-isomer was achieved employing the same conditions described for Example 11. Yield: 7.5 g; colorless liquid.
¹H NMR (CDCl₃) δ 4.6 (t, 2H), 2.5 (m, 4H), 2.0 (m, 6H), 1.3 (s, 18H), 0.9 (t, 3H) ppm.

### (d) 2-Dodecyl-α,α-spirocyclopentyl-2H-tetrazole-5-acetic acid

The nitrile obtained in (c) above (7.5 g; 0.022 moles) was dissolved in absolute ethanol (150 mL) and treated with aqueous (50%) sodium hydroxide (18 g; 0.022 moles). The solution was refluxed for 4 hours, cooled to room temperature, and then concentration of the solvent in vacuo. The sodium salt was dissolved in H₂O, acidified to a pH of 1.0, and then the product was extracted with ethyl ether. The organic solution was dried over magnesium sulfate, filtered, and concentration of the solvent in vacuo leaving a viscous liquid which gradually solidified over several days. Yield: 5.8 g.
¹H NMR (CDCl₃) δ 4.6 (t, 2H), 2.5 (m, 4H), 2.0 (m, 2H), 1.8 (m, 4H), 1.3 (s, 18H), 0.9 (t, 3H) ppm.

### (e) 2-Dodecyl-α,α-spirocyclopentyl-N-(2,4,6-trimethoxyphenyl)-2H-tetrazole-5-acetamide

The acid obtained in (d) above (1.5 g; 0.0042 moles) was dissolved in dichloromethane (50 mL), cooled to -10°C, and then treated with 2,4,6-trimethoxyaniline hydrochloride (0.94 g; 0.0042 moles). Soon after, triethylamine (0.43 g; 0.0042 moles) was added and then dicyclohexylcarbodiimide (0.88 g; 0.0042 moles) in one portion. This suspension gradually warmed to room temperature with stirring for overnight. The mixture was filtered and the filtrate was washed with aqueous HCl (1N), brine, dried over magnesium sulfate, and then filtered. Concentration of the solvent in vacuo afforded a viscous liquid that was dissolved in 50% ethyl acetate/50% hexane and purified by silica gel chromatography. Yield: 1.6 g colorless liquid.
¹H NMR (CDCl₃) δ 7.3 (bs, 1H), 6.1 (s, 2H), 4.6 (t, 2H), 3.8 (d, 9H), 2.6 (m, 2H), 2.5 (m, 2H), 2.0 (m, 2H), 1.9 (m, 2H), 1.6 (m, 2H), 1.2 (s, 18H), 0.9 (t, 3H) ppm.

### EXAMPLE 30

### (±) N-(1,1-dimethylethyl)-2-dodecyl-α-phenyl-2H-tetrazole-5-acetamide

When in the procedure of Example 13(e) an appropriate amount of tert-butylamine was substituted for 2,4,6-trimethoxyaniline and the general procedure of Example 13(e) was followed, the title compound was obtained.
¹H NMR (CDCl₃) δ 7.3 (m, 5H), 6.4 (bs, 1H), 5.1 (s, 1H), 4.6 (t, 2H), 2.0 (m, 2H), 1.3 (s, 18H), 1.2 (s, 9H), 0.9 (t, 3H) ppm.

### EXAMPLE 31

### (±)-2-Octyl-α-phenyl-N-(2,4,6-trimethoxyphenyl)-2H-tetrazole-5-acetamide

When in the procedure of Example 13(b) an appropriate amount of 1-bromooctane was substituted for 1-bromododecane and the general procedure of Example 13(b), (d), and (e) was followed, the title compound was obtained, mp 113-116°C.

### EXAMPLE 32

### (±) 2-Hexadecyl-α-phenyl-N-(2,4,6-trimethoxyphenyl)-2H-tetrazole-5-acetamide

When in the procedure of Example 13(b) an appropriate amount of 1-bromohexadecane was substituted for 1-bromododecane and the general procedure of Example 13(b), (d), and (e) was followed, the title compound was obtained, mp 134-135°C.

### EXAMPLE 33

### 2-Tridecyl-α,α-dimethyl-N-(2,4,6-trimethoxyphenyl)-2H-tetrazole-5-acetamide

When in the procedure of Example 27(c) an appropriate amount of 1-bromotridecane was substituted for 1-bromododecane and the general procedure of Example 27(c), (d), and (e) was followed, the title compound was obtained.
¹H NMR (CDCl₃) δ 7.5 (br.s, 1H), 6.05 (s, 2H), 4.6 (t, 2H), 3.8 (s, 3H), 3.75 (s, 6H), 1.8 (s, 6H), 1.2-1.4 (m, 22H), 0.9 (m, 3H) ppm.

### EXAMPLE 34

### 2-Dodecyl-N-(2,4,6-trimethoxyphenyl)-2H-tetrazole-5-propanamide

(a) A mixture of methyl 3-cyanopropanoate (27.3 g, 0.241 mol), NH₄Cl (11.5 g, 0.215 mol), and NaN₃ (13.9 g, 0.214 mol) in dimethylformamide (225 mL) was heated at 100°C for 6 hours. The mixture was allowed to cool and filtered. The filtrate was concentrated in vacuo. The residue was dissolved in H₂O (200 mL). The solution was acidified with concentrated HCl (52 mL) and extracted with EtOAc (9 x 200 mL). The extracts were washed (saturated NaCl), dried (MgSO₄), and concentrated in vacuo to an oil; yield 29.2 g. The oil was dissolved in CH₃CN (590 mL) and Et₃N (29.5 mL, 0.21 mol). The solution was heated to 60°C. To this solution was added in one portion 1-bromododecane (49.5 mL, 0.21 mol), and the mixture was refluxed for 50 hours. The mixture was allowed to cool and filtered. The filtrate was concentrated in vacuo to a thick suspension, and the suspension was triturated with ether (500 mL). The ether was concentrated in vacuo to an oil, and the oil was chromatographed on silica gel (470 g, 70-230 mesh) using petroleum ether-EtOAc (15:1, 15 x 900 mL and 10:1, 20 x 900 mL) as eluent. A white solid was obtained; yield 12.0 g (15%) of methyl 2-dodecyl-2H-tetrazole-5-propanoate, mp 39-42°C.
   Chromatography gave a white solid; yield 8.64 g (11%) of methyl 1-dodecyl-1H-tetrazole-5-propanoate, mp 43-45°C.
(b) To a stirred, room temperature solution of KOH (2.5 g) in absolute ethanol (210 mL) was added in one portion the 2-dodecyl-2H-tetrazole ester (11.5 g, 0.0354 mol), and the resulting solution was stirred for 3 days. The solution was concentrated in vacuo to a white solid. The solid was partitioned between 0.4 M HCl (310 mL) and CH₂Cl₂. The CH₂Cl₂ layer was dried (MgSO₄) and concentrated in vacuo to a white solid; yield: 10.63 g (96.6%) of 2-dodecyl-2H-tetrazole-5-propanoic acid, mp 63-65°C.
(c) To a stirred, room temperature solution of the 2-dodecyl-2H-tetrazole acid (1.60 g, 0.00515 mol) in tetrahydrofuran (50 mL) was added in one portion carbonyldiimidazole (0.93 g, 0.0057 mol), and the mixture was stirred for 2 hours. To the mixture was added a solution of 2,4,6-trimethoxyaniline (0.99 g, 0.0054 mol) in THF (50 mL), and the mixture was refluxed for 3 days. The mixture was concentrated in vacuo to a viscous liquid that was chromatographed on silica gel (400 g, 70-230 mesh) using petroleum ether-ETOAc (1:1, 11 x 500 mL; 2:3, 18 x 500 mL) as eluent. The product was rechromatographed on silica gel (300 g, 70-230 mesh) using petroleum ether-acetone (3:1, 13 x 500 mL) as eluent to give an off-white solid; yield: 1.2 g (49%) of N-(2,4,6-trimethoxyphenyl)-2-dodecyl-2H-tetrazole-5-propanamide, mp 86-88°C.

### EXAMPLE 35

### N-(2,6-Bis(1-methylethyl)phenyl)-2-dodecyl-2H-tetrazole-5-propanamide

In a manner similar to Example 34, 2-dodecyl-2H-tetrazole-5-propanoic acid was condensed with 2,6-bis(1-methylethyl)aniline to give the title compound, mp 41-43°C.

### EXAMPLE 36

### N-(2,4-Difluorophenyl)-2-dodecyl-2H-tetrazole-5-propanamide

In a manner similar to Example 34, 2-dodecyl-2H-tetrazole-5-propanoic acid was condensed with 2,4-difluoroaniline to give the title compound, mp 86-87°C.

### EXAMPLE 37

### 1-Dodecyl-N-(2,4,6-trimethoxyphenyl)-1H-tetrazole-5-propanamide

In a manner similar to Example 34, methyl 1-dodecyl-1H-tetrazole-5-propanoate was saponified with KOH to give 1-dodecyl-1H-tetrazole-5-propanoic acid. The acid was condensed with 2,4,6-trimethoxyaniline to give the title compound, mp 57-61°C.

### EXAMPLE 38

### (±)-2-Dodecyl-α-(2-pyridyl)-N-(2,4,6-trimethoxyphenyl)-2H-tetrazole-5-acetamide hydrochloride

### (a) 5-(2-Pyridylmethyl)-1H-tetrazole

2-Pyridylacetonitrile (10.0 g; 0.084 moles) was dissolved in p-dioxane (200 mL) and then treated with tributyltin azide (30.9 g; 0.093 moles) in one portion. The solution was refluxed for 20 hours, cooled to room temperature, and then concentrated in vacuo. The viscous syrup was taken up in ethyl ether and treated with gaseous HCl for over 15 minutes, affording a maroon-colored precipitate that was recrystallized from ethanol. Yield: 9.1 g (55%).
¹H NMR (DMSO): δ 10.4 (bs, 1H), 8.9 (d, 1H), 8.4 (t, 1H), 7.9 (t, 2H), 4.8 (s, 2H) ppm.

### (b) 4-(2-Pyridylmethyl)-2-dodecyl-2H-tetrazole

The tetrazole (a) (3.0 g; 0.015 moles) was taken up in acetonitrile (50 mL) containing two equivalents of triethylamine (3.0 g; 0.030 moles). The suspension was heated to reflux and then treated with 1-bromododecane (3.7 g; 0.015 moles) dropwise for several minutes. The solution was refluxed for 16 hours, cooled to room temperature, and the solvent removed in vacuo. The residue was triturated with ethyl acetate, filtered, and concentration of the filtrate in vacuo leaving a maroon-colored liquid. The 2-isomer was obtained by dissolving the crude product in 50% hexane/50% ethyl acetate and removing the impurities, including the 1-regioisomer, by silica gel chromatography. Yield: 2.0 g (41%).
¹H NMR (CDCl₃): δ 8.5 (d, 1H), 7.7 (t, 1H), 7.3 (d, 1H), 7.2 (m, 1H), 4.5 (t, 2H), 4.4 (s, 2H), 1.9 M, 2H), 1.3 (s, 18H), 0.9 (t, 3H) ppm.

### (c) (±)-2-Dodecyl-α-(2-pyridyl)-N-(2,4,6-trimethoxyphenyl)-2H-tetrazole-5-acetamide-HCl

Compound (b) (2.0 g; 6.0 mmoles) was dissolved in dry tetrahydrofuran (40 mL), cooled to -20°C, and then treated with n-butyllithium (4.0 mL; 6.0 mmoles) dropwise for over 5 minutes. The bright yellow solution was stirred at -20°C for 10 minutes before adding 2,4,6-trimethoxyphenyl isocyanate (1.3 g; 6.5 mmoles) in one portion. The solution gradually warmed to room temperature for over 3 hours and was then quenched with water. The product was extracted with several portions of chloroform, which were combined, dried over MgSO₄, and filtered. The solution was concentrated in vacuo, leaving a viscous yellow syrup that was purified by silica gel chromatography employing a gradient elution composed of hexane/ethyl acetate. The purified product was dissolved in ethyl ether and added dropwise to an ethereal HCl solution. The ether was removed in vacuo leaving a tan-colored solid. Yield: 1.8 g (51).
¹H NMR (DMSO): δ 9.4 (s, 1H), 8.7 (d, 1H), 8.3 (t, 1H), 7.9 (d, 1H), 7.7 (t, 1H), 6.2 (s, 2H), 5.9 (s, 1H), 4.7 (t, 2H), 3.7 (d, 9H), 1.9 (m, 2H), 1.2 (s, 18H), 0.9 (t, 3H) ppm.

### EXAMPLE 39

### 4-Amino-1,3,5-trimethylpyrazole

### (a) (1,3,5-Trimethylpyrazole

2, 4-Pentanedione (3.8 g; 0.038 moles) was dissolved in acetic acid (30 mL) and then treated with methyl hydrazine sulfate (5.9 g; 0.041 moles) and sodium acetate (3.36 g; 0.041 moles). The suspension was heated on a steam bath for 2 hours, cooled to room temperature, and then added dropwise to saturated aqueous potassium carbonate. The product was extracted with two portions of ethyl acetate and the extracts were combined, dried over magnesium sulfate, and filtered. The filtrate was concentrated in vacuo, leaving an orange liquid. Yield: 3.4 g (81%). ¹H NMR (CDCl₃) δ 5.7 (s, 1H), 3.7 (s, 3H), 2.2 (s, 6H) ppm.

### (b) 4-Nitro-1,3,5-trimethylpyrazole

The pyrazole from (a) above (3.1 g; 0.028, moles) was dissolved in cold sulfuric acid (15 mL), cooled to 0°C, and then treated with fuming nitric acid (12 mL). The acidic solution was heated on a steam bath for 2 hours, cooled to room temperature, and poured over ice. The solution was made basic (pH = 12) and the precipitate was collected by filtration and washed with water. Yield: 2.3 g (53%), white solid. ¹H NMR (CDCl₃) δ 3.7 (s, 3H), 2.6 (s, 3H), 2.5 (s, 3H) ppm.

### (c) 4-Amino-1,3,5-trimethylpyrazole

The compounds from (b) above (2.3 g; 0.014 moles) was catalytically hydrogenated using Raney nickel (1 g) in methanolic ammonia (100 mL) under a hydrogen atmosphere at 50 psi. The catalyst was filtered and the solution concentrated in vacuo, leaving a residue that was triturated several times with ethyl ether. The decanted solvent was concentrated to dryness, leaving a pale red solid. Yield: 1.3 g (70%).
¹H NMR (CDCl₃) δ 3.6 (s, 3H), 2.4 (bs, 2H), 2.1 (s, 6H) ppm.

### EXAMPLE 40

Following the general procedure of Example 39 only substituting 2-pyridylhydrazine for methylhydrazine sulfate, the following compound was obtained:
2-[4-amino-3,5-dimethyl-1H-pyrazol-1-yl]pyridine. ¹H NMR (CDCl₃) δ 8.4 (d, 1H), 7.7 (m, 2H), 7.2 (m, 1H), 2.4 (s, 3H), 2.2 (s, 3H), 2.0 (bs, 2H) ppm.

### EXAMPLE 41

Following the general procedure of Example 13 only substituting the compound of Example 40 for 2,4,6-trimethoxyaniline in Step (e) of Example 13 the following compound was obtained:
(±)2-dodecyl-α-phenyl-N-[[1-(2-pyridyl)-3,5-dimethyl]pyrazol-4-yl]-2H-tetrazole-5-acetamide. ¹H NMR (CDCl₃) δ 8.3 (d, 1H), 7.8 (bs, 1H), 7.7 (d, 2H), 7.5 (d, 2H), 7.3 (m, 3H), 7.1 (t, 1H), 5.4 (s, 1H), 4.6 (5, 2H), 2.4 (s, 3H), 2.1 (s, 3H), 2.0 (m, 2H), 1.3 (s, 18H), 0.9 (t, 3H) ppm.

### EXAMPLE 42

The following compound is prepared according to the procedure set forth in Chart VII:
2-dodecyl-N-(2,4,6-trimethoxphenyl)-2H-tetrazole-5-(3,3-dimethylpropanamide).

### EXAMPLE 43

### Isolation of the pure enantiomers of (±) 2-dodecyl-α-phenyl-N-(2,4,6-trimethoxyphenyl)-2H-tetrazole-5-acetamide

A chromatographic charge is prepared by completely dissolving 1.85 g of racemic 2-dodecyl-α-phenyl-N-(2,4,6-trimethoxyphenyl)-2H-tetrazole-5-acetamide, Example 13, in 45 mL of a solution of 80:20 2-propanol:hexane and warming to 65°C. Two milliliters of this solution is injected onto a 500 x 20.0 mm Chiralcel OG® preparative column (Diacel Chemical Industries, Tokyo, Japan). This charge is chromatographed over the support with 80:20 hexane:2-propanol at a flow rate of 8.0 mL/min. The column and injector are jacketed in an Advanced Air Oven (Kariba Instruments Cardiff, South Wales, UK) at a constant temperature of 40°C. The eluate is monitored by measuring its ultraviolet absorbance at 290 nm.

The first major ultraviolet absorbing fraction is the (-) enantiomer, (-)-2-dodecyl-α-phenyl-N-(2,4,6-trimethoxyphenyl)-2H-tetrazole-5-acetamide. The capacitance Factor k' for this enantiomer is approximately 5.6 (112 mL solution) and the solution is designated as "Solution A". The value for the capacitance Factor k' is given by the expression k' = (Vₑ - Vₒ)/Vₒ where Vₒ is the void volume, 90 mL, and Vₑ is the volume of mobile phase eluted at the maximum ultraviolet absorbance of the first (-) enantiomer, i.e., (-)-2-dodecyl-α-phenyl-N-(2,4,6-trimethoxyphenyl) -2H-tetrazole-5-acetamide. The second major ultraviolet absorbing fraction is the (+) enantiomer, (+)-2-dodecyl-α-phenyl-N-(2,4,6-trimethoxyphenyl)-2H-tetrazole-5-acetamide. This component elutes at a k' of 7.3 (208 mL solution) and is designated as "Solution B". An intermediate fraction eluting at a k' of 6.7 (48 mL solution), which corresponds to the ultraviolet minimum between the two enantiomers contains approximately equal parts of each enantiomer.

This preparative procedure is repeated an additional 19 times. All the "Solution A" fractions are combined and concentrated to a dried film in an open beaker. This film is scraped from the sides of the beaker. The solid is collected and weighed. The resulting 708 mg of (-)-2-dodecyl-α-phenyl-N-(2,4,6-trimethoxyphenyl)-2H-tetrazole-5-acetamide, is found to be 98% enantiomerically pure by high performance liquid chromatography using the conditions listed in Table A. The 20 fractions labeled "Solution B" are combined and dried as described for the "Solution A" fractions. The resulting 727 mg of solid, (+)-2-dodecyl-α-phenyl-N-(2,4,6-trimethoxyphenyl)-2H-tetrazole-5-acetamide, is found to be 96% enantiomerically pure by high performance liquid chromatography using the system described in Table A. The physical properties of (-)-2-dodecyl-α-phenyl-N-(2,4,6-trimethoxyphenyl)-2H-tetrazole-5-acetamide and (+)-2-dodecyl-α-phenyl-N-(2,4,6-trimethoxyphenyl)-2H-tetrazole-5-acetamide appear in Table B.

**TABLE A**

| |
|---|
| Column: Chiralcel OG 4.6 x 250 mm 10 µm spherical particles |
| Mobile Phase: 80:20 hexane:2-propanol |
| Detection: 214 nm |
| Temperature: 40°C |
| Injection Volume: 20 µL |
| Charge Conc.: 0.150 mg/mL in the mobile phase |

**TABLE B**

| | (-)-2-docecyl-α-phenyl-N-(2,4,6-trimethoxyphenyl)-2H-tetrazole-5-acetamide | (+)-2-docecyl-α-phenyl-N-(2,4,6-trimethoxyphenyl)-2H-tetrazole-5-acetamide |
|---|---|---|
| Optical Rotation | [ȧ]_{D} = -58.0 | [ȧ]_{D} = +55.1 |
| | | |
| | (c. 1.00 MeOH) | (c. 1.00 MeOH) |
| | | |
| Retention Volume | 16.2 mL | 18.8 mL |

### EXAMPLE 44

### (±)-2-Dodecyl-α-methyl-α-phenyl-N-(2,4,6-trimethoxyphenyl)-2H-tetrazole-5-acetamide

### (a) (±)-2-Dodecyl-α-methyl-α-phenyl-2H-tetrazole-5-acetic acid

To a THF solution (30 mL) of n-BuLi (0.0055 mol, 1.6 M in hexanes) at -78°C under N₂ with stirring was added 1.0 g (0.00027 mol) of (±)-2-dodecyl-α-phenyl-2H-tetrazole-5-acetic acid (Compound d, Example 13). The resulting yellow solution was stirred at -78°C for 30 minutes before iodomethane (0.34 mL, 0.0055 mol) was added. This solution was stirred for 3 hours before quenching with 1N HCl (20 mL). The mixture was then partitioned between ethyl acetate and water. The organic phase was washed with water, brine, dried over MgSO₄, filtered, and concentrated in vacuo to yield 1.12 g of pure product.
¹H NMR (CDCl₃) δ 9.9 (br.s, 1H), 7.3 (s, 5H), 4.6 (tr, 2H), 2.2 (s, 3H), 2.1 (tr, 2H), 1.4 (s, 18H), 0.9 (m, 3H) ppm.

### (b) (±)-2-Dodecyl-α-methyl-α-phenyl-N-(2,4,6-trimethoxy-phenyl)-2H-tetrazole-5-acetamide

To a dichloromethane solution (90 mL) of compound in step (a) was added 2,4,6-trimethoxyaniline·HCl (0.64 g, 0.0029 mol) and triethylamine (0.4 mL, 0.0029 mol) at 0°C under a nitrogen atmosphere with stirring. After 40 minutes, DCC (0.63 g, 0.003 mol) was added in one portion. After 10 minutes a precipitate resulted and the resulting suspension was allowed to warm to room temperature over 72 hours. The suspension was then filtered and the organic layers washed with 1N HCl, water, brine, dried over MgSO₄, filtered, and concentrated in vacuo. Flash chromatography (10%-20% EtOAc-Hex or eluant) on SiO₂ yielded 0.5 g of pure product.
¹H NMR (CDCl₃) δ 8.1 (s, 1H), 7.2-7.4 (m, 5H), 6.05 (s, 2H), 4.6 (tr, 2H), 3.8 (s, 3H), 3.75 (s, 6H), 2.1 (s, 3H), 2.0 (tr, 2H), 1.4 (s, 18H), 0.9 (m, 3H) ppm.

### EXAMPLE 45

### (±)-2-Dodecyl-β-phenyl-N-(2,4-6-trimethoxyphenyl)-2H-tetrazole-5-propanamide and (±)-1-dodecyl-β-phenyl-N-(2,4,6-trimethoxyphenyl)-¹H-tetrazole-5-acetamide

### (a) β-cyano-N-(2,4,6-trimethoxyphenyl)benzene propanamide

To a dichloromethane (150 mL) solution of 3-cyano-3-phenylpropionic acid (5 g, 0.0286 mol) at 0°C under a nitrogen atmosphere was added triethylamine (4 mL, 0.0286 mol) and 2,4,6-trimethoxyaniline·HCl (6.3 g, 0.0286 mol). To this solution was added DCC (6.2 g, 0.29 mol). The resulting mixture was allowed to warm to room temperature over 3 hours. This was then filtered and the filtrate partitioned between 1N HCl and dichloromethane. The organic layer was washed with brine, dried over MgSO₄, filtered, and concentrated in vacuo. The resulting solid (5.1 g) was recrystallized from dichloromethane/hexanes, mpt 157-160°C.

### (b) (±)-2-Dodecyl-β-phenyl-N-(2,4-6-trimethoxyphenyl)-2H-tetrazole-5-propanamide and (±)-1-dodecyl-β-phenyl-N-(2,4,6-trimethoxyphenyl)-¹H -tetrazole-5-acetamide

To a suspension of the material from step (a) (5.1 g, 0.016 mol) in dioxane (150 mL) at room temperature was added tri-n-butyltin azide (9.36 g, 0.016 mol) under N₂ with stirring. The resulting solution was heated to reflux for 24 hours. The solution was then cooled and concentrated in vacuo. The residue was redissolved in ether and HCl gas was then passed through the solution for 30 minutes. This was then concentrated in vacuo to give β-(1H-tetrazol-5-yl)-N-(2,4,6-trimethoxyphenyl)benzene propanamide as a white solid (2.1 g) which was used without further purification.

This was dissolved in acetonitrile (50 mL) and triethylamine (0.006 mol) and then heated to reflux. 1-Bromodecane (1.3 mL, 0.0055 mol) was added and the resulting solution heated to reflux for 24 hours. This was then cooled to room temperature and concentrated in vacuo. The residue was treated with ethyl acetate and filtered. The filtrate was washed with brine, dried over MgSO₄, filtered, and concentrated in vacuo. Flash chromatography (90% EtOAc-Hex as eluant, SiO₂) gave 2.6 g of a 2:1 mixture of regioisomers of the title compounds. ¹H NMR (CDCl₃) δ 7.3 (m, 10H, both regioisomers), 6.1 (s, 4H), both regioisomers), 5.0 (tr, 1H, regioisomer A), 4.8 (tr, 1H, regioisomer B), 4.5 (m, 2H, regioisomer A), 4.2 (m, 2H, regioisomer B), 3.8 (s, 18H, both regioisomers), 3.5 (m, 2H, regioisomer A), 3.1 (m, 2H, regioisomer B), 2.0 (tr, 4H, both regioisomers), 1.3 (s, 36H, both regioisomers), 0.9 (m, 6H, both regioisomers) ppm.

### EXAMPLE 46

### N-[2,6-Bis(1-methylethyl)phenyl]-2-dodecyl-α,α-diphenyl-2H-tetrazole-5-acetamide

### (a) 5-(Diphenylmethyl-1H-tetrazole

To a dioxan solution (500 mL) of diphenylacetonitrile (25.0 g, 0.129 mol) at room temperature under a nitrogen atmosphere was added tri-n-butyltin azide. The resulting solution was heated to reflux for 8 hours. This was then concentrated in vacuo. The residue was redissolved in ether (500 mL) and then treated with HCl gas for 30 minutes. This solution was then concentrated in vacuo and the resulting white solid triturated with hexane. This was then dried in vacuo to yield 15 g (50%) of the title compound, mp 154-156°C.

### (b) 5-(Diphenylmethyl-2-dodecyl-2H-tetrazole

To a solution of (a) (14.8 g, 0.063 mol) in acetonitrile (250 mL) was added triethylamine (9.6 mL, 0.069 mol) at room temperature under N₂ with stirring. This solution was then heated to reflux and 1-bromododecane (15.1 mL, 0.063 mol) was added and the resulting solution was heated to reflux for 24 hours. The solution was then concentrated in vacuo and the residue redissolved in ethyl acetate. This was then washed with water, brine, dried over MgSO₄, filtered, and concentrated in vacuo to yield a mixture of both regioisomers.

These were then separated using silica gel flash chromatography (hexane as eluant) to yield 7.7 g of the title compound as a clear oil and 5.43 g of 5-(diphenylmethyl-1-dodecyl-1H-tetrazole, mp 81-84°C. ¹H NMR (CDCl₃) δ 7.2 (s, 10H), 5.8 (s, 1H), 4.5 (tr, 2H), 1.9 (tr, 2H), 1.3 (s, 18H), 0.9 (m, 3H) ppm.

### (c) N-[2,6-Bis(1-methylethyl)phenyl]-2-dodecyl-α,α-diphenyl-2H-tetrazole-5-acetamide

To a THF solution (30 mL) of 5-(diphenylmethyl)-2-dodecyl-2H-tetrazole (1.0 g, 0.0025 mol) at -30°C under a nitrogen atmosphere with stirring was added n-BuLi (1.62 mL, 1.6 M in hexanes, 0.0026 mol). The resulting deep-red solution was stirred for 30 minutes before a THF solution (10 mL) of 2,6-diisopropylphenylisocyanate (0.53 mL, 0.0024 mol) was added dropwise over 10 minutes. The resulting yellow solution was allowed to warm to room temperature over 24 hours. Water (10 mL) was then added and the solution partitioned between ethyl acetate and water. The organic extract was washed with water, brine, dried over MgSO₄, filtered, and concentrated in vacuo to yield a yellow oil which was flash chromatographed (5% EtOAc-Hex as eluant, SiO₂) to yield 1.16 g of the title product as a clear oil.
¹H NMR (CDCl₃) δ 9.3 (s, 1H), 7.0-7.5 (m, 13H), 4.6 (tr, 2H), 2.9 (heptet, 2H), 2.0 (tr, 2H), 1.4 (s, 18H), 1.0 (s, 6H), 1.1 (s, 6H), 0.9 (m, 3H) ppm.

The following compounds were prepared by methods described previously and referred to as a reference example:

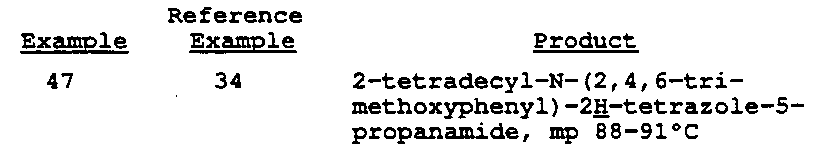

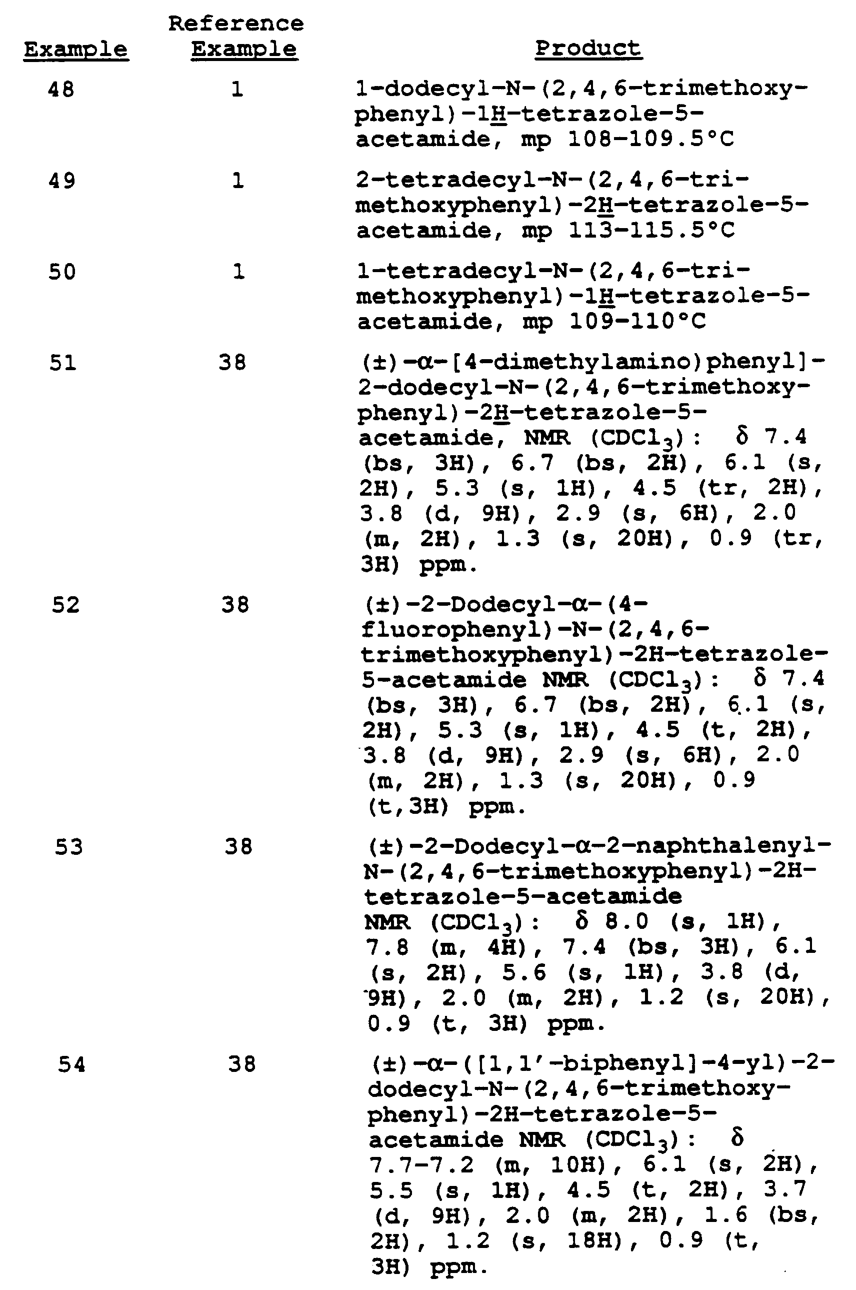

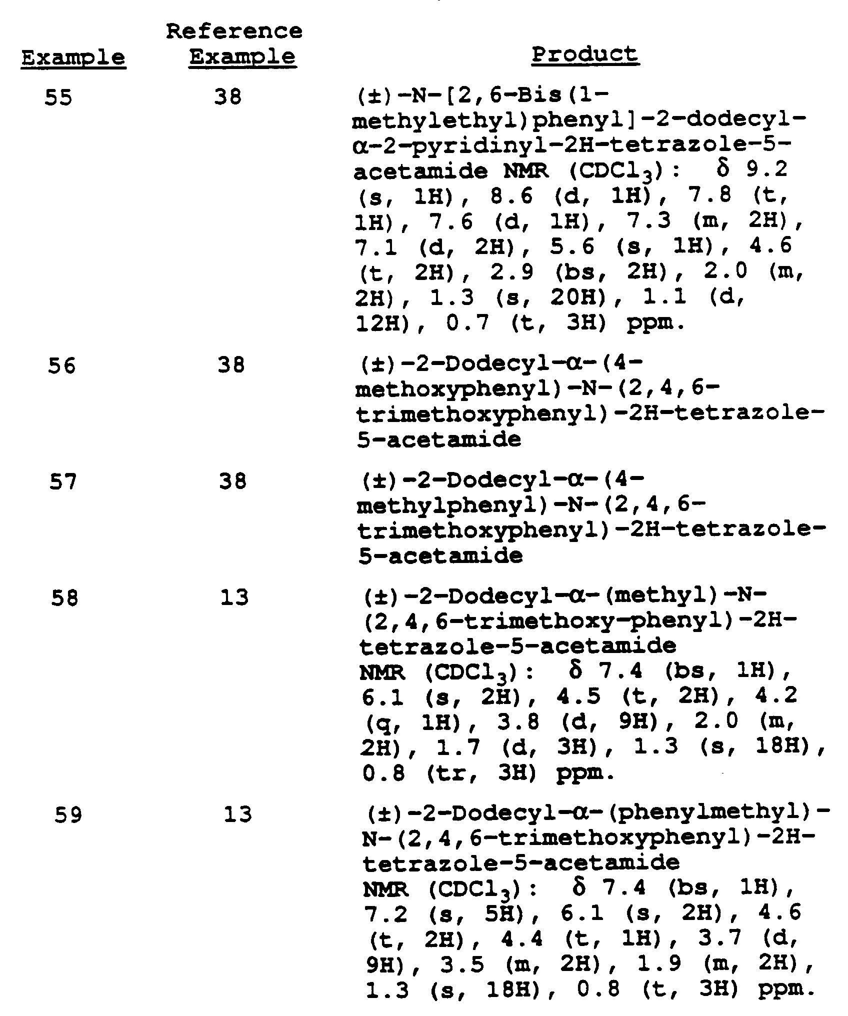

Compounds of Formula (I) containing cycloalkyl groups having from 3 to 8 carbon atoms can also be prepared employing this previously described methodology.

Alternatively, Example 13e can be catalytically hydrogenated to give the corresponding cyclohexyl analog (R₂ = cyclohexyl, R₃ = hydrogen).

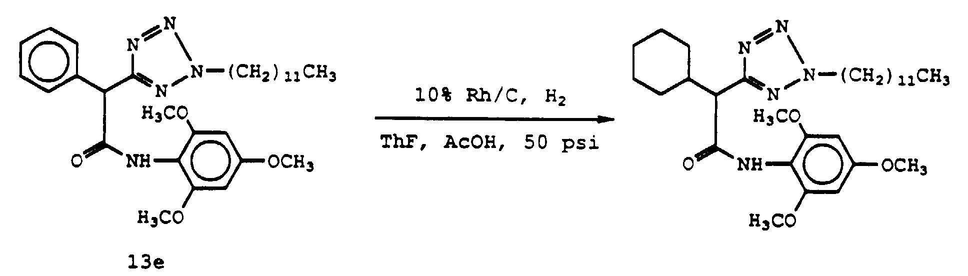

| Example | Product |
|---|---|
| 60 | (±)-2-Dodecyl-α-(cyclohexyl)-N-(2,4,6-trimethoxy-phenyl)-2H-tetrazole-5-acetamide NMR (CDCl₃): δ 7.7 (s, 1H), 6.1 (s, 2H), 4.6 (t, 2H), 3.7 (d, 9H), 3.8 (d, 1H), 2.2 (m, 1H), 2.0 (m, 3H), 1.6 (m, 6H), 1.2 (s, 20H), 1.1 (m, 3H), 0.9 (t, 3H) ppm. |

The following chiral analogs of Formula 13e have also been isolated.

| Example | Product |
|---|---|
| 61 | (-)-2-Dodecyl-α-phenyl-N-(2,4,6-trimethoxy-phenyl)-2H-tetrazole-5-acetamide[α]_{D} = -58° (1% in CH₃OH); mp 101-102°C |
| 62 | (+)-2-Dodecyl-α-phenyl-N-(2,4,6-trimethoxy-phenyl)-2H-tetrazole-5-acetamide [α]_{D} = +55.1° (1% in CH₃OH); mp 100-101°C |

Vinylic amides (11,12) are prepared from Compound 5 in Chart I as follows: 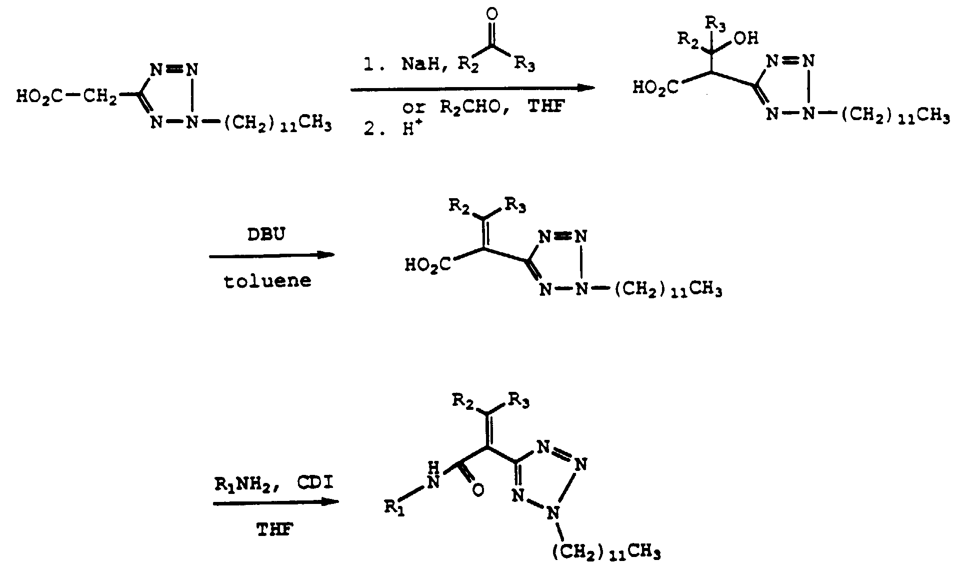 where R₁, R₂, and R₃ have been previously defined in Formula I.

Several examples are:

### EXAMPLE 63

### 2-Dodecyl-α-(phenylmethylene)-N-(2,4,6-trimethoxy-phenyl)-2H-tetrazole-5-acetamide

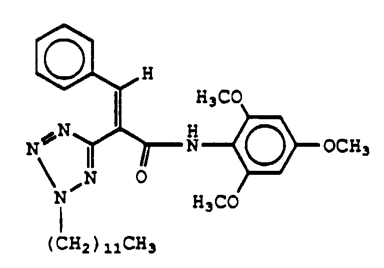

### EXAMPLE 64

### 2-Dodecyl-α-(1-methylethylidene)-N-(2,4,6-trimethoxy-phenyl)-2H-tetrazole-5-acetamide

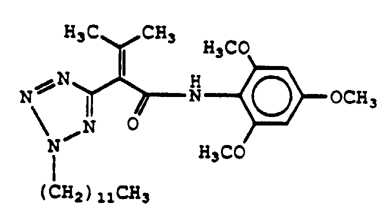

### EXAMPLE 65

### (±)-N-[2,6-Bis(1-methylethyl)phenyl]-2-dodecyl-α-fluoro-α-phenyl-2H-tetrazole-5-acetamide

### (a) 2-Dodecyl-α-hydroxy-α-phenyl-2H-tetrazole-5-acetic acid, ethyl ester

n-Butyllithium (6.9 mL of a 1.6 M hexanes solution, Aldrich) was added dropwise to a -78°C solution of tetramethylethylenediamine (1.66 mL, 11 mmole, distilled from CaH₂) in 10 mL of anhydrous THF (distilled from Na-benzophene) under dry nitrogen. The mixture was stirred for 15 minutes, then 2-dodecyltetrazole (2.38 g, 10 mmole) in anhydrous THF (5 mL) was added dropwise. The mixture was stirred for 3 hours at -78°C, then ethyl phenyl glyoxylate (1.75 mL, 11 mmole) was added dropwise. The mixture was stirred a further 2 hours, then quenched by dropwise addition of dilute HCl (pH 1). The mixture was allowed to warm to room temperature, then partitioned between ethyl acetate (200 mL) and brine (50 mL). The organic layer was dried, filtered, and concentrated to afford an oil which was flash chromatographed (silica gel, 15:1 heptane-ethyl acetate). This provided 1.55 g (37%) of the title compound as an oil. Anal. Calcd. for C₂₃H₃₆N₄O₃:
C, 66.32; H, 8.71; N, 13.45.
Found; C, 66.47; H, 8.52; N, 12.32.
250 MHz NMR (CDCl₃): δ 0.88 (t, 3H, J = 7 Hz), 1.26 (m, 23H), 2.02 (m, 2H), 4.30 (m, 2H), 4.60 (t, 2H, J = 7 Hz), 7.38 (m, 3H), 7.66 (m, 2H), IR (film) 2928, 2856, 1735, 1449, 1256, 697 cm⁻¹.

### (b) 2-Dodecyl-α-fluoro-α-phenyl-2H-tetrazole-5-acetic acid, ethyl ester

A solution of 2-dodecyl-α-hydroxy-α-phenyl-2H-tetrazole-5-acetic acid, ethyl ester (0.45 g, 1.08 mmole) in CH₂Cl₂ (2 mL) was added dropwise to a -78°C solution of diethyl amino sulfur trifluoride (DAST, J. Org. Chem. (40):574:578, 1975, 0.15 mL, 1.1 mmole) in CH₂Cl₂ (1 mL) under dry nitrogen. The mixture was stirred for 60 minutes at -78°C before the cooling bath was removed and the solution allowed to warm to room temperature, where it was stirred an additional 3 hours. The mixture was poured into ice water and extracted with ethyl acetate (2 x 100 mL). The combined ethyl acetate extracts were washed with brine (50 mL) and dried. Filtration and concentration produced an oil which was flash chromatographed (silica gel, 7:1 hexane-ethyl acetate) to afford 0.3 g (66%) of the title compound as an oil.
Anal. Calcd. for C₂₃H₃₅FN₄O₂:
C, 66.00; H, 8.43; N, 13.39.
Found; C, 66.37; H, 8.60; N, 13.20.
IR (film) 2928, 2856, 1760, 1466, 1266, 695, 406 cm⁻¹.

### (c) 2-Dodecyl-α-fluoro-α-phenyl-2H-tetrazole-5-acetic acid

NaOH (0.12 g, 3 mmole) was added in one portion to a stirred solution of 2-dodecyl-α-fluoro-α-phenyl-2H-tetrazole-5-acetic acid, ethyl ester (0.59 g, 1.4 mmole) dissolved in 6 mL of 5:1 CH₃OH-H₂O at room temperature. After stirring for 3 hours, the mixture was concentrated, diluted with H₂O, acidified with 6N HCl (pH 1) and extracted with ethyl acetate (2 x 150 mL). The combined ethyl acetate extracts were washed with brine (50 mL) and dried. Filtration and concentration afforded 0.5 g (91%) of the title compound as an oil.

### (d) (±)-N-[2,6-Bis(1-methylethyl)phenyl]-2-dodecyl-α-fluoro-α-phenyl-2H-tetrazole-5-acetamide

Oxalyl chloride (0.08 mL, 0.92 mmole) was added to a stirred solution of 2-dodecyl-α-fluoro-α-phenyl-2H-tetrazole-5-acetic acid (0.24 g, 0.61 mmole) in 5 mL of CH₂Cl₂ at room temperature. The mixture was stirred 60 minutes, the one drop of DMF was added (immediate gas evolution). The solution was stirred overnight, concentrated (rotovap), toluene was added, and the solution concentrated again. The residue was dissolved in CH₂Cl₂ (3 mL) and added to a stirred solution of 2,6-diisopropylaniline (0.12 mL, 0.61 mmole) and Et₃N (0.14 mL, 1.0 mmole) in CH₂Cl₂ (2 mL) cooled to 0°C under dry nitrogen. After 20 minutes, the ice bath was removed and the solution allowed to warm to room temperature and stirred for 3 days. The mixture was then diluted with ethyl acetate (150 mL) and washed with dilute HCl (50 mL), bicarbonate (50 mL), brine (50 mL), and dried. Filtration and concentration afforded an oil which was flash chromatographed (silica gel, 10:1 hexanes-ethyl acetate) to produce 150 mg of the title compound as an oil which solidified on standing.
¹H NMR (200 MHz) 7.97 (m, 1H), 7.76 (m, 2H), 7.46 (m, 2H), 7.10 (m, 3H) 4.63 (t, 2H, J = 7 Hz), 3.03 (m, 2H), 2.05 (m, 2H), 1.25 (m, 18H), 1.10 (m, 12H), 0.88 (m, 3H) ppm.

When in the procedure of Example 65(d) an appropriate amount of 2,4,6-trimethoxyaniline was substituted for 2,6-diisopropylaniline the following Example 66 was obtained.

### EXAMPLE 66

### (±)-2-Dodecyl-α-fluoro-α-phenyl-N-(2,4,6-trimethoxy phenyl)-2H-tetrazole-5-acetamide

¹H NMR 7.75 (m, 3H), 7.44 (m, 2H), 6.13 (s, 2H), 4.62 (t, 2H, J = 7.5 Hz), 3.80 (s, 3H), 3.76 (s, 6H), 2.04 (m, 2H), 1.25 (m, 18H), 0.88 (m, 3H) ppm,
mp 82°C-83°C.

### EXAMPLE 67

### Synthesis of 5-decyl-1H-tetrazole

A mixture of n-cyanodecane (20.0 g, 0.12 mol), sodium azide (8.57 g, 0.132 mol), and ammonium chloride (12.8 g, 0.24 mol) in 100 mL DMF was heated to 90°C for 72 hours. Concentrated in vacuo to one-half original volume and acidified to pH 3.0 with 1N HCl. Concentrated again and partitioned the resulting oily white solid between ethyl acetate and water. The organic layer was dried over magnesium sulfate, filtered, and concentrated in vacuo to give an oily solid. Triturated with ice-cold hexanes to give the title compound (15.53 g, 69%), mp 57-59°C.

### EXAMPLE 68

### Synthesis of 5-dodecyl-1H-tetrazole

When in the general procedure of Example 67 an appropriate amount of n-cyanododecane was substituted for n-cyanodecane, the title compound was obtained, mp 68-70°C.

### EXAMPLE 69

### Synthesis of 5-(diphenylmethyl)-1H-tetrazole

Tributyltin azide (51.55 g, 0.155 mol) and diphenyl acetonitrile (20.0 g, 0.103 mol) were mixed in 400 mL dioxane and heated to reflux for 20 hours. Concentrated in vacuo and redissolved the residue in ether. HCl(g) was bubbled through the solution for 1 hour and the resulting precipitate was collected and washed with hexanes to give the HCl salt of the title compound (15.88 g, 58%), mp 156-160°C.

### EXAMPLE 70

### Synthesis of 5-(dodecylthio)-1H-tetrazole

When in the general procedure of Example 69 an appropriate amount of n-dodecylthiocyanate was substituted for diphenyl acetonitrile, the title compound was obtained, mp 85-87°C.

### EXAMPLE 71

### Synthesis of ethyl(±)-5-decyl-α-phenyl-2H-tetrazole-2-acetate

The 5-decyl-1H-tetrazole, (4.0 g, 0.019 mol), triethylamine (2.9 mL, 0.021 mol) and ethyl 2-bromophenylacetate (5.09 g, 0.021 mol) were dissolved in 200 mL acetonitrile and heated to reflux for 2 hours. Cooled and concentrated in vacuo to give a yellow oil. Chromatography to separate the regioisomers gave ethyl (±)-5-decyl-α-phenyl-2H-tetrazole-2-acetate as a clear oil (2.40 g, 34%). ¹H NMR (CDCl₃): δ 7.44-7.28 (m, 5H), 6.43 (s, 1H), 4.37-4.30 (q, 2H), 2.82-2.69 (m, 1H), 2.62-2.49 (m, 1H), 1.73-1.48 (m, 2H), 1.32-1.21 (m, 14H), and 0.90-0.85 (t, 3H) ppm.

### EXAMPLE 72

### Synthesis of ethyl 5-decyl-2H-tetrazole-2-acetate and ethyl 4-decyl-1H-tetrazole-1-acetate

When in the general procedure of Example 71 an appropriate amount of ethyl bromoacetate was substituted for ethyl 2-bromophenylacetate, ethyl 5-decyl-2H-tetrazole-2-acetate was obtained.
¹H NMR (CDCl₃): δ 5.37 (s, 2H), 4.31-4.23 (q, 2H), 2.94-2.88 (t, 2H), 1.82-1.74 (m, 2H), 1.40-1.22 (m, 14H), and 0.90-0.85 (t, 3H) ppm.

Also isolated the 1,5-regioisomer ethyl 5-decyl-1H-tetrazole-1-acetate.
¹H NMR (CDCl₃): δ 5.10 (s, 2H), 4.32-4.23 (q, 2H), 2.82-2.76 (t, 2H), 1.90-1.78 (m, 2H): 1.42-1.19 (m, 14H), and 0.90-0.85 (t, 3H) ppm.

### EXAMPLE 73

### Synthesis of ethyl (±)-5-(dodecylthio)-α-phenyl-2H-tetrazole-2-acetate

When in the general procedure of Example 71 an appropriate amount of 5-(dodecylthio)-1H-tetrazole was substituted for 5-decyl-1H-tetrazole, the title compound was obtained.
¹H NMR (CDCl₃): δ 7.57-7.42 (m, 5H), 6.57 (s, 1H), 4.34-4.23 (q, 2H), 3.20-3.14 (t, 2H): 1.78-1.66 (m, 2H), 1.43-1.22 (m, 14H), 0.90-0.85 (t, 3H) ppm.

### EXAMPLE 74

### Synthesis of ethyl(±)-5-(diphenylmethyl)-α-phenyl-2H-tetrazole-2-acetate

When in the general procedure of Example 71 an appropriate amount of 5-(diphenylmethyl)-1H-tetrazole was substituted for 5-decyl-1H-tetrazole, the title compound was obtained.
¹H NMR (CDCl₃): δ 7.57-7.20 (m, 15H), 6.61 (s, 1H), 5.83 (s, 1H), 4.34-4.15 (m, 2H), 1.22-1.16 (t, 3H) ppm.

### EXAMPLE 75

### Synthesis of ethyl(±)-5-dodecyl-α-phenyl-2H-tetrazole-2-acetate

When in the general procedure of Example 71 an appropriate amount of 5-dodecyl-1H-tetrazole was substituted for 5-decyl-1H-tetrazole, the title compound was obtained.
¹H NMR (CDCl₃): δ 7.58-7.24 (m, 5H), 6.59 (s, 1H), 4.34-4.21 (m, 2H), 2.91-2.85 (t, 2H), 1.82-1.66 (m, 2H), 1.31-1.21 (m, 18H), 0.90-0.85 (t, 3H) ppm.

### EXAMPLE 76

### Synthesis of ethyl 5-dodecyl-2H-tetrazole-2-acetate

When in the general procedure of Example 71 an appropriate amount of 5-dodecyl-1H-tetrazole was substituted for 5-decyl-1H-tetrazole and an appropriate amount of ethyl bromoacetate was substituted for ethyl 2-bromophenylacetate, the title compound was obtained, mp 38-40°C.

### EXAMPLE 77

### Synthesis of ethyl(±)-5-dodecyl-α-pentyl-2H-tetrazole-2-acetate

When in the general procedure of Example 71 an appropriate amount of 5-dodecyl-1H-tetrazole was substituted for 5-decyl-1H-tetrazole and an appropriate amount of ethyl-2-bromoheptanoate was substituted for ethyl 2-bromophenylacetate, the title compound was obtained.
¹H NMR (CDCl₃): δ 5.48-5.30 (t, 1H), 4.29-4.04 (q, 2H), 2.95-2.79 (t, 2H), 2.52-2.20 (m, 2H), 1.90-1.60 (m, 2H), 1.42-0.70 (m, 33H) ppm.

### EXAMPLE 78

### Synthesis of ethyl(±)-5-dodecyl-α,α-dimethyl-2H-tetrazole-2-acetate

When in the general procedure of Example 71 an appropriate amount of 5-dodecyl-1H-tetrazole was substituted for 5-decyl-1H-tetrazole and an appropriate amount of ethyl-2-bromoisobutyrate was substituted for ethyl 2-bromophenylacetate, the title compound was obtained.
¹H NMR (CDCl₃) : δ 4.22-4.13 (q, 2H), 2.92-2.86 (t, 2H), 2.01 (s, 6H), 1.81-1.72 (m, 2H), 1.32-1.15 (m, 18H), 0.90-0.85 (t, 3H) ppm.

### EXAMPLE 79

### Synthesis of (±)-5-decyl-α-phenyl-2H-tetrazole-2-acetic acid

Solid NaOH (0.33 g, 0.0084 mol) was added to a solution of ethyl (±)-5-decyl-α-phenyl-2H-tetrazole-2-acetate in 50 mL ethanol (90%). The resulting solution was stirred for 1 hour and concentrated in vacuo. The residue was partitioned between diethyl ether and water and the aqueous layer was acidified with 1N HCl. The acidified aqueous layer was extracted with diethyl ether and this ether layer was dried over MgSO₄, filtered, and evaporated to give the title compound (1.78 g, 92%), mp 62-64°C.

### EXAMPLE 80

### Synthesis of 5-decyl-2H-tetrazole-2-acetic acid

When in the general procedure of Example 79 an appropriate amount of ethyl 5-decyl-2H-tetrazole-2-acetate was substituted for ethyl (±)-5-decyl-α-phenyl-2H-tetrazole-2-acetate, the title compound was obtained, mp 83-86°C.

### EXAMPLE 81

### Svnthesis of 5-decyl-1H-tetrazole-1-acetic acid

When in the general procedure of Example 79 an appropriate amount of ethyl 5-decyl-1H-tetrazole-1-acetate was substituted for ethyl (±)-5-decyl-α-phenyl-2H-tetrazole-2-acetate, the title compound was obtained, mp 104-106°C.

### EXAMPLE 82

### Synthesis of (±)-5-(diphenylmethyl)-α-phenyl-2H-tetrazole-2-acetic acid

When in the general procedure of Example 79 an appropriate amount of ethyl (±)-5-(diphenylmethyl)-α-phenyl-2H-tetrazole-2-acetate was substituted for ethyl (±)-5-decyl-α-phenyl-2H-tetrazole-2-acetate, the title compound was obtained, mp 158-161°C.

### EXAMPLE 83

### Synthesis of 5-dodecyl-2H-tetrazole-2-acetic acid

When in the general procedure of Example 79 an appropriate amount of ethyl 5-dodecyl-2H-tetrazole-2-acetate was substituted for ethyl (±)-5-decyl-α-phenyl-2H-tetrazole-2-acetate, the title compound was obtained, mp 89-91°C.

### EXAMPLE 84

### Svnthesis of (±)-5-dodecyl-α-phenyl-2H-tetrazole-2-acetic acid

When in the general procedure of Example 79 an appropriate amount of ethyl (±)-5-dodecyl-α-phenyl-2H-tetrazole-2-acetate was substituted for ethyl (±)-5-decyl-α-phenyl-2H-tetrazole-2-acetate, the title compound was obtained, mp 76-78°C.

### EXAMPLE 85

### Synthesis of (±)-5-dodecyl-α-pentyl-2H-tetrazole-2-acetic acid

When in the general procedure of Example 79 an appropriate amount of ethyl (±)-5-dodecyl-α-pentyl-2H-tetrazole-2-acetate was substituted for ethyl (±)-5-decyl-α-phenyl-2H-tetrazole-2-acetate, the title compound was obtained.
¹H NMR (CDCl₃): δ 9.24 (bs, 1H), 5.54-5.48 (t, 1H), 2.94-2.88 (t, 2H), 2.54-2.30 (m, 2H), 1.81-1.75 (m, 2H), 1.30-1.25 (m, 24H), 0.90-0.86 (t, 6H) ppm.

### EXAMPLE 86

### Synthesis of 5-dodecyl-α,α-dimethyl-2H-tetrazole-2-acetic acid

When in the general procedure of Example 79 an appropriate amount of ethyl (±)-5-dodecyl-α,α-dimethyl-2H-tetrazole-2-acetate was substituted for ethyl (±)-5-decyl-α-phenyl-2H-tetrazole-2-acetate, the title compound was obtained, mp 68-71°C.

### EXAMPLE 87

### Synthesis of (±)-5-(dodecylthio)-α-phenyl-2H-tetrazole-2-acetic acid

When in the general procedure of Example 79 an appropriate amount of ethyl (±)-5-(dodecylthio)-α-phenyl-2H-tetrazole-2-acetate was substituted for ethyl (±)-5-decyl-α-phenyl-2H-tetrazole-2-acetate, the title compound was obtained, mp 64-67°C.

### EXAMPLE 88

### Synthesis of N-[2,6-bis(1-methylethyl)phenyl]-5-decyl-2H-tetrazole-2-acetamide

A solution of 2,6-diisopropyl aniline (0.97 g, 0.006 mol) and 5-decyl-2H-tetrazole-2-acetic acid (1.47 g, 0.006 mol) in 100 mL dichloromethane was cooled to 0°C under an atmosphere of nitrogen. Solid DCC (1.19 g, 0.006 mol) was added in one portion and the resulting suspension was warmed to room temperature and stirred for 16 hours. Concentrated in vacuo and triturated the residue with diethyl ester. Filtered to remove the dicyclohexyl urea byproduct. Concentrated the filtrate and triturated with hexanes to give the title compound (2.02 g, 86%) as an off-white solid, mp 108-110°C.

### EXAMPLE 89

### Synthesis of N-[2,6-bis(1-methylethyl)phenyl]-5-decyl-1H-tetrazole-1-acetamide

When in the general procedure of Example 88 an appropriate amount of 5-decyl-1H-tetrazole-1-acetic acid was substituted for 5-decyl-2H-tetrazole-2-acetic acid, the title compound was obtained, mp 71-73°C.

### EXAMPLE 90

### Synthesis of (±)-N-[2,6-bis(1-methylethyl)phenyl]-5-(diphenylmethyl)-α-phenyl-2H-tetrazole-2-acetamide

When in the general procedure of Example 88 an appropriate amount of (±)-5-(diphenyl-methyl)-α-phenyl-2H-tetrazole-2-acetic acid was substituted for 5-decyl-2H-tetrazole-2-acetic acid, the title compound was obtained, mp 180-183°C.

### EXAMPLE 91

### Synthesis of N-[2,6-bis(1-methylethyl)phenyl-5-dodecyl-2H-tetrazole-2-acetamide

When in the general procedure of Example 88 an appropriate amount of 5-dodecyl-2H-tetrazole-2-acetic acid was substituted for 5-decyl-2H-tetrazole-2-acetic acid, the title compound was obtained, mp 91-93°C.

### EXAMPLE 92

### Synthesis of (±)-N-[2,6-bis(1-methylethyl)phenyl]-5-dodecyl-α-phenyl-2H-tetrazole-2-acetamide

When in the general procedure of Example 88 an appropriate amount of (±)-5-dodecyl-α-phenyl-2H-tetrazole-2-acetic acid was substituted for 5-decyl-2H-tetrazole-2-acetic acid, the title compound was obtained, mp 93-95°C.

### EXAMPLE 93

### Synthesis of (±)-N-[2,6-bis(1-methylethyl)phenyl]-5-dodecyl-α-pentyl-2H-tetrazole-2-acetamide

When in the general procedure of Example 88 an appropriate amount of (±)-5-dodecyl-α-pentyl-2H-tetrazole-2-acetic acid was substituted for 5-decyl-2H-tetrazole-2-acetic acid, the title compound was obtained.
¹H NMR (CDCl₃): δ 7.53 (bs, 1H), 7.33-7.05 (m, 3H), 5.64-5.57 (t, 1H), 2.98-2.92 (t, 2H), 2.47-2.42 (m, 2H), 1.87-1.75 (m, 2H), 1.33-1.09 (m, 24H), 0.90-0.85 (t, 6H) ppm.

### EXAMPLE 94

### Synthesis of (±)-N-[2,6-bis(1-methylethyl)phenyl-5-(dodecylthio)-α-phenyl-2H-tetrazole-2-acetamide

When in the general procedure of Example 88 an appropriate amount of (±)-5-(dodecylthio)-α-phenyl-2H-tetrazole-2-acetic acid was substituted for 5-decyl-2H-tetrazole-2-acetic acid, the title compound was obtained, mp 102-105°C.

### EXAMPLE 95

### Synthesis of (±)-5-decyl-α-phenyl-N-(2,4,6-trimethoxyphenyl-2H-tetrazole-2-acetamide

When in the general procedure of Example 88 an appropriate amount of (±)-5-decyl-α-phenyl-2H-tetrazol-2-acetic acid was substituted for 5-decyl-2H-tetrazole-2-acetic acid and 2,4,6-trimethoxyaniline was substituted for 2,6-diisopropylaniline, the title compound was obtained, mp 145-147°C.

### EXAMPLE 96

### Synthesis of (±)-5-(diphenylmethyl)-α-phenyl-N-(2,4,6-trimethoxyphenyl)-2H-tetrazole-2-acetamide

When in the general procedure of Example 88 an appropriate amount of (±)-5-(diphenylmethyl)-α-phenyl-2H-tetrazole-2-acetic acid was substituted for 5-decyl-2H-tetrazole-2-acetic acid and 2,4,6-trimethoxyaniline was substituted for 2,6-diisopropylaniline, the title compound was obtained,
mp 114-117°C.

### EXAMPLE 97

### Synthesis of 5-dodecyl-N-(2,4,6-trimethoxy-phenyl)-2H-tetrazole-2-acetamide

When in the general procedure of Example 88 an appropriate amount of 5-dodecyl-2H-tetrazole-2-acetic acid was substituted for 5-decyl-2H-tetrazole-2-acetic acid and 2,4,6-trimethoxyaniline was substituted for 2,6-diisopropylaniline, the title compound was obtained, mp 144-146°C.

### EXAMPLE 98

### Synthesis of (±)-5-dodecyl-α-phenyl-N-(2,4,6-trimethoxyphenyl)-2H-tetrazole-2-acetamide

When in the general procedure of Example 88 an appropriate amount of (±)-5-dodecyl-α-phenyl-2H-tetrazole-2-acetic acid was substituted for 5-decyl-2H-tetrazole-2-acetic acid and 2,4,6-trimethoxyaniline was substituted for 2,6-diisopropylaniline, the title compound was obtained, mp 141-145°C.

### EXAMPLE 99

### Synthesis of (±)-5-dodecyl-α-pentyl-N-(2,4,6-trimethoxyphenyl)-2H-tetrazole-2-acetamide

When in the general procedure of Example 88 an appropriate amount of (±)-5-dodecyl-α-pentyl-2H-tetrazole-2-acetic acid was substituted for 5-decyl-2H-tetrazole-2-acetic acid and 2,4,6-trimethoxyaniline was substituted for 2,6-diisopropylaniline, the title compound was obtained, mp 152-155°C.

### EXAMPLE 100

### Synthesis of (±)-N-(2,4-difluorophenyl-5-dodecyl-α-phenyl-2H-tetrazole-2-acetamide

When in the general procedure of Example 88 an appropriate amount of (±)-5-dodecyl-α-phenyl-2H-tetrazole-2-acetic acid was substituted for 5-decyl-2H-tetrazole-2-acetic acid and 2,4-difluoroaniline was substituted for 2,6-diisopropylaniline, the title compound was obtained, mp 62-64°C.

### EXAMPLE 101

### Synthesis of N-(2,4-difluorophenyl)-5-dodecyl-2H tetrazole-2-acetamide

When in the general procedure of Example 88 an appropriate amount of 5-dodecyl-2H-tetrazole-2-acetic acid was substituted for 5-decyl-2H-tetrazole-2-acetic acid and 2,4-difluoroaniline was substituted for 2,6-diisopropylaniline, the title compound was obtained, mp 103-106°C.

### EXAMPLE 102

### Synthesis of 5-dodecyl-α,α-dimethyl-N-(2,4,6-trimethoxyphenyl)-2H-tetrazole-2-acetamide

When in the general procedure of Example 88 an appropriate amount of 5-dodecyl-α,α-dimethyl-2H-tetrazole-2-acetic acid was substituted for 5-decyl-2H-tetrazole-2-acetic acid and 2,4,6-trimethoxyaniline was substituted for 2,6-diisopropylaniline, the title compound was obtained.
¹H NMR (CDCl₃): δ 6.78 (bs, 1H), 6.09 (s, 2H), 3.78 (s, 3H), 3.73 (s, 6H), 2.97-2.91 (t, 2H), 2.11 (s, 6H), 1.90-1.75 (m, 2H), 1.34-1.24 (m, 18H), 0.90-0.85 (t, 3H) ppm.

### EXAMPLE 103

### Synthesis of (±)-5-(dodecylthio)-α-phenyl-N-(2,4,6-trimethoxyphenyl)-2H-tetrazole-2-acetamide

When in the general procedure of Example 88 an appropriate amount of (±)-5-(dodecylthio)-α-phenyl-2H-tetrazole-2-acetic acid was substituted for 5-decyl-2H-tetrazole-2-acetic acid and 2,4,6-trimethoxyaniline was substituted for 2,6-diisopropylaniline, the title compound was obtained, mp 141-143°C.

### EXAMPLE 104

### Synthesis of (±)-5-(dodecylsulfinyl)-α-phenyl-N-(2,4,6)-trimethoxyphenyl)-2H-tetrazole-2-acetamide

Solid m-chloroperbenzoic acid (0.5 g, 0.002 mol) was added in one portion to a solution of (±)-5-(dodecylthio)-α-phenyl-N-(2,4,6-trimethoxy-phenyl)-2H-tetrazole-2-acetamide (1.15 g, 0.002 mol) in 125 mL dichloromethane at 0°C under a nitrogen atmosphere. Stirred for 3 hours and then washed with aqueous Na₂CO₃ solution, dried over MgSO₄, filtered, and concentrated to give a cream colored solid. Washed with solid with boiling hexanes to give the title compound (0.87 g, 74%), mp 140-143°C.

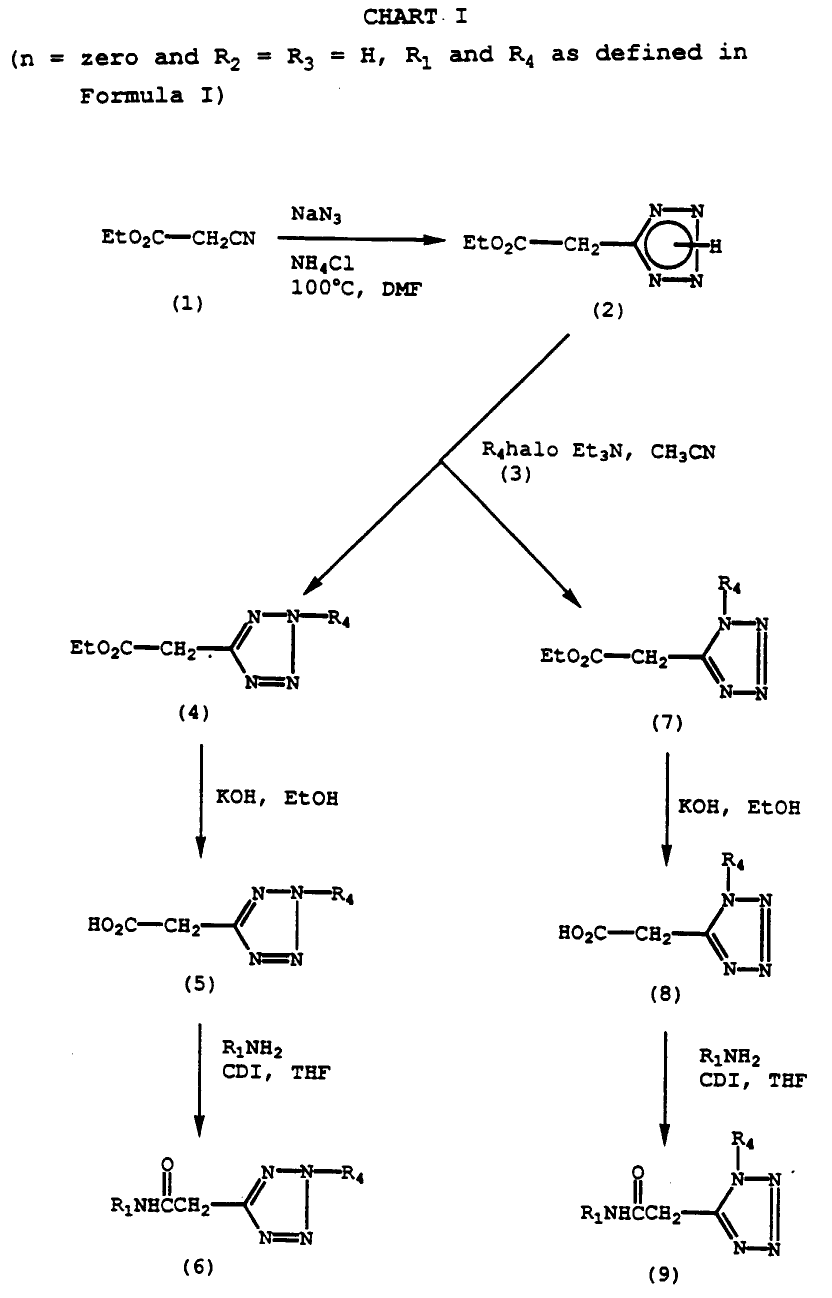

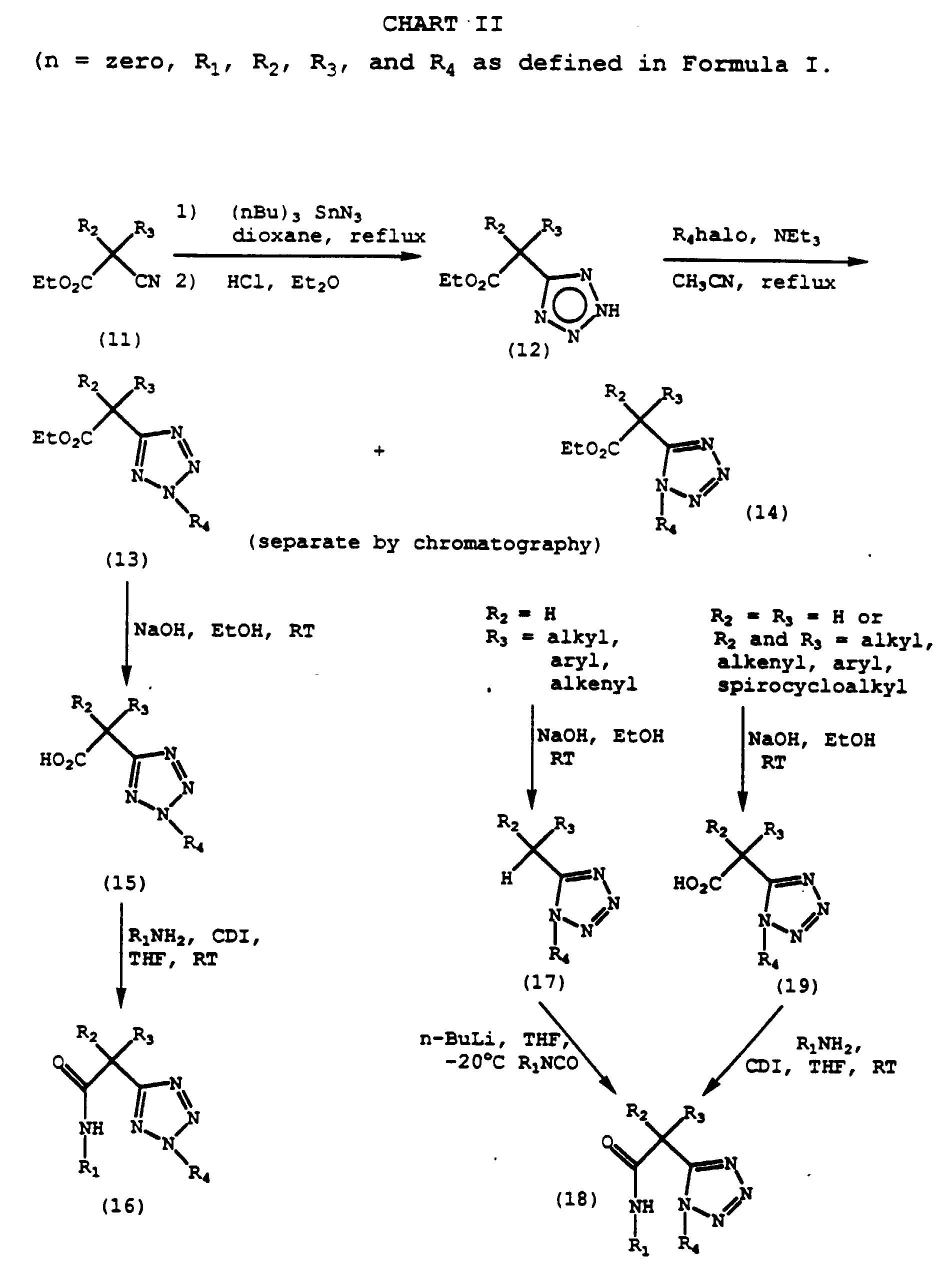

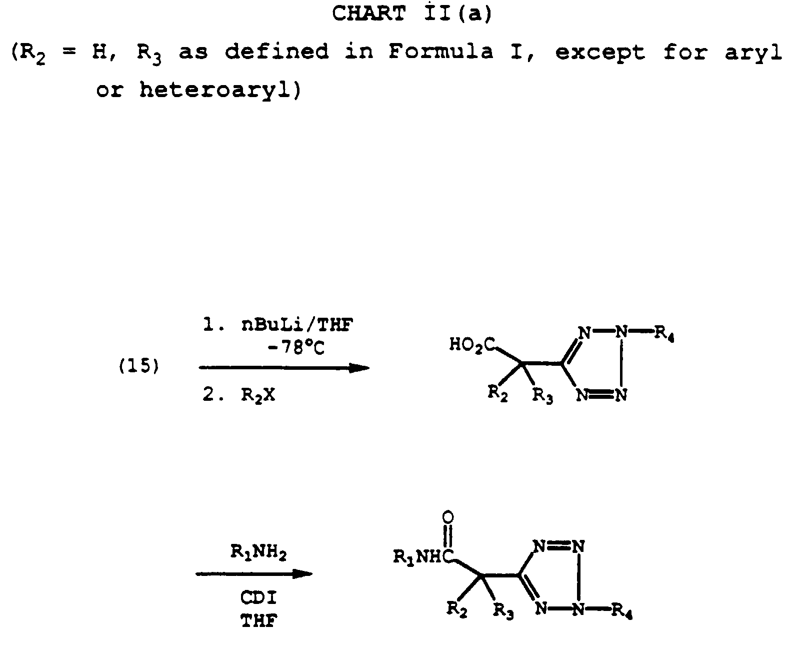

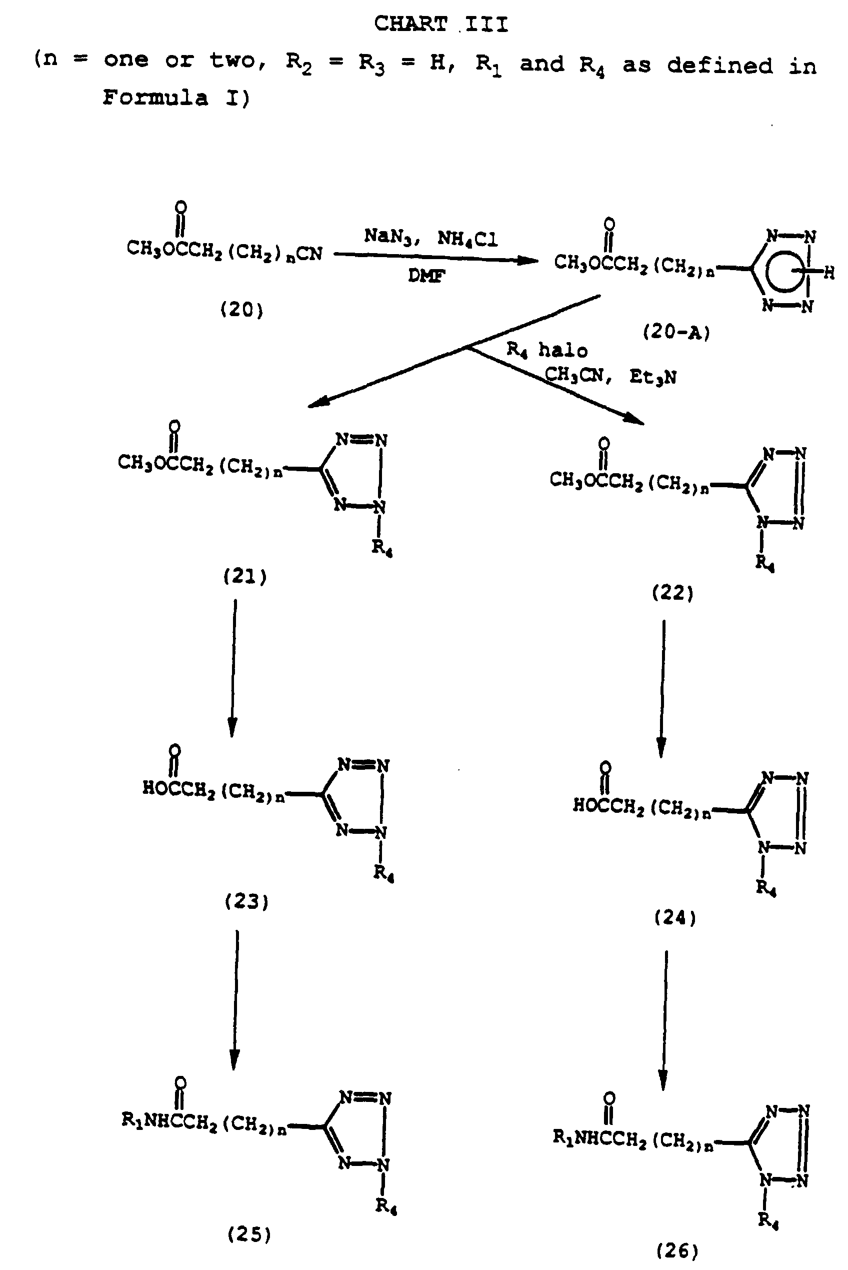

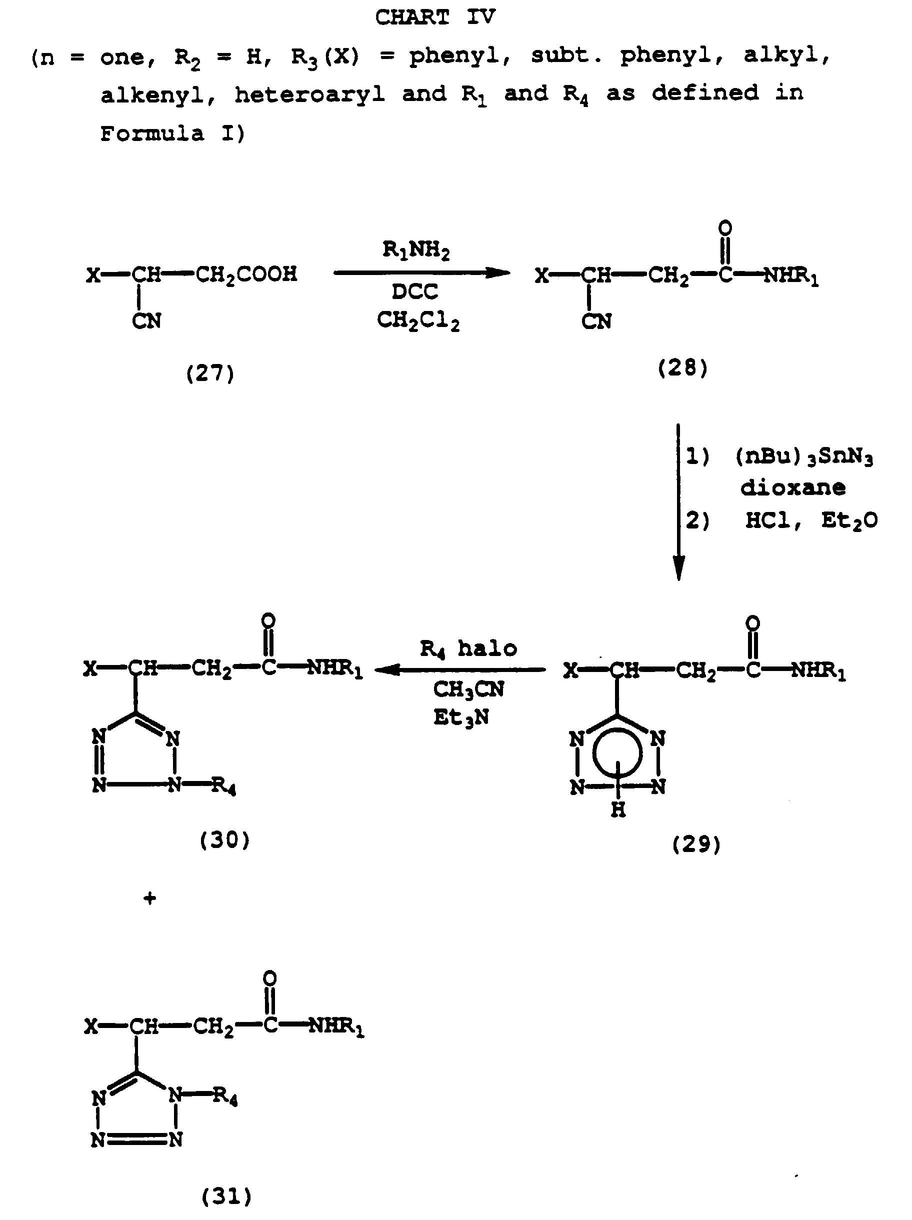

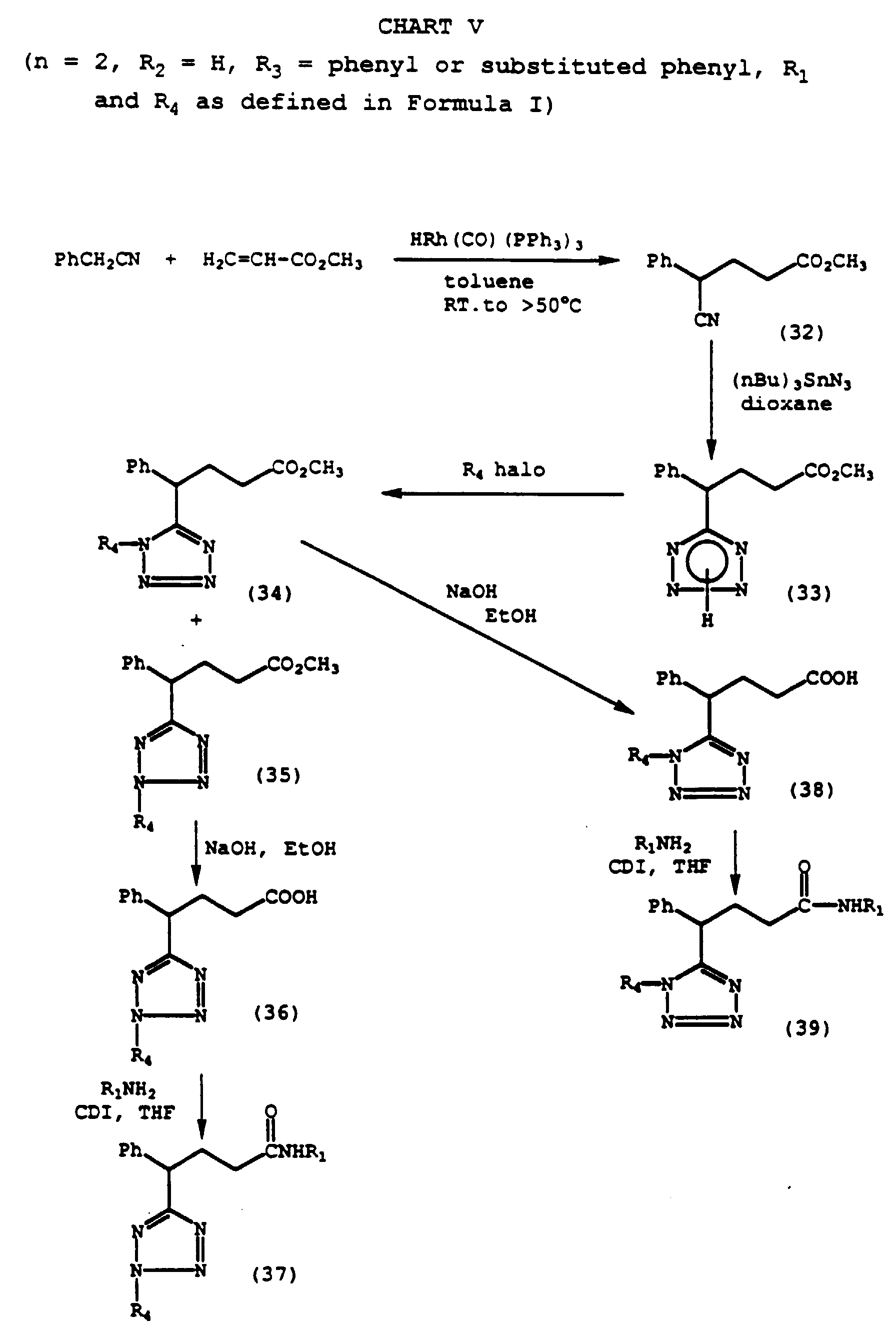

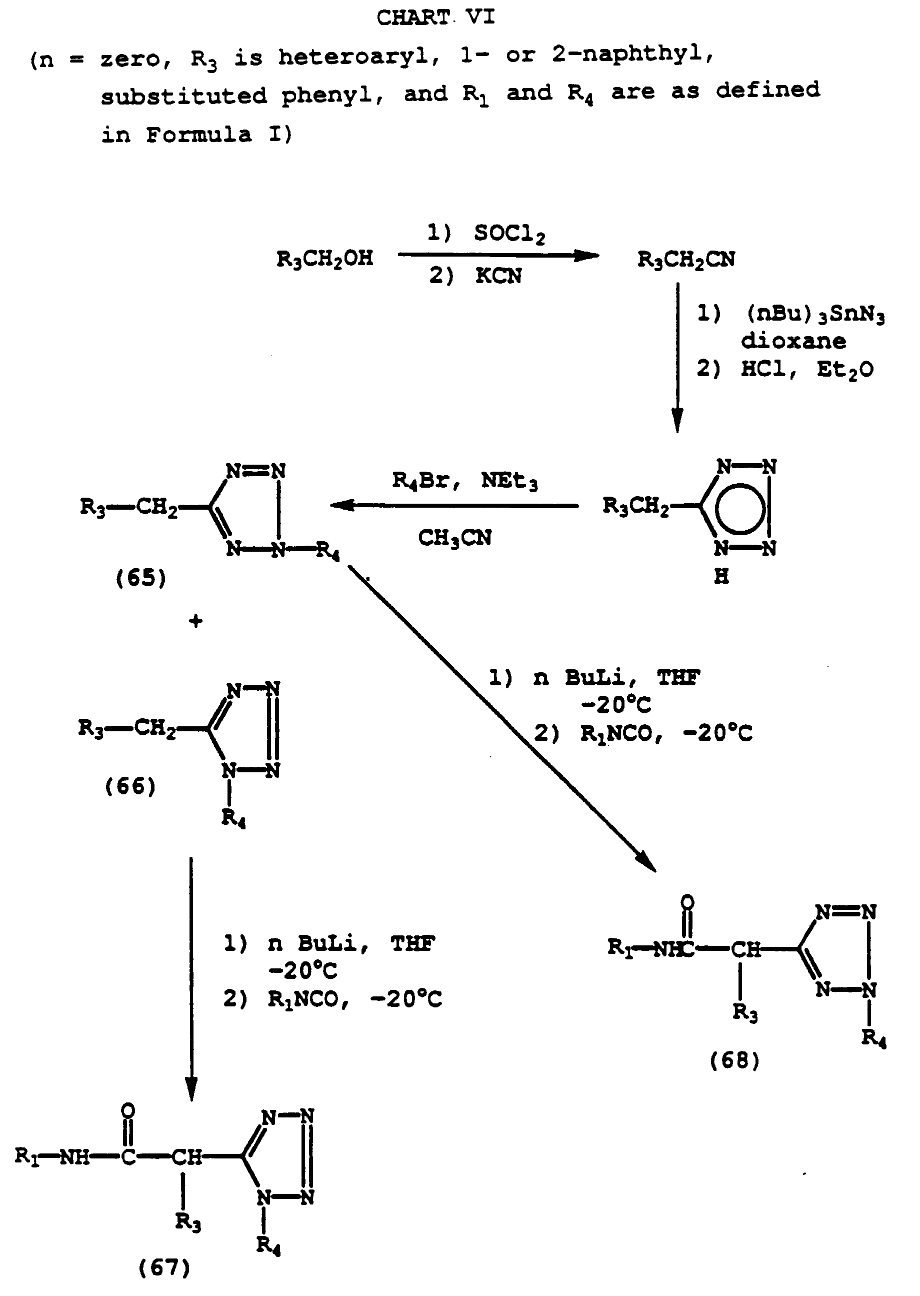

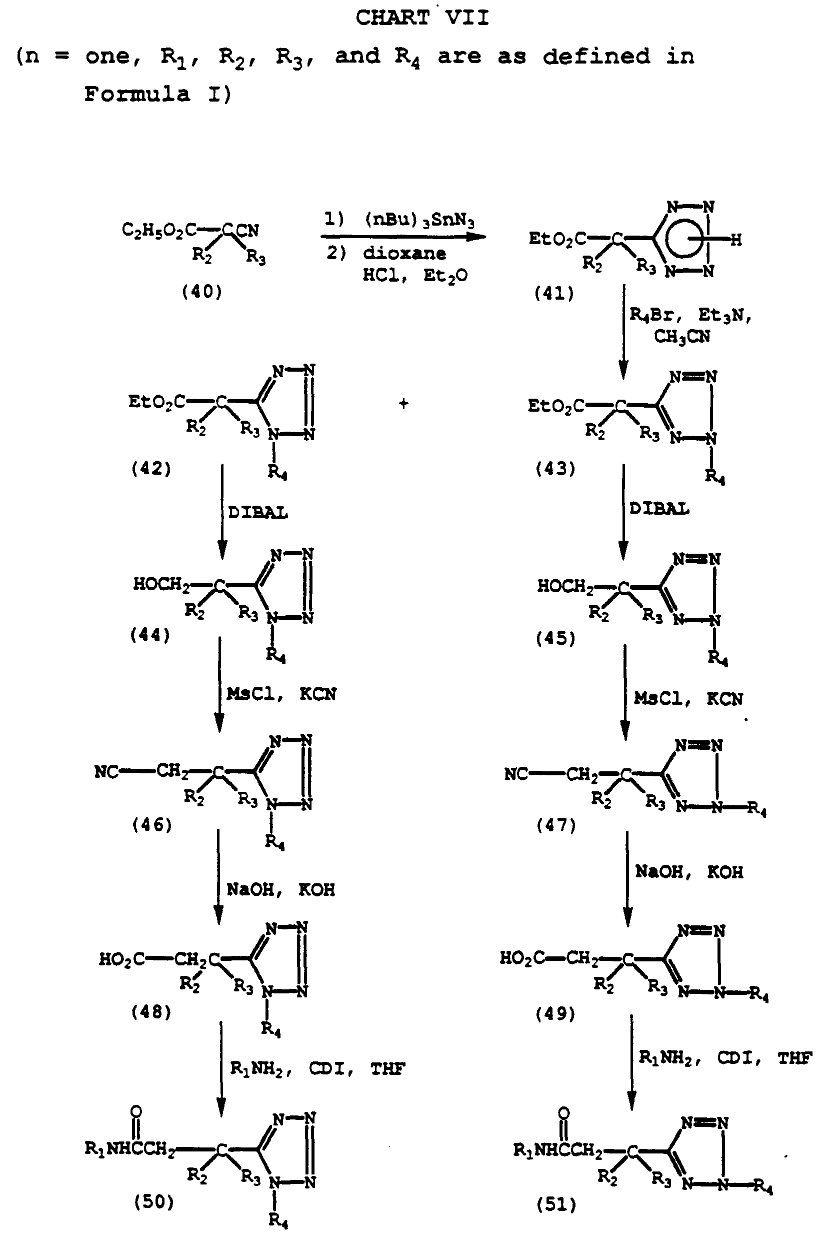

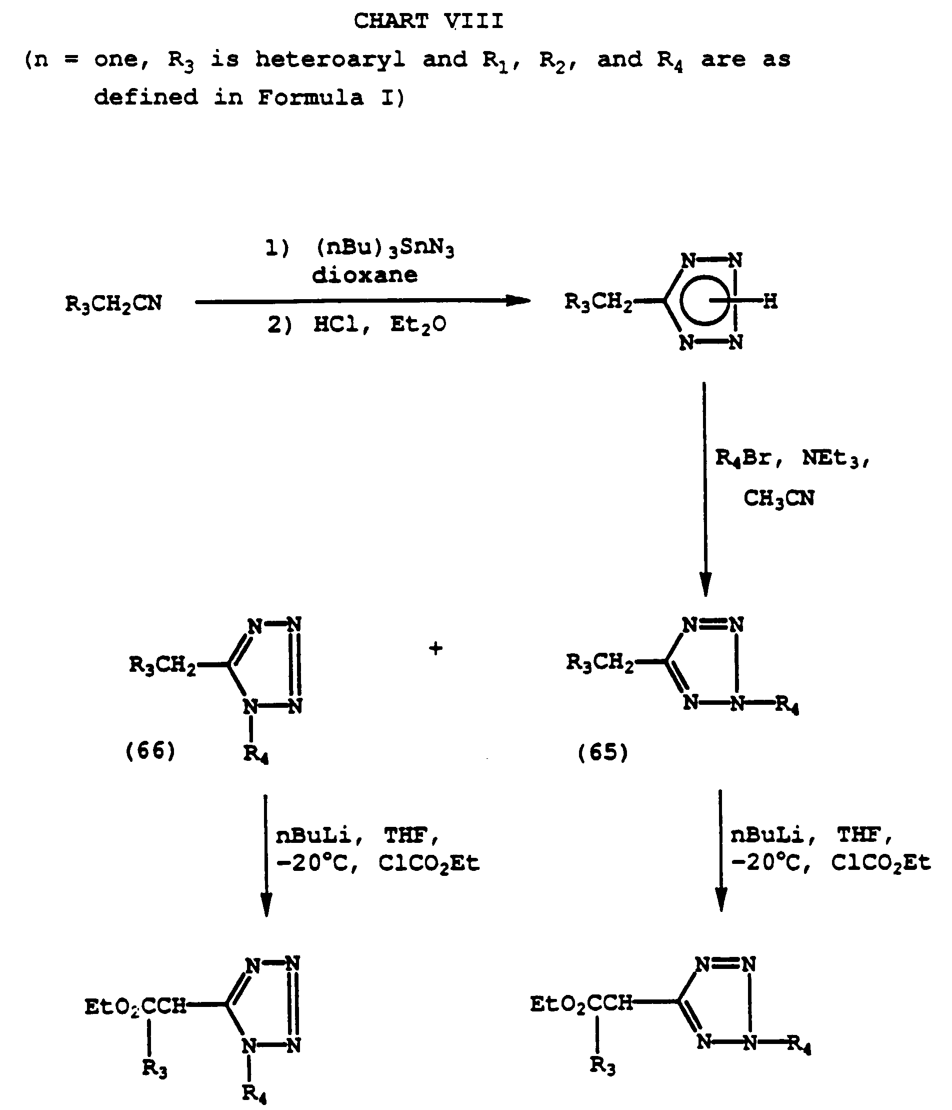

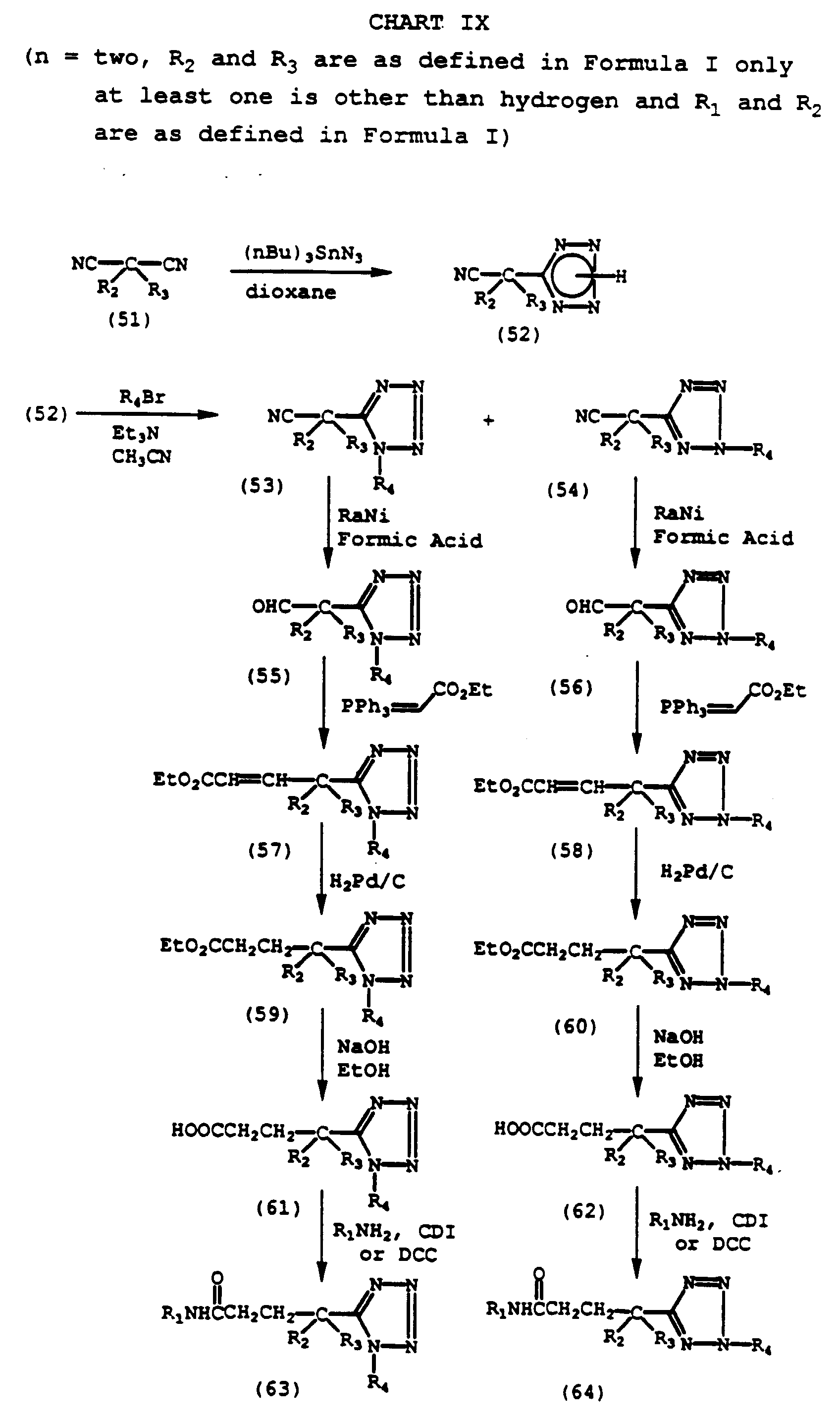

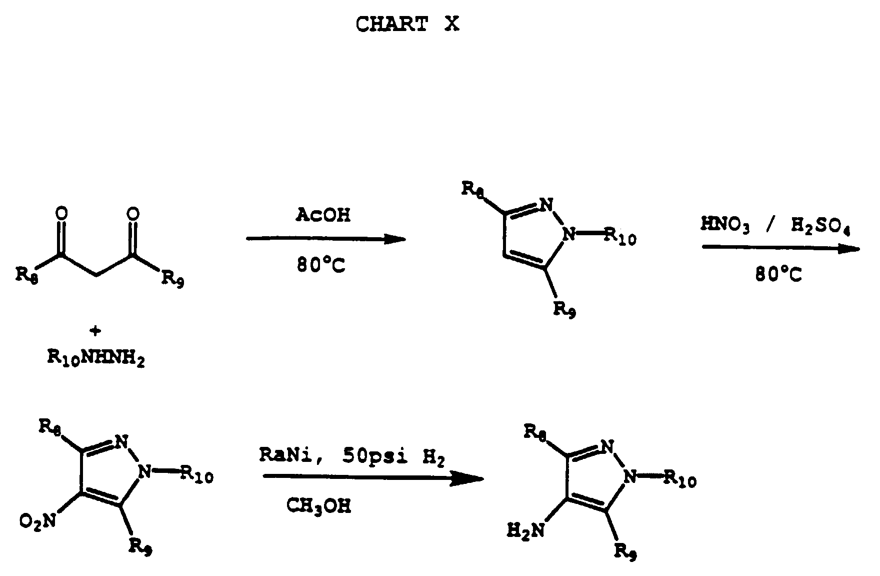

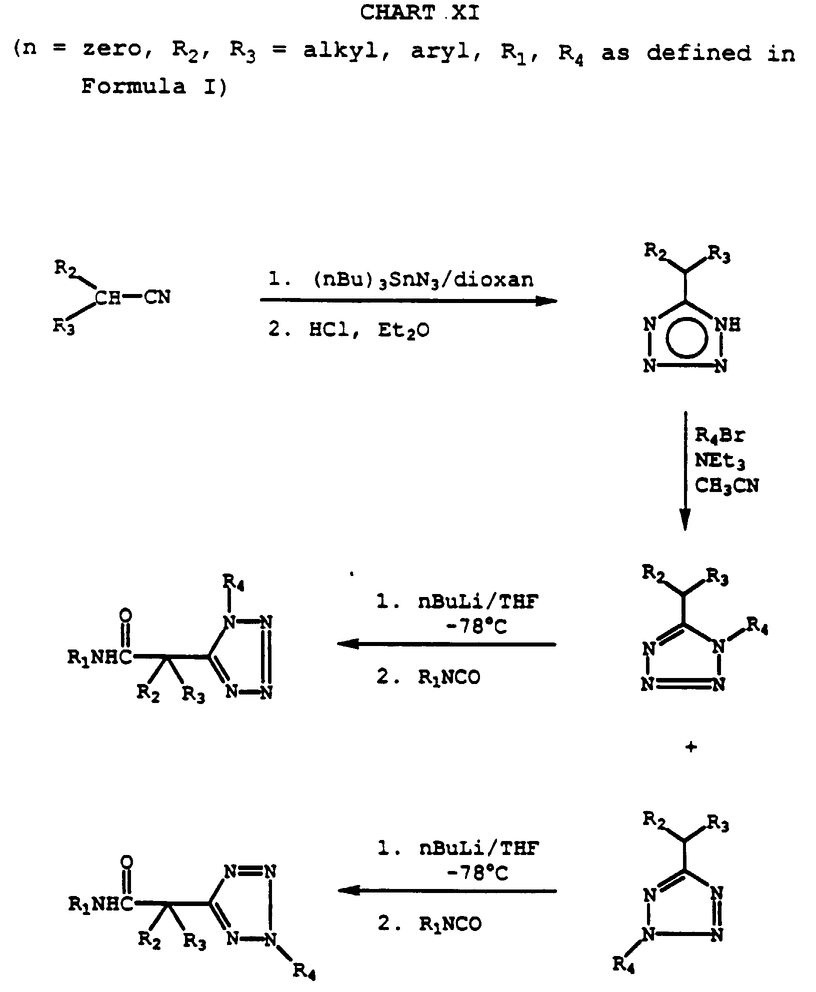

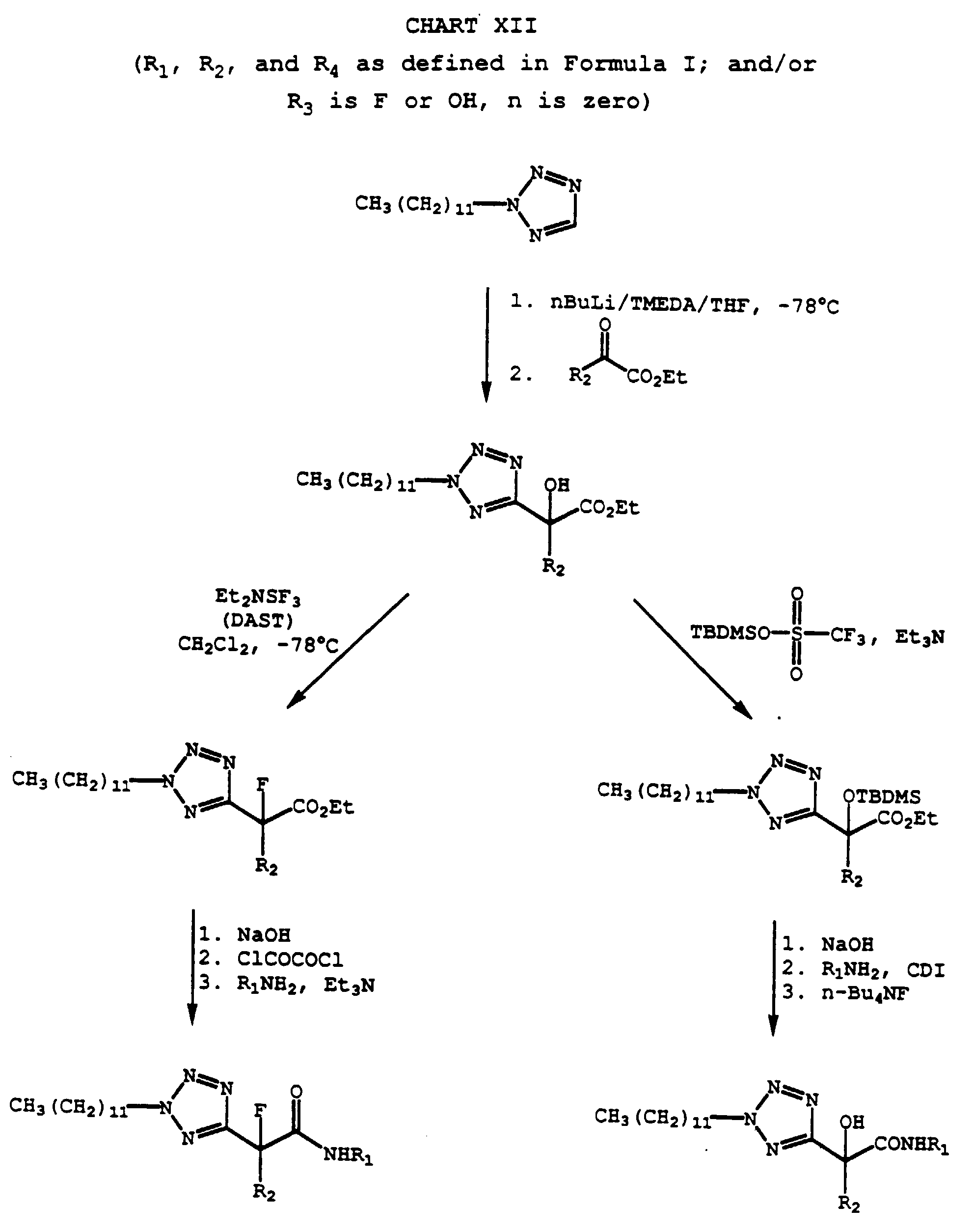

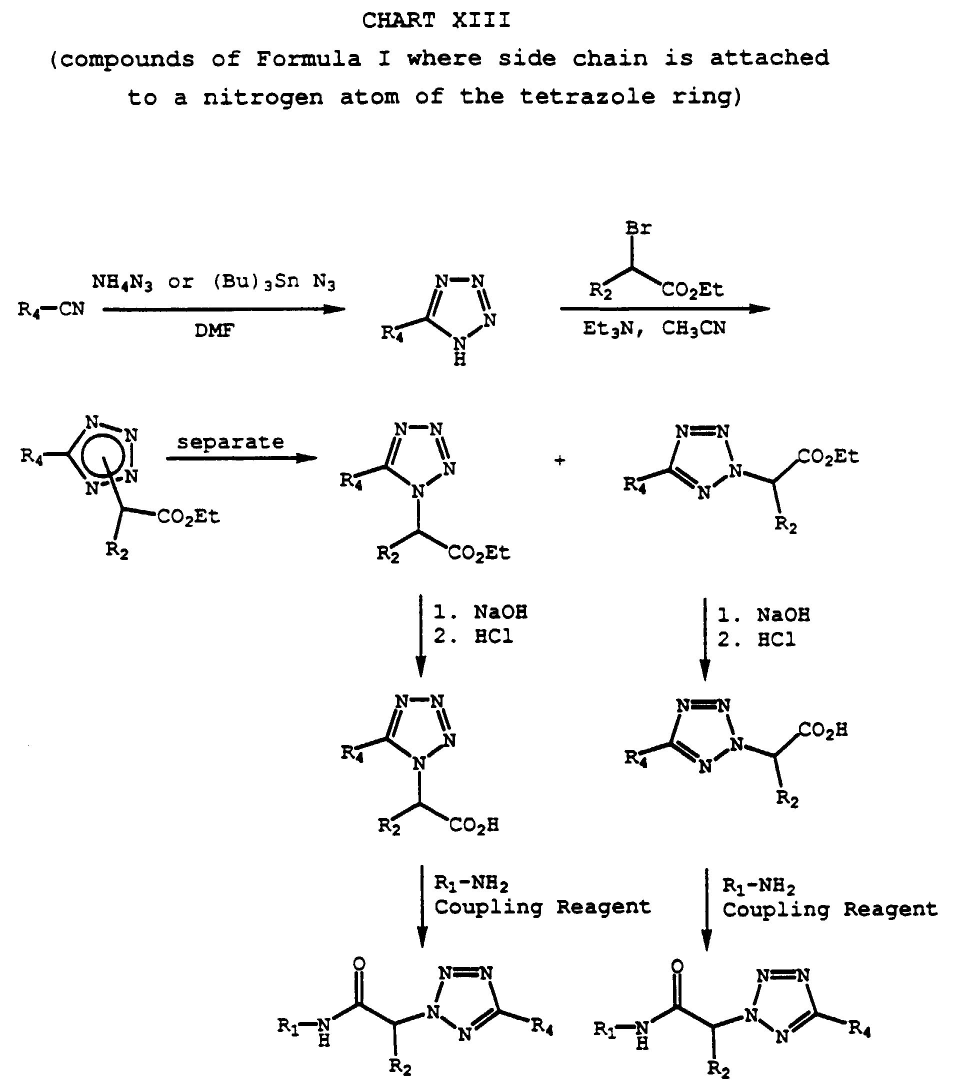

## Claims

1. A compound of the formula 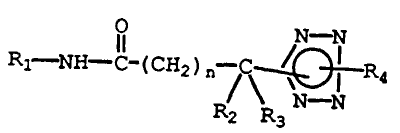 wherein n is zero, one or two;
wherein R₁ is selected from
(a) phenyl which is unsubstituted or is substituted with from one to three substituents selected from:
alkyl having from 1 to 4 carbon atoms and which is straight or branched,
alkoxy having from 1 to 3 carbon atoms and which is straight or branched,
alkylthio having from 1 to 3 carbon atoms and which is straight or branched,
hydroxy,
phenyl,
fluorine,
chlorine,
bromine,
nitro,
cyano,
trifluoromethyl,
-COOH,
-COOalkyl wherein alkyl has from 1 to 4 carbon atoms and which is straight or branched,
-(CH₂)ₘNR₅R₆ wherein m is zero or one, and each of R₅ and R₆ is hydrogen or a straight or branched alkyl group having 1 to 4 carbon atoms;
(b) 1- or 2-naphthyl which is unsubstituted or substituted with one to three substituents selected from:
alkyl having from 1 to 4 carbon atoms and which is straight or branched,
alkoxy having from 1 to 3 carbon atoms and which is straight or branched,
hydroxy,
fluorine,
chlorine,
bromine,
nitro,
cyano,
trifluoromethyl,
-COOH,
-COOalkyl wherein alkyl has from 1 to 4 carbon atoms and is straight or branched,
-(CH₂)ₘNR₅R₆ wherein m, R₅, and R₆ have the meanings defined above;
(c) the group 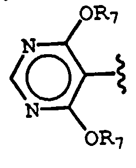 wherein R₇ is a lower alkyl group having from 1 to 3 carbon atoms and is straight or branched;
(d) the group 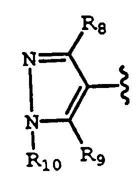 wherein R₈ and R₉ are straight or branched alkyl having from 1 to 4 carbon atoms or phenyl, and R₁₀ is a straight or branched hydrocarbon group having from 1 to 18 carbon atoms which is saturated or is unsaturated containing one double bond or two nonadjacent double bonds; phenyl; phenyl substituted with from one to three substituents selected from straight or branched alkyl having 1 to 4 carbon atoms, straight or branched alkoxy having from 1 to 3 carbon atoms, hydroxy, fluorine, chlorine, bromine, nitro, cyano, trifluoromethyl, -COOH, -COOalkyl wherein alkyl has from 1 to 4 carbon atoms and is straight or branched or (CH₂)ₘNR₅R₆ wherein m, R₅, and R₆ are as defined above; or a heterocyclic group selected from 2-, 3-, or 4-pyridyl, 2-, 4-, or 5-pyrimidinyl, 2- or 3-pyrazinyl, 2-, 3-, 4-, 5-, 6-, 7-, or 8-quinolinyl, or 3- or 4-pyridazinyl and the N-oxides thereof;
(e) the group 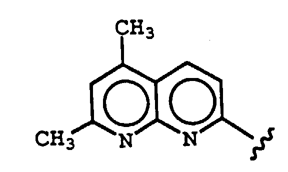
(f) the group 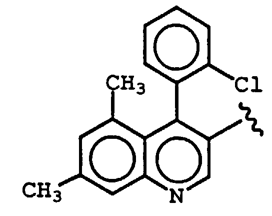
(g) a straight or branched hydrocarbon group having from 1 to 18 carbon atoms which is saturated or is unsaturated containing one double bond or two nonadjacent double bonds;
(h) a cycloalkyl group having from 3 to 8 carbon atoms;
(i) a heteroaromatic group selected from 2-, 3-, or 4-pyridyl which is unsubstituted or substituted with an alkyl group having from 1 to 4 carbon atoms or 2-, 4-, or 5-pyrimidinyl, and the N-oxides thereof;
(j) the group 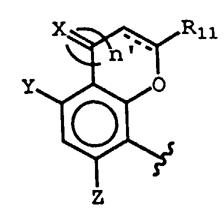 wherein --- denotes a single or double bond;
Y and Z are each independently hydrogen, a straight or branched alkyl group of 1 to 4 carbon atoms, an alkoxy group of 1 to 3 carbon atoms or halo;
X is oxygen or two hydrogen atoms;
R₁₁ is hydrogen or a straight or branched alkyl group of 1 to 4 carbon atoms, and n' is zero or one; or
(k) is selected from the group 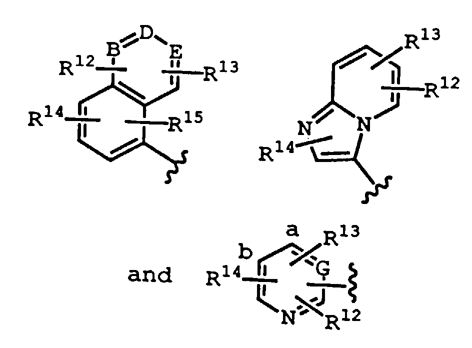 wherein R¹², R¹³, R¹⁴, and R¹⁵ are each independently hydrogen, halo, a straight or branched alkyl group of 1 to 4 carbon atoms, an alkoxy group of 1 to 3 carbon atoms, and alkylthio group of 1 to 3 carbon atoms, cycloaklylthio of five to seven carbon atoms, phenylalkylthio in which alkyl is 1 to 4 carbon atoms, substituted phenylthio, heteroarylthio, or heteroaryloxy; and B, D, E, and G are nitrogen or carbon where one or more of B, D, and E is nitrogen; with the proviso that when G = nitrogen the group is attached to the nitrogen atom of formula I at the 4- or 5-position of the pyrimidine ring (a and b);
wherein R₂ and R₃ are the same or different and are selected from:
(a) hydrogen, halo or one of R₂ or R₃ is hydroxy;
(b) a straight or branched alkyl group having from 1 to 12 carbon atoms, or a cycloalkyl group having from 3 to 8 carbon atoms;
(c) a phenyl or phenylalkyl group where alkyl is from 1 to 4 carbon atoms and which the phenyl ring unsubstituted or substituted with from 1 to 3 substituents selected from straight or branched alkyl having from 1 to 4 carbon atoms, straight or branched alkoxy having from 1 to 4 carbon atoms, alkythio, straight or branched having 1 to 4 carbon atoms, hydroxy, fluorine, chlorine, bromine, trifluoromethyl, cyano, nitro, phenyl, or (CH₂)ₘNR₅R₆ wherein m, R₅, and R₆ have the meanings defined above;
(d) a straight or branched alkenyl group having from 2 to 6 carbon atoms; or
(e) R₂ and R₃ taken together with the carbon atom to which they are attached form an alkylidene group of 1 to 4 carbon atoms, a benzylidene or a spiroalkyl group having from 3 to 7 carbon atoms; or
(f) when R₂ is hydrogen, F, alkyl of C₁₋₁₂ atoms, R₃ is a heteroaryl selected from a 5- or 6-membered monocyclic or fused bicyclic heterocyclic group containing at least 1 to 4 heteroatoms in at least one ring, said heteroatoms being nitrogen, oxygen, or sulfur and combinations thereof, said heterocyclic group being unsubstituted or substituted with an alkyl group having from 1 to 4 carbon atoms and the N-oxides thereof;
(g) 1- or 2-naphthyl which is unsubstituted or substituted with one to three substituents selected from:
alkyl having from 1 to 4 carbon atoms and which is straight or branched,
alkoxy having from 1 to 3 carbon atoms and which is straight or branched,
wherein R₄ is a straight or branched hydrocarbon chain having from 1 to 20 carbon atoms and is saturated or is unsaturated and has 1 double bond or has 2 nonadjacent double bonds or is alkylthio having 1 to 20 carbon atoms and is saturated; or a pharmaceutically acceptable salt or individual enantiomeric isomer thereof.

2. A compound of Claim 1 wherein R₄ is in the 2-position of the tetrazole ring and the side chain is attached to the carbon atom of the tetrazole ring.

3. A compound of Claim 2 wherein n is zero.

4. A compound of Claim 3 wherein each of R₂ and R₃ is hydrogen.

5. A compound of Claim 4 wherein R₄ is a saturated hydrocarbon chain and has from 8 to 18 carbon atoms.

6. A compound of Claim 5 wherein R₁ is phenyl or substituted phenyl.

7. A compound of Claim 6 which is
N-[2,6-Bis(1-methylethyl)phenyl]-2-dodecyl-2H-tetrazole-5-acetamide;
2-Dodecyl-N-(2,4,6-trimethoxyphenyl)-2H-tetrazole-5-acetamide;
N-(2,4-Difluorophenyl)-2-dodecyl-2H-tetrazole-5-acetamide;
2-tetradecyl-N-(2,4,6-tri-methoxyphenyl)-2H-tetrazole-5-acetamide.

8. A compound of Claim 5 wherein R₁ is the group 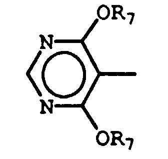 wherein R₇ is a lower alkyl group having from 1 to 3 carbon atoms and is straight or branched.

9. A compound of Claim 8 which is
N-(4,6-dimethoxy-5-pyrimidinyl)-2-dodecyl-2H-tetrazole-5-acetamide; or
N-(4,6-dimethoxy-5-pyrimidinyl)-2-dodecyl-1H-tetrazole-5-acetamide.

10. A compound of Claim 5 wherein R₁ is a heteroaromatic group selected from 2-, 3-, or 4-pyridyl which is unsubstituted or substituted with an alkyl group having from 1 to 4 carbon atoms, or 2-, 4-, or 5-pyrimidinyl and the N-oxides thereof.

11. A compound of Claim 10 which is
2-dodecyl-N-(3-methyl-2-pyridinyl)-2H-tetrazole-5-acetamide.

12. A compound of Claim 5 wherein R₁ is the group 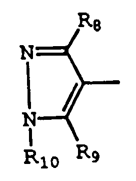 wherein R₈ and R₉ are straight or branched alkyl having from 1 to 4 carbon atoms or phenyl, and R₁₀ is a straight or branched hydrocarbon group having from 1 to 18 carbon atoms which is saturated or is unsaturated containing one double bond or two nonadjacent double bonds; phenyl; phenyl substituted with from one to three substituents selected from straight or branched alkyl having 1 to 4 carbon atoms, straight or branched alkoxy having from 1 to 3 carbon atoms, hydroxy, fluorine, chlorine, bromine, nitro, cyano, trifluoromethyl, -COOH, -COOalkyl wherein alkyl has from 1 to 4 carbon atoms and is straight or branched or (CH₂)ₘNR₅R₆ wherein m, R₅, and R₆ are as defined above; or a heterocyclic group selected from 2-, 3-, or 4-pyridyl, 2-, 4-, or 5-pyrimidinyl, 2- or 3-pyrazinyl, 2-, 3-, 4-, 5-, 6-, 7-, or 8-quinolinyl, or 3- or 4-pyridazinyl and the N-oxides thereof.

13. A compound of Claim 12 which is
2-dodecyl-N-(1,3,5-trimethyl-1H-pyrazol-4-yl)-2H-tetrazole-5-acetamide; or
1-dodecyl-N-(1,3,5-trimethyl-1H-pyrazol-4-yl)-1H-tetrazole-5-acetamide.

14. A compound of Claim 3 wherein one of R₂ and R₃ is hydrogen and the other is phenyl which is unsubstituted or substituted.

15. A compound of Claim 14 wherein R₄ is saturated hydrocarbon chain having from 8 to 18 carbon atoms.

16. A compound of Claim 15 wherein R₁ is a phenyl group which is unsubstituted or is substituted.

17. A compound of Claim 16 which is
(±) 2-dodecyl-α-phenyl-N-(2,4,6-trimethoxyphenyl)-2H-tetrazole-5-acetamide,
(±) 2-dodecyl-N,α-diphenyl-2H-tetrazole-5-acetamide,
(±)-N-[2,6-bis(1-methylethyl)phenyl]-2-dodecyl-α-phenyl-2H-tetrazole-5-acetamide,
(±)-N-(2,4-difluorophenyl)-2-dodecyl-α-phenyl-2H-tetrazole-5-acetamide,
(±)-2-octyl-α-phenyl-N-(2,4,6-trimethoxyphenyl)-2H-tetrazole-5-acetamide, or
(±)-2-hexadecyl-α-phenyl-N-(2,4,6-trimethoxyphenyl)-2H-tetrazole-5-acetamide.

18. A compound of Claim 15 which is
(±)-N-(4,6-dimethoxy-5-pyrimidinyl)-2-dodecyl-α-phenyl-2H-tetrazole-5-acetamide,
(±)-N-(5,7-dimethyl-1,8-naphthyridine-2-yl)-2-dodecyl-α-phenyl-2H-tetrazole-5-acetamide,
(±)-2-dodecyl-α-phenyl-N-(1,3,5-trimethyl-1H-pyrazol-4-yl)-2H-tetrazole-5-acetamide,
(±)-N-cyclopropyl-2-dodecyl-α-phenyl-2H-tetrazole-5-acetamide,
(±)-2-dodecyl-α-phenyl-N-2-pyridinyl-2H-tetrazole-5-acetamide,
(±)-2-dodecyl-N-(3-methyl-2-pyridinyl)-α-phenyl-2H-tetrazole-5-acetamide,
(±)-2-dodecyl-N-(3-methyl-2-pyridinyl)-2-phenyl-2H-tetrazole-5-acetamide, N-oxide, or
(±)-N-(1,1-dimethylethyl)-2-dodecyl-α-phenyl-2H-tetrazole-5-acetamide.

19. A compound of Claim 3 wherein R₃ is a 5- or 6-membered monocyclic or fused bicyclic heterocyclic group containing at least 1 to 4 heteroatoms in at least one ring, said heteroatom being nitrogen, oxygen, or sulfur, and combinations thereof, with said heterocyclic group being unsubstituted or substituted with an alkyl group having from 1 to 4 carbon atoms, and the N-oxides thereof.

20. A compound of Claim 19 wherein R₃ is 2-, 3-, or 4-pyridyl.

21. A compound of Claim 20 which is (±)-2-dodecyl-α-(2-pyridyl)-N-(2,4,6-trimethoxyphenyl)-2H-tetrazole-5-acetamide, or
(±)-N-[2,6-Bis(1-methylethyl)phenyl]-2-dodecyl-α-2-pyridinyl-2H-tetrazole-5-acetamide.

22. A compound of Claim 3 wherein each of R₂ and R₃ is other than hydrogen.

23. A compound of Claim 22 which is
2-dodecyl-α,α-dimethyl-N-(2,4,6-trimethoxyphenyl)-2H-tetrazole-5-acetamide,
2-dodecyl-α,α'-(2-propenyl)-N-(2,4,6-trimethoxyphenyl)-2H-tetrazole-5-acetamide,
1-(2-dodecyl-2H-tetrazol-5-yl)-N-(2,4,6-trimethoxyphenyl)cyclopentanecarboxamide, or
2-tridecyl-α,α-dimethyl-N-(2,4,6-trimethoxyphenyl)-2H-tetrazole-5-acetamide.

24. A compound of Claim 2 wherein n is one or two.

25. A compound of Claim 24 wherein R₁ is phenyl which is unsubstituted or which is substituted.

26. A compound of Claim 25 which is
2-dodecyl-N-(2,4,6-trimethoxyphenyl)-2H-tetrazole-5-propanamide,
N-(2,6-bis(1-methylethyl)phenyl)-2-dodecyl-2H-tetrazole-5-propanamide,
N-(2,4-difluorophenyl)-2-dodecyl-2H-tetrazole-5-propanamide, or
1-dodecyl-N-(2,4,6-trimethoxyphenyl)-1H-tetrazole-5-propanamide.

27. A compound of Claim 1 which is
(±)-n-(2,4-difluorophenyl)-1-dodecyl-α-phenyl-1H-tetrazole-5-acetamide,
(±)-N-[2,6-bis(1-methylethyl)phenyl]-1-dodecyl-α-phenyl-1H-tetrazole-5-acetamide.

28. A compound of Claim 6 wherein R₁ is
2,6-(1-methylethyl)phenyl or 2,4,6-trimethoxyphenyl; n is zero; R₂ and R₃ are each independently hydrogen, methyl, fluoro, cyclohexyl, phenyl, or substituted phenyl, phenylalkyl, or naphthyl, and R₄ is in the 2-position and has 12 carbon atoms and the side chain is attached to the carbon atom of the tetrazole ring.

29. A compound of Claim 28 which is
(±)-2-Dodecyl-α-methyl-α-phenyl-N-(2,4,6-trimethoxyphenyl)-2H-tetrazole-5-acetamide,
(±)-2-Dodecyl-α-(4-fluorophenyl)-N-(2,4,6-trimethoxyphenyl)-2H-tetrazole-5-acetamide,
(±)-2-Dodecyl-α-2-naphthalenyl-N-(2,4,6-trimethoxyphenyl)-2H-tetrazole-5-acetamide,
(±)-α-([1,1'-biphenyl]-4-yl)-2-dodecyl-N-(2,4,6-trimethoxy-phenyl)-2H-tetrazole-5-acetamide,
(±)-2-Dodecyl-α-methyl-N-(2,4,6-trimethoxyphenyl)-2H-tetrazole-5-acetamide,
(±)-2-Dodecyl-α-phenylmethyl-N-(2,4,6-trimethoxyphenyl)-2H-tetrazole-5-acetamide,
(±)-2-Dodecyl-α-cyclohexyl-N-(2,4,6-trimethoxy-phenyl)-2H-tetrazole-5-acetamide,
(-)-2-Dodecyl-α-phenyl-N-(2,4,6-trimethoxyphenyl)-2H-tetrazole-5-acetamide [α]_{D} = -58° (1% in CH₃OH),
(+)-2-Dodecyl-α-phenyl-N-(2,4,6-trimethoxyphenyl)-2H-tetrazole-5-acetamide [α]_{D} = +55.1° (1% in CH₃OH),
(±)-N-[2,6-Bis(1-methylethyl)phenyl]-2-dodecyl-α-fluoro-α-phenyl-2H-tetrazole-5-acetamide, or
(±)-2-Dodecyl-α-fluoro-α-phenyl-N-(2,4,6-trimethoxy phenyl)-2H-tetrazole-5-acetamide.

30. A compound of Claim 1 wherein R₄ is attached to the carbon atom of the tetrazole ring and the side chain is on the 2-position of the tetrazole ring.

31. A compound of Claim 30 which is
N-[2,6-bis(1-methylethyl)phenyl]-5-decyl-2H-tetrazole-2-acetamide;
N-[2,6-bis(1-methylethyl)phenyl-5-dodecyl-2H-tetrazole-2-acetamide;
(±)-N-[2,6-bis(1-methylethyl)phenyl]-5-dodecyl-α-phenyl-2H-tetrazole-2-acetamide;
(±)-N-[2,6-bis(1-methylethyl)phenyl]-5-dodecyl-α-pentyl-2H-tetrazole-2-acetamide;
(±)-N-[2,6-bis(1-methylethyl)phenyl-5-(dodecylthio)-α-phenyl-2H-tetrazole-2-acetamide;
(±)-5-decyl-α-phenyl-N-(2,4,6-trimethoxyphenyl-2H-tetrazole-2-acetamide;
5-dodecyl-N-(2,4,6-trimethoxy-phenyl)-2H-tetrazole-2-acetamide;
(±)-5-dodecyl-α-phenyl-N-(2,4,6-trimethoxyphenyl)-2H-tetrazole-2-acetamide;
(±)-5-dodecyl-α-pentyl-N-(2,4,6-trimethoxyphenyl)-2H-tetrazole-2-acetamide;
(±)-N-(2,4-difluorophenyl-5-dodecyl-α-phenyl-2H-tetrazole-2-acetamide;
5-dodecyl-α,α-dimethyl-N-(2,4,6-trimethoxyphenyl)-2H-tetrazole-2-acetamide;
(±)-5-(dodecylthio)-α-phenyl-N-(2,4,6-trimethoxyphenyl)-2H-tetrazole-2-acetamide; or,
(±)-5-(dodecylsulfinyl)-α-phenyl-N-(2,4,6)-trimethoxyphenyl)-2H-tetrazole-2-acetamide.

32. A pharmaceutical composition comprising a compound according to any one of claims 1 to 31 and a pharmaceutically acceptable liquid or solid carrier, diluent or adjuvant.

33. Use of a compound according to any one of claims 1 to 31 for the preparation of pharmaceutical compositions acting as inhibitors of acyl-CoA: cholesterol acyltransferase (ACAT) decreasing the absorption of dietary cholesterol and providing a therapy for hypercholesterolemia.

34. A process for preparing a compound of formula I, 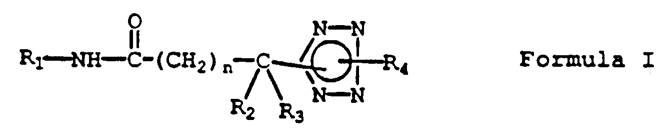 wherein n is zero and R₂ and R₃ are hydrogen and R₁ and R₄ are defined according to formula I of claim 1, characterized by the following routes of synthesis:

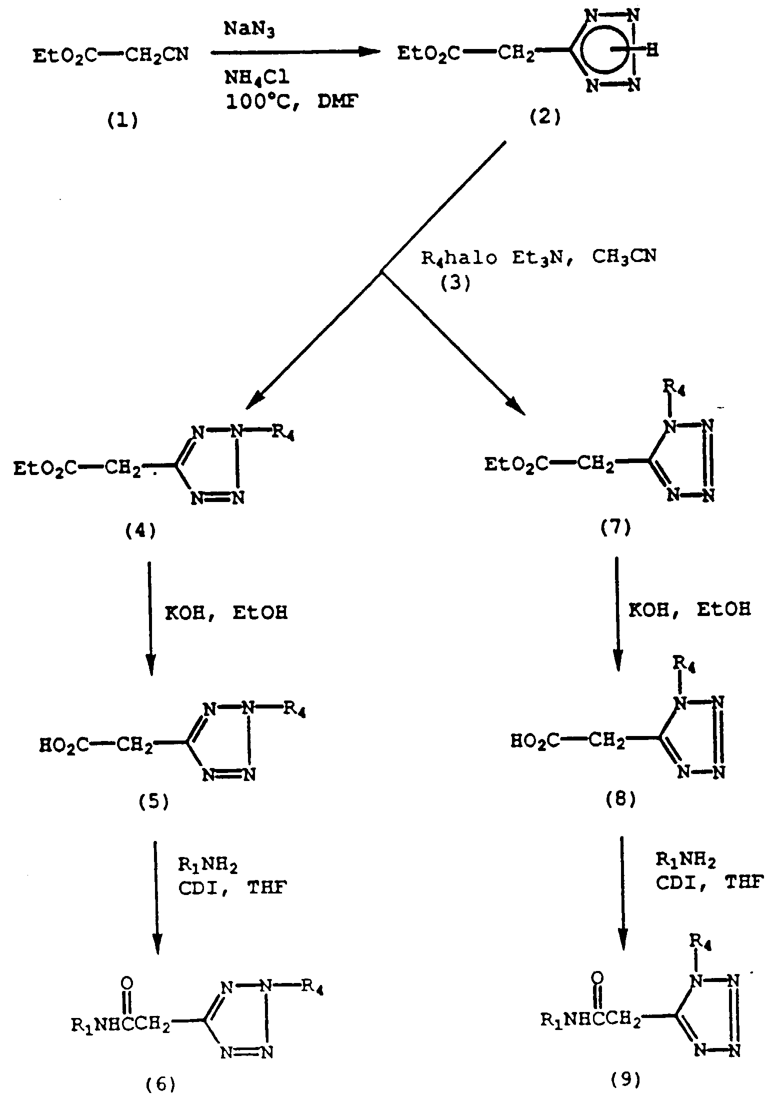

35. A process for preparing a compound of formula I, 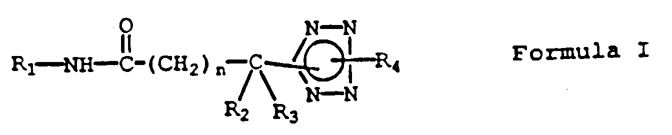 wherein n is zero and R₁, R₂, R₃ and R₄ are as defined in formula I of claim 1 characterized by the following routes of synthesis:

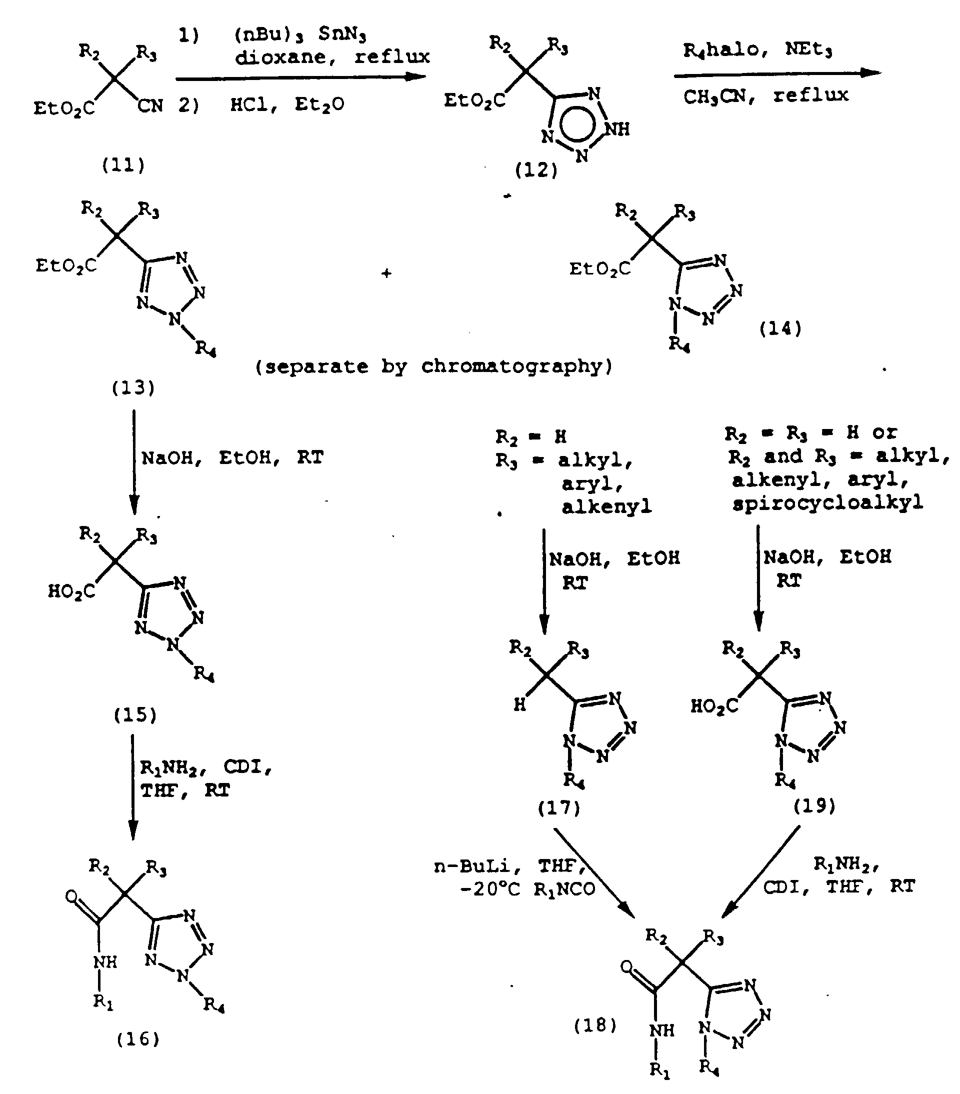

36. A process for preparing a compound of formula I 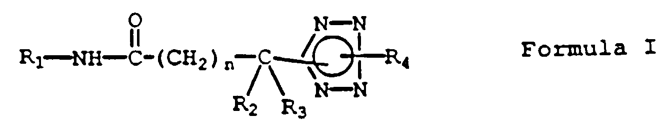 wherein n is one or two, R₂ and R₃ are hydrogen and R₁ and R₄ are as defined in formula I of claim 1, characterized by the following routes of synthesis:

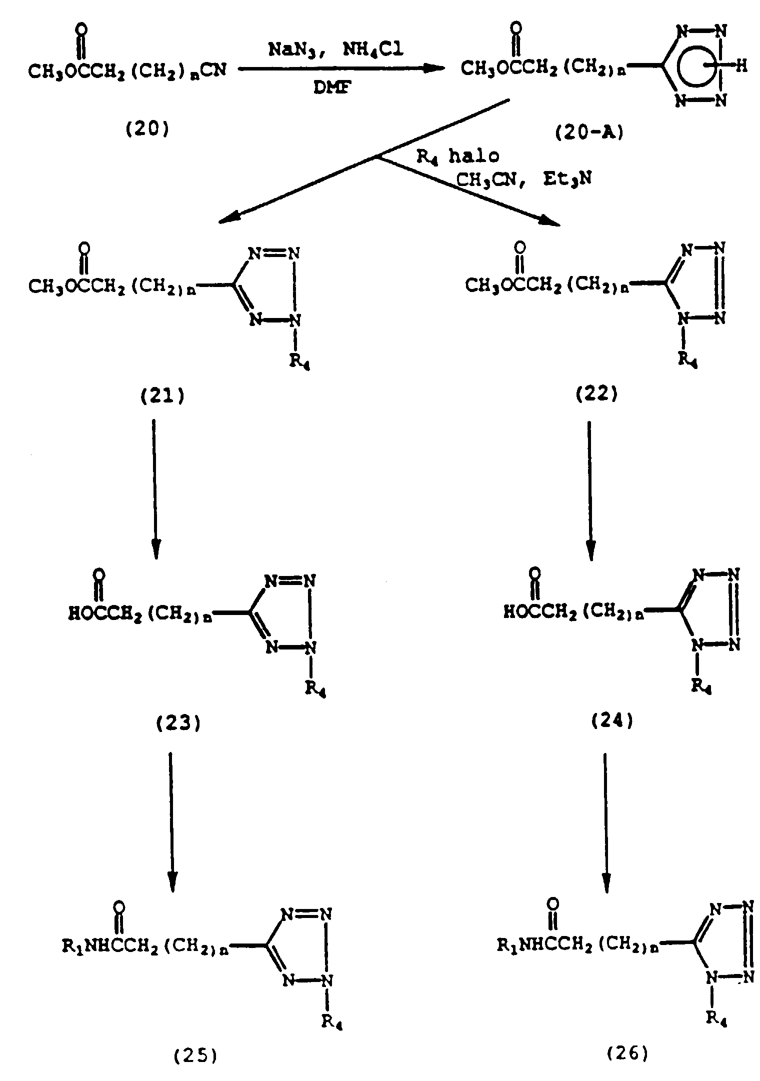

37. A process for preparing a compound of formula I according to claim 1, 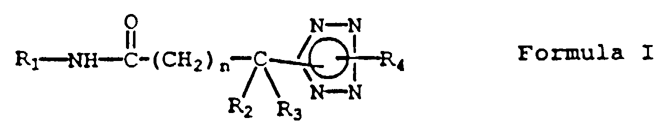 wherein n is one, R₂ is hydrogen and R₃ (x) is phenyl, substituted phenyl, alkyl, alkenyl or heteroaryl, characterized by the following routes of synthesis:

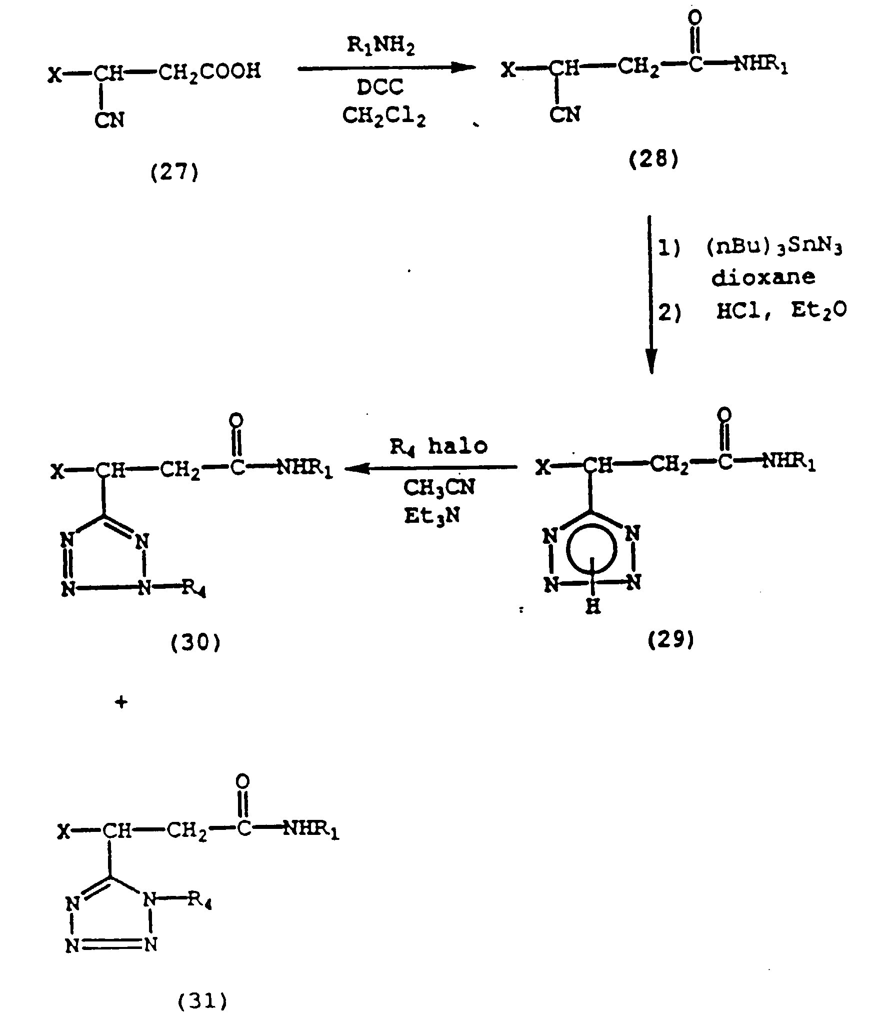

38. A process for preparing a compound of formula I according to claim 1 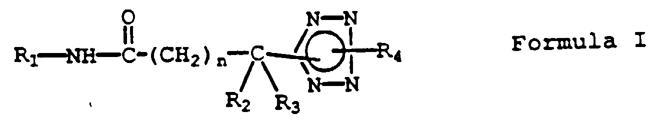 wherein n is zero and R₂ and R₃ is alkyl or aryl, characterized by the following routes of synthesis:

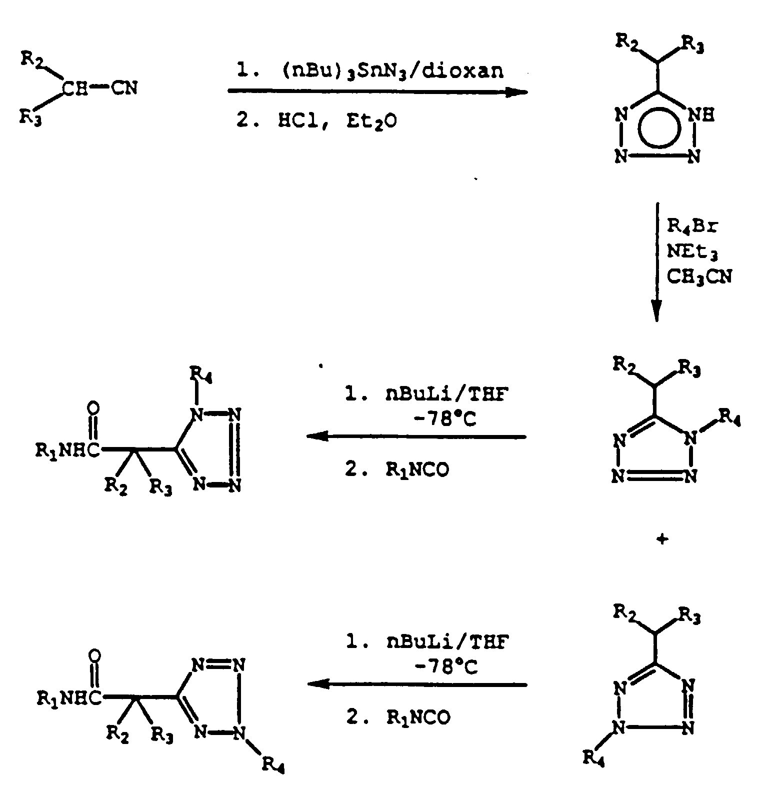

39. A process for preparing a compound of formula I according to claim 1 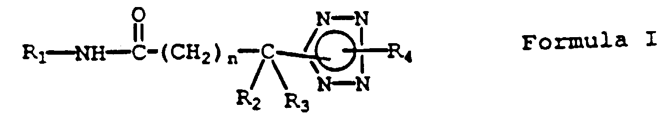 wherein n is two, R₂ is hydrogen and R₃ is phenyl or substituted phenyl, characterized by the following routes of synthesis:

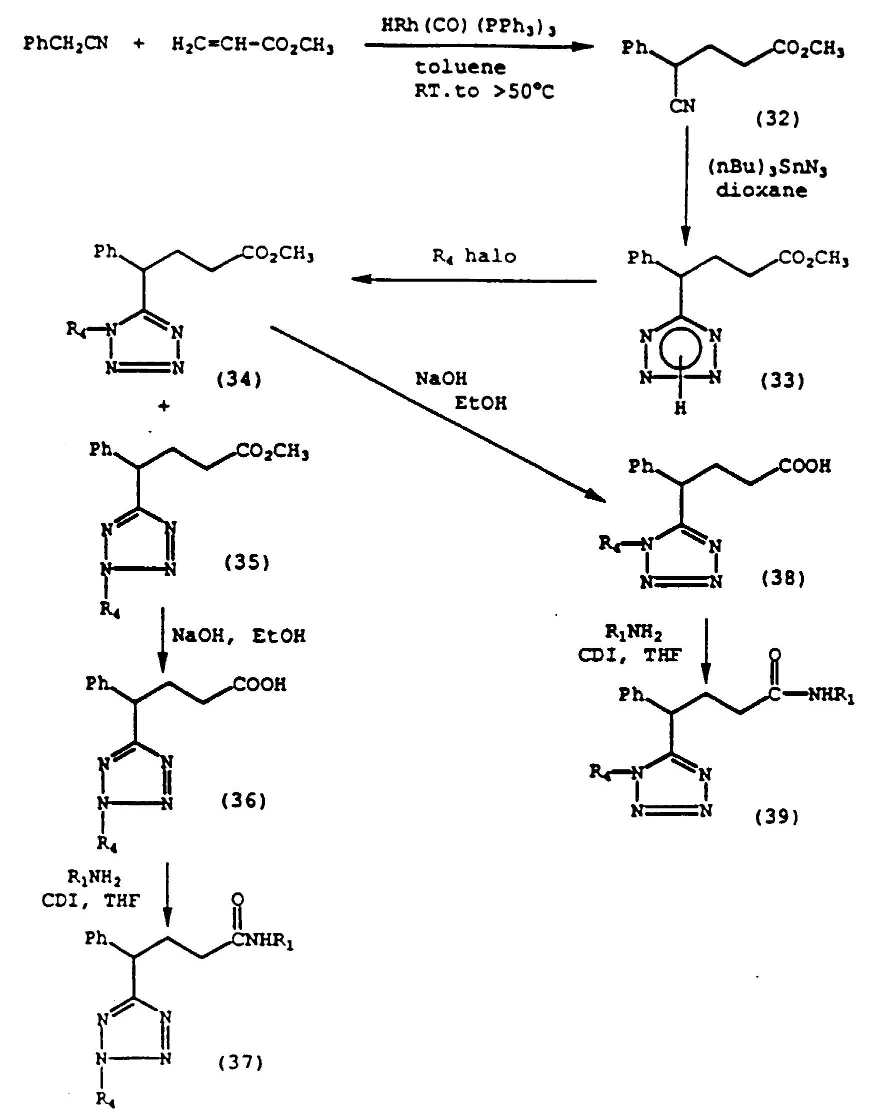

40. A process for preparing a compound of formula I according to claim 1 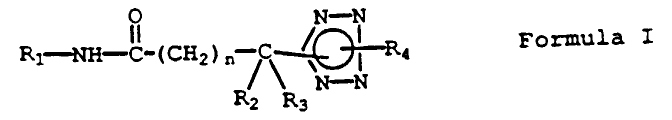 wherein n is zero, R₂ is hydrogen and R₃ is heteroaryl, 1- or 2-naphthyl or substituted phenyl, characterized by the following routes of synthesis:

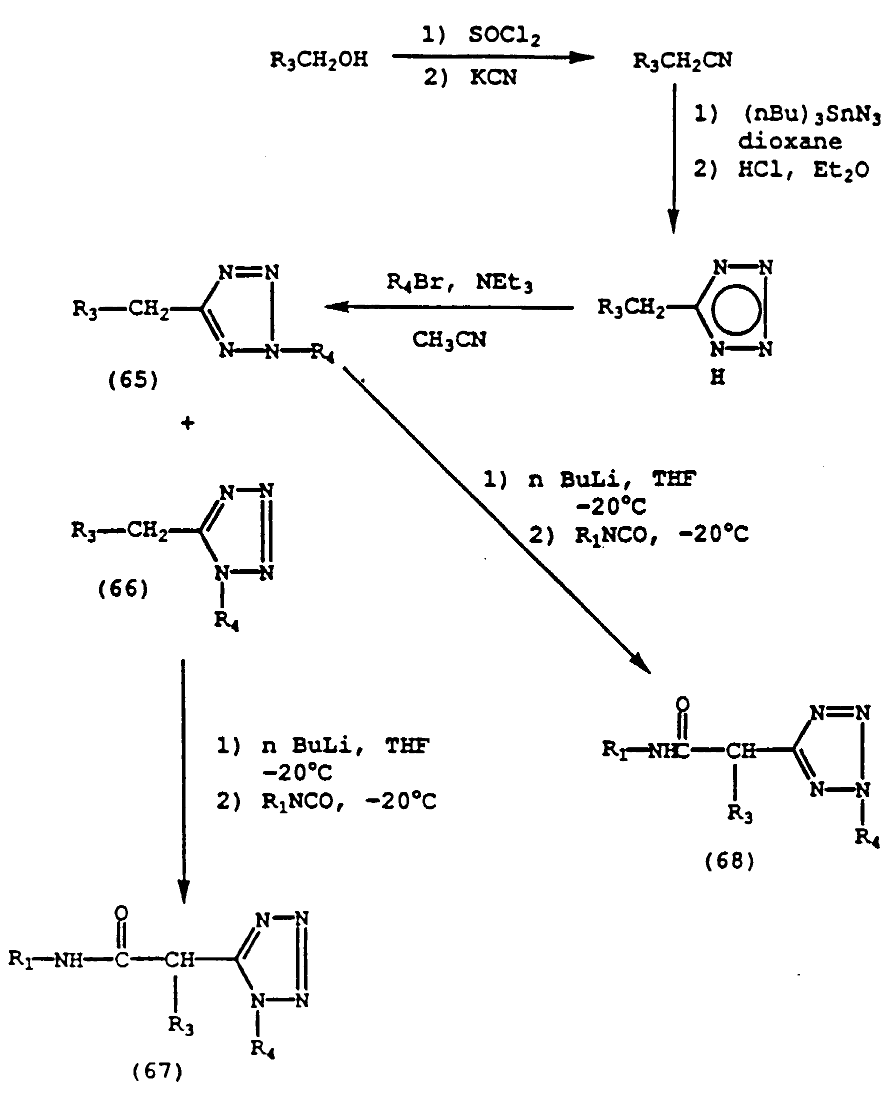

41. A process for preparing a compound of formula I, 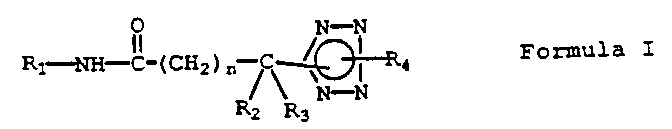 wherein n is one, R₁, R₂, R₃ and R₄ are as defined in formula I of claim 1 characterized by the following routes of synthesis:

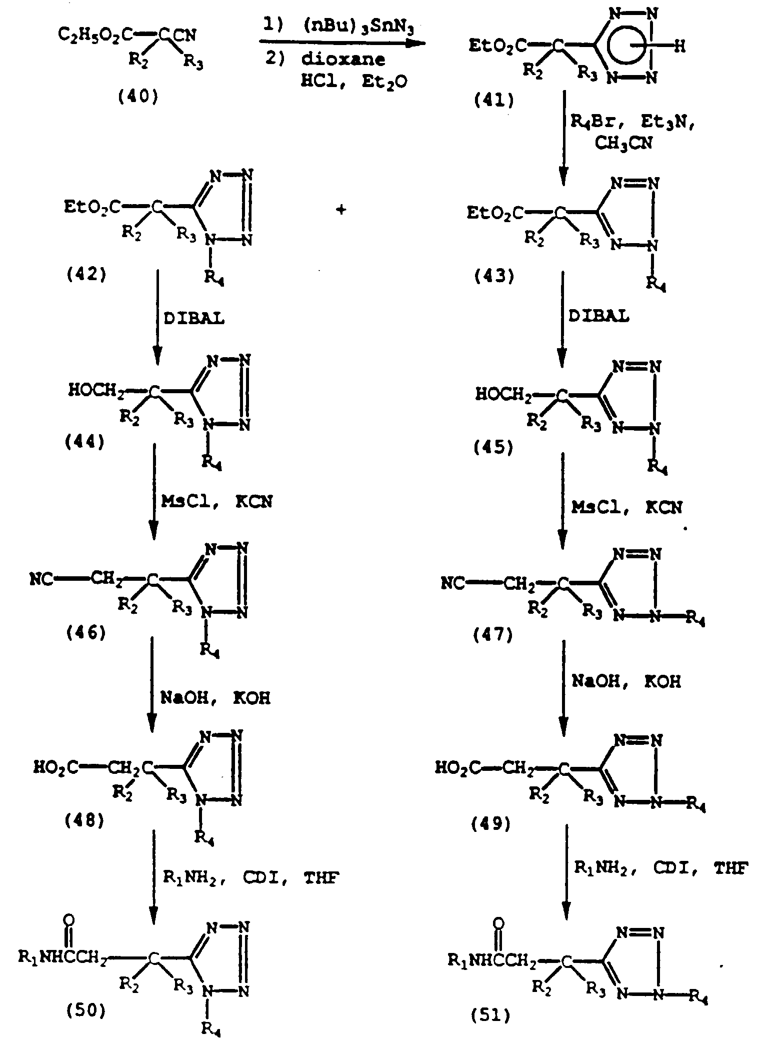

42. A process for preparing a compound of formula I according to claim 1 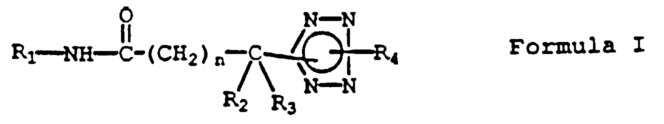 wherein n is one and R₃ is heteroaryl, characterized by the following routes of synthesis:

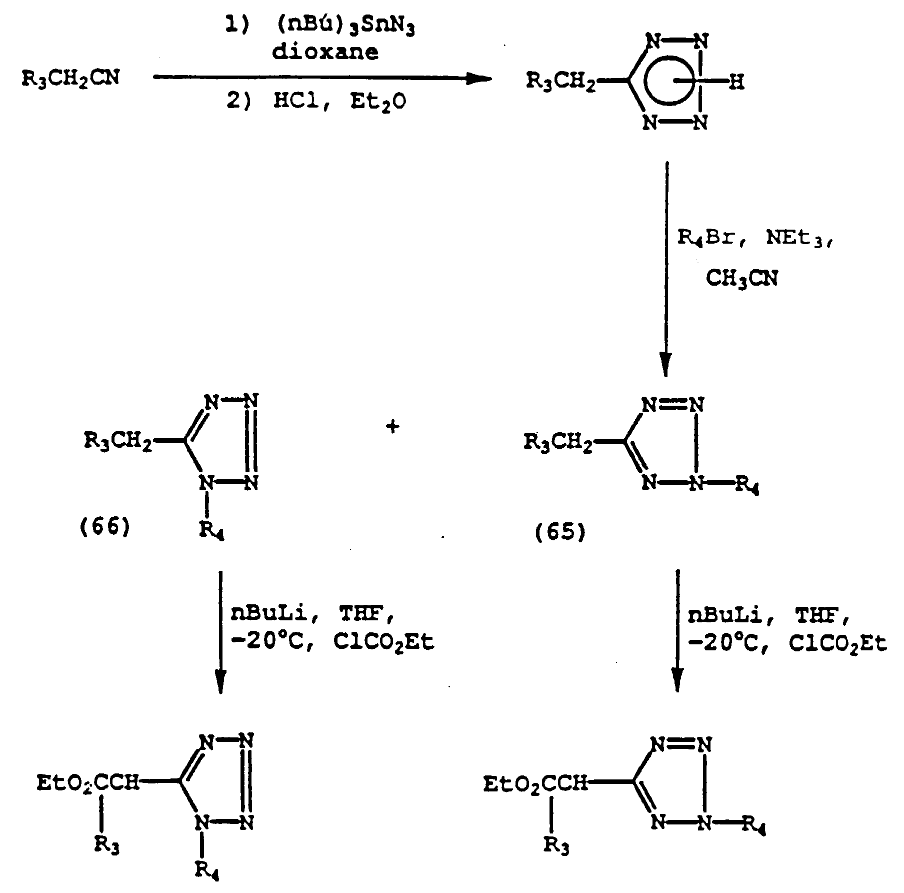

43. A process for preparing a compound of formula I according to claim 1 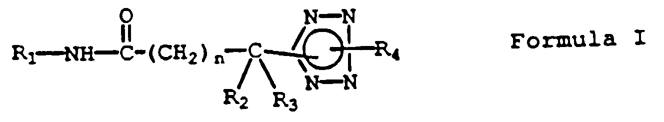 wherein n is two, with the proviso that at least one of R₂ and R₃ is other than hydrogen characterized by the following routes of synthesis:

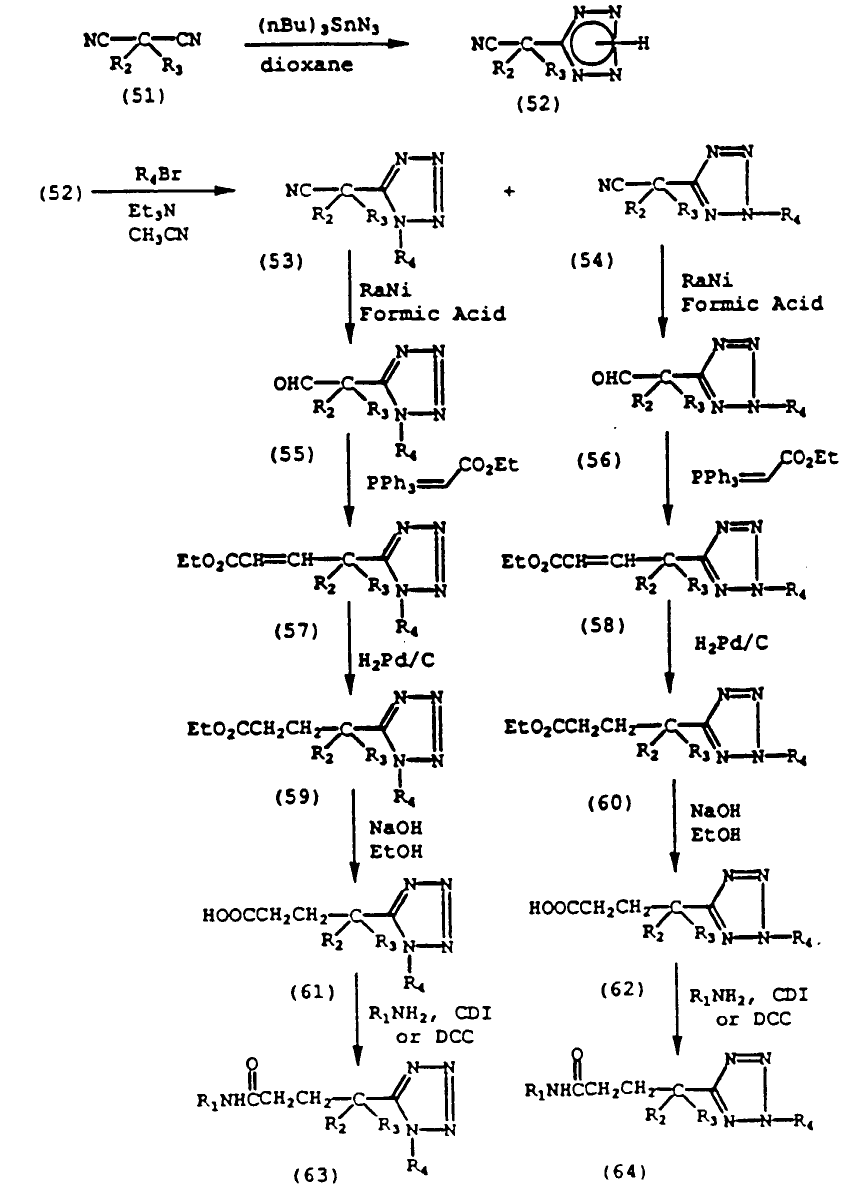

44. A process for preparing a compound of formula I, 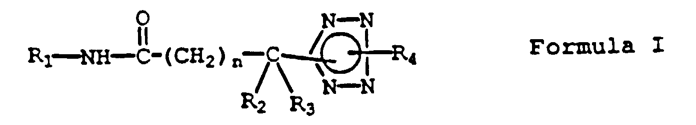 wherein n is zero, R₃ is F or OH and R₁, R₂ and R₄ are as defined in formula I of claim 1 characterized by the following routes of synthesis:

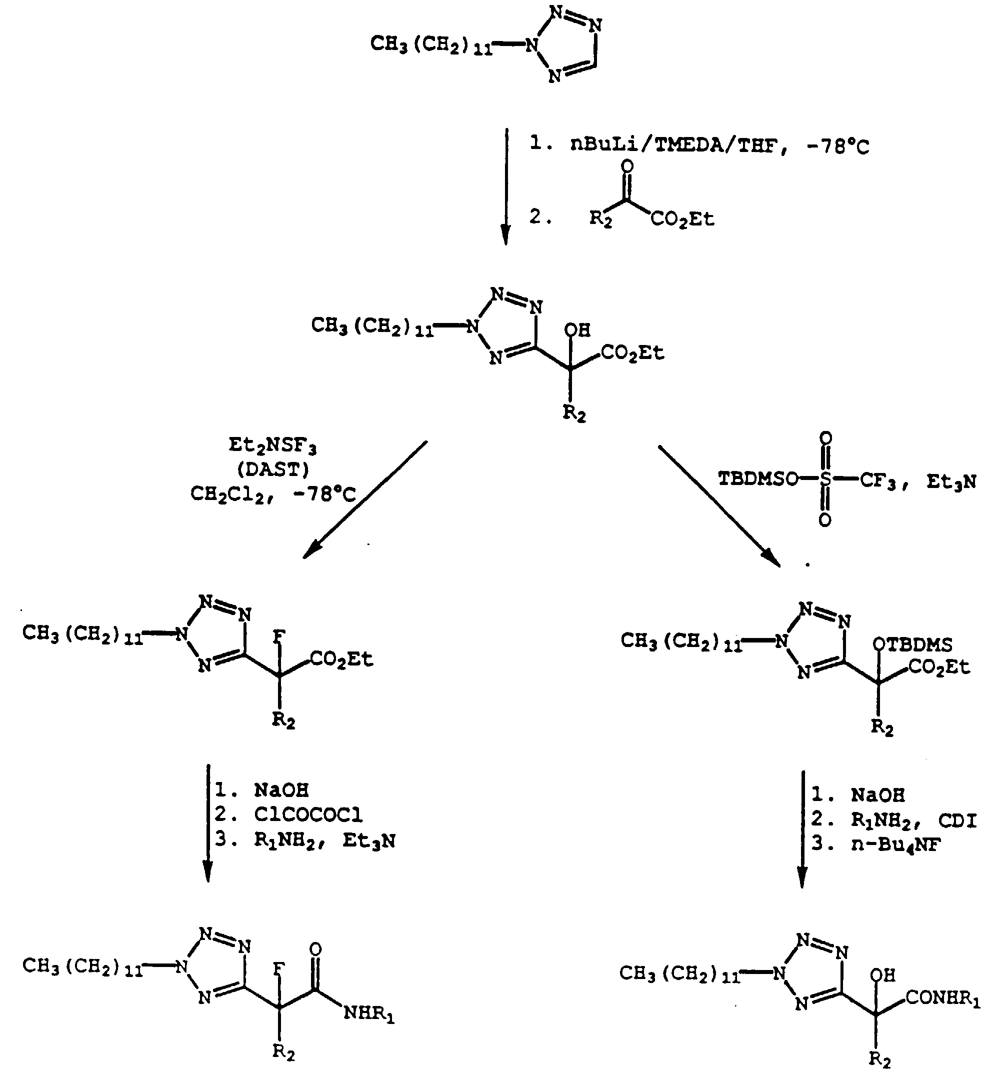

45. A process for preparing a pharmaceutical composition characterized by combining a compound manufactured according to anyone of claims 34 to 43 with a pharmaceutically acceptable liquid or solid carrier, diluent or adjuvant.

## Patentansprüche

1. Verbindung der Formel 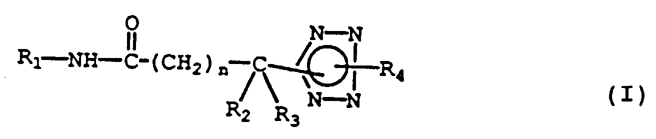 worin n gleich Null, Eins oder Zwei ist;
worin R₁ aus nachstehenden ausgewählt ist:
(a) Phenyl, das unsubstituiert oder substituiert ist mit einem bis drei unter den nachstehenden ausgewählten Substituenten:
geradkettiges oder verzweigtes Alkyl mit 1 bis 4 Kohlenstoffatomen,
geradkettiges oder verzweigtes Alkoxy mit 1 bis 3 Kohlenstoffatomen,
geradkettiges oder verzweigtes Alkylthio mit 1 bis 3 Kohlenstoffatomen,
Hydroxy,
Phenyl,
Fluor,
Chlor,
Brom,
Nitro,
Cyan,
Trifluormethyl,
-COOH,
-COOAlkyl, worin Alkyl 1 bis 4 Kohlenstoffatome aufweist und geradkettig oder verzweigt ist,
-(CH₂)ₘNR₅R₆, worin m gleich Null oder Eins ist und von R₅ und R₆ ein jeder Wasserstoff oder einen geradkettigen oder verzweigten Alkylrest mit 1 bis 4 Kohlenstoffatomen bedeutet;
(b) 1- oder 2-Naphthyl, das unsubstituiert oder substituiert ist mit einem bis drei unter den nachstehenden ausgewählten Substituenten:
geradkettiges oder verzweigtes Alkyl mit 1 bis 4 Kohlenstoffatomen,
geradkettiges oder verzweigtes Alkoxy mit 1 bis 3 Kohlenstoffatomen,
Hydroxy,
Fluor,
Chlor,
Brom,
Nitro,
Cyan,
Trifluormethyl,
-COOH,
-COOAlkyl, worin Alkyl 1 bis 4 Kohlenstoffatome aufweist und geradkettig oder verzweigt ist,
-(CH₂)ₘNR₅R₆, worin m, R₅ und R₆ die im vorstehenden definierte Bedeutung haben;
(c) der Rest 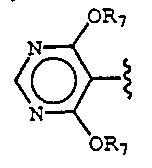 worin R₇ einen geradkettigen oder verzweigten Niederalkylrest mit 1 bis 3 Kohlenstoffatomen bedeutet;
(d) der Rest 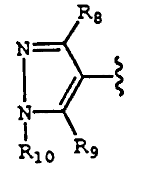 worin R₈ und R₉ jeweils geradkettiges oder verzweigtes Alkyl mit 1 bis 4 Kohlenstoffatomen oder Phenyl bedeuten und R₁₀ bedeutet: einen geradkettigen oder verzweigten Kohlenwasserstoffrest mit 1 bis 18 Kohlenstoffatomen, der gesättigt oder ungesättigt ist mit einer Doppelbindung oder zwei nicht nebeneinanderliegenden Doppelbindungen; Phenyl; substituiertes Phenyl mit einem bis drei Substituenten, die ausgewählt sind unter geradkettigem oder verzweigtem Alkyl mit 1 bis 4 Kohlenstoffatomen, geradkettigem oder verzweigtem Alkoxy mit 1 bis 3 Kohlenstoffatomen, Hydroxy, Fluor, Chlor, Brom, Nitro, Cyan, Trifluormethyl, -COOH, -COOAlkyl, worin Alkyl 1 bis 4 Kohlenstoffatome aufweist und geradkettig oder verzweigt ist, oder -(CH₂)ₘNR₅R₆, worin m, R₅ und R₆ die im vorstehenden definierte Bedeutung haben; oder einen unter 2-, 3- oder 4-Pyridyl, 2-, 4- oder 5-Pyrimidinyl, 2- oder 3-Pyrazinyl, 2-, 3-, 4-, 5-, 6-, 7- oder 8-Chinolinyl, oder 3- oder 4-Pyridazinyl sowie deren N-Oxiden ausgewählten hetercyclischen Rest;
(e) der Rest 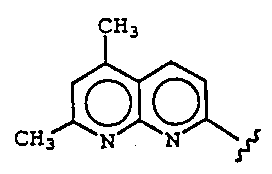
(f) der Rest 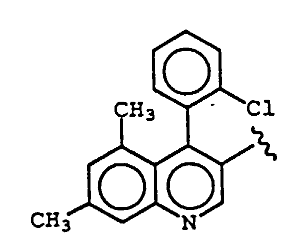
(g) ein geradkettiger oder verzweigter Kohlenwasserstoffrest mit 1 bis 18 Kohlenstoffatomen, der gesättigt oder ungesättigt ist mit einer Doppelbindung oder zwei nicht nebeneinanderliegenden Doppelbindungen;
(h) ein Cycloalkylrest mit 3 bis 8 Kohlenstoffatomen;
(i) ein unter 2-, 3- oder 4-Pyridyl ausgewählter heteroaromatischer Rest, der unsubstituiert oder mit einem Alkylrest mit 1 bis 4 Kohlenstoffatomen substituiert ist, oder 2-, 4- oder 5-Pyrimidinyl sowie deren N-Oxide;
(j) der Rest 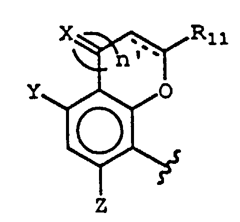 worin
--- eine Einfach- oder Doppelbindung bedeutet;
Y und Z unabhängig voneinander Wasserstoff, einen geradkettigen oder verzweigten Alkylrest mit 1 bis 4 Kohlenstoffatomen, einen Alkoxyrest mit 1 bis 3 Kohlenstoffatomen, oder Halogen bedeuten;
X Sauerstoff oder zwei Wasserstoffatome bedeutet;
R₁₁ Wasserstoff oder einen geradkettigen oder verzweigten Alkylrest mit 1 bis 4 Kohlenstoffatomen bedeutet und n' gleich Null oder Eins ist; oder
(k) eine Auswahl unter den Resten 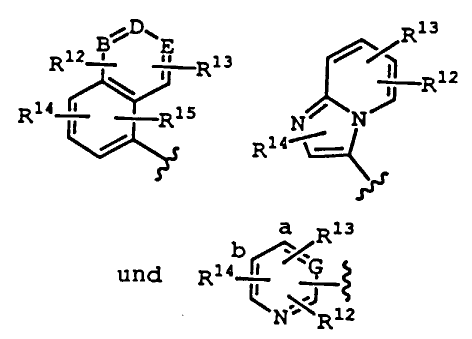 worin
R₁₂, R₁₃, R₁₄ und R₁₅ unabhängig voneinander Wasserstoff, Halogen, einen geradkettigen oder verzweigten Alkylrest mit 1 bis 4 Kohlenstoffatomen, einen Alkoxyrest mit 1 bis 3 Kohlenstoffatomen, einen Alkylthiorest mit 1 bis 3 Kohlenstoffatomen, Cycloalkylthio mit 5 bis 7 Kohlenstoffatomen, Phenylalkylthio, bei welchem Alkyl 1 bis 4 Kohlenstoffatome aufweist, substituiertes Phenylthio, Heteroarylthio oder Heteroaryloxy bedeuten; und
B, D, E und G Stickstoff oder Kohlenstoff bedeuten, wobei von B, D und E einer oder mehrere Stickstoff bedeuten;
mit der Massgabe, dass, wenn G Stickstoff bedeutet, der Rest mit Position 4 oder 5 des Pyrimidinsringes (a und b) am Stickstoffatom der Formel I sitzt;
worin R₂ und R₃ gleich oder verschieden sind und aus nachstehendem ausgewählt sind:
(a) Wasserstoff, Halogen, oder es bedeutet einer von R₂ und R₃ Hydroxy;
(b) ein geradkettiger oder verzweigter Alkylrest mit 1 bis 12 Kohlenstoffatomen oder ein Cycloalkylrest mit 3 bis 8 Kohlenstoffatomen;
(c) ein Phenyl- oder Phenylalkylrest, bei welchem Alkyl 1 bis 4 Kohlenstoffatome aufweist und der Phenylring unsubstituiert ist oder substituiert ist mit einem bis drei unter den nachstehenden ausgewählten Substituenten: geradkettiges oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Hydroxy, Fluor, Chlor, Brom, Trifluormethyl, Cyan, Nitro, Phenyl oder (CH₂)ₘNR₅R₆, worin m, R₅ und R₆ die im vorstehenden definierte Bedeutung haben;
(d) ein geradkettiger oder verzweigter Alkenylrest mit 2 bis 6 Kohlenstoffatomen; oder
(e) R₂ und R₃ bilden zusammen mit dem Kohlenstoffatom, an das sie gebunden sind, einen Alkylidenrest mit 1 bis 4 Kohlenstoffatomen, einen Benzylidenrest oder einen Spiroalkylrest mit 3 bis 7 Kohlenstoffatomen; oder
(f) wenn R₂ die Bedeutung Wasserstoff, Fluor oder C₁₋₁₂-Alkyl hat, dann bedeutet R₃ Heteroaryl, das unter 5- oder 6-gliedrigen monocyclischen oder verschmolzenen bicyclischen heterocyclischen Resten mit mindestens 1 bis 4 Heteroatomen in mindestens einem Ring ausgewählt ist, wobei diese Heteroatome Stickstoff, Sauerstoff, Schwefel oder Kombinationen davon sind, wobei dieser heterocyclische Rest unsubstituiert oder mit einem Alkylrest mit 1 bis 4 Kohlenstoffatomen substituiert ist, sowie deren N-Oxide;
(g) 1- oder 2-Naphthyl, das unsubstituiert oder substituiert ist mit einem bis drei unter den nachstehenden ausgewählten Substituenten:
geradkettiges oder verzweigtes Alkyl mit 1 bis 4 Kohlenstoffatomen,
geradkettiges oder verzweigtes Alkoxy mit 1 bis 3 Kohlenstoffatomen,
worin R₄ einen geradkettigen oder verzweigten Kohlenwasserstoffrest mit 1 bis 20 Kohlenstoffatomen, der gesättigt oder ungesättigt ist mit einer Doppelbindung oder zwei nicht nebeneinanderliegenden Doppelbindungen, oder gesättigtes Alkylthio mit 1 bis 20 Kohlenstoffatomen bedeutet;
oder ein pharmazeutisch annehmbares Salz oder ein einzelnes enantiomeres Isomer davon.

2. Verbindung nach Anspruch 1, worin R₄ sich in Position 2 des Tetrazolringes befindet und die Seitenkette an das Kohlenstoffatom des Tetrazolringes gebunden ist.

3. Verbindung nach Anspruch 2, worin n gleich Null ist.

4. Verbindung nach Anspruch 3, worin von R₂ und R₃ ein jeder Wasserstoff bedeutet.

5. Verbindung nach Anspruch 4, worin R₄ eine gesättigte Kohlenwasserstoffkette mit 8 bis 18 Kohlenstoffatomen bedeutet.

6. Verbindung nach Anspruch 5, worin R₁ Phenyl oder substituiertes Phenyl bedeutet.

7. Verbindung nach Anspruch 6, bestehend aus
N-[2,6-Bis(1-methylethyl)phenyl]-2-dodecyl-2H-tetrazol-5-acetamid;
2-Dodecyl-N-(2,4,6-trimethoxyphenyl)-2H-tetrazol-5-acetamid;
N-(2,4-Difluorophenyl)-2-dodecyl-2H-tetrazol-5-acetamid; oder
2-Tetradecyl-N-(2,4,6-trimethoxyphenyl)-2H-tetrazol-5-acetamid.

8. Verbindung nach Anspruch 5, worin R₁ den Rest 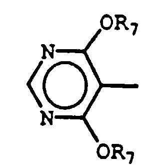 bedeutet, worin R₇ einen geradkettigen oder verzweigten Niederalkylrest mit 1 bis 3 Kohlenstoffatomen bedeutet.

9. Verbindung nach Anspruch 8, bestehend aus
N-(4,6-Dimethoxy-5-pyrimidinyl)-2-dodecyl-2H-tetrazol-5-acetamid; oder
N-(4,6-Dimethoxy-5-pyrimidinyl)-2-dodecyl-1H-tetrazol-5-acetamid.

10. Verbindung nach Anspruch 5, worin R₁ einen unter 2-, 3- oder 4-Pyridyl ausgewählten heteroaromatischen Rest, der unsubstituiert oder mit einem Alkylrest mit 1 bis 4 Kohlenstoffatomen substituiert ist, oder 2-, 4- oder 5-Pyrimidinyl sowie deren N-Oxide bedeutet.

11. Verbindung nach Anspruch 10, bestehend aus
2-Dodecyl-N-(3-methyl-2-pyridinyl)-2H-tetrazol-5-acetamid.

12. Verbindung nach Anspruch 5, worin R₁ den Rest 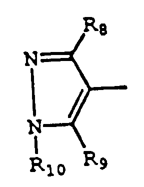 bedeutet, worin R₈ und R₉ jeweils geradkettiges oder verzweigtes Alkyl mit 1 bis 4 Kohlenstoffatomen oder Phenyl bedeuten und R₁₀ bedeutet: einen geradkettigen oder verzweigten Kohlenwasserstoffrest mit 1 bis 18 Kohlenstoffatomen, der gesättigt oder ungesättigt ist mit einer Doppelbindung oder zwei nicht nebeneinanderliegenden Doppelbindungen; Phenyl; substituiertes Phenyl mit einem bis drei Substituenten, die ausgewählt sind unter geradkettigem oder verzweigtem Alkyl mit 1 bis 4 Kohlenstoffatomen, geradkettigem oder verzweigtem Alkoxy mit 1 bis 3 Kohlenstoffatomen, Hydroxy, Fluor, Chlor, Brom, Nitro, Cyan, Trifluormethyl, -COOH, -COOAlkyl, worin Alkyl 1 bis 4 Kohlenstoffatome aufweist und geradkettig oder verzweigt ist, oder -(CH₂)ₘNR₅R₆, worin m, R₅ und R₆ die im vorstehenden definierte Bedeutung haben; oder einen unter 2-, 3- oder 4-Pyridyl, 2-, 4- oder 5-Pyrimidinyl, 2- oder 3-Pyrazinyl, 2-, 3-, 4-, 5-, 6-, 7- oder 8-Chinolinyl, oder 3- oder 4-Pyridazinyl sowie deren N-Oxiden ausgewählten hetercyclischen Rest.

13. Verbindung nach Anspruch 12, bestehend aus
2-Dodecyl-N-(1,3,5-trimethyl-1H-pyrazol-4-yl)-2H-tetrazol-5-acetamid; oder
1-Dodecyl-N-(1,3,5-trimethyl-1H-pyrazol-4-yl)-1H-tetrazol-5-acetamid.

14. Verbindung nach Anspruch 3, worin von R₂ und R₃ der eine Wasserstoff und der andere unsubstituiertes oder substituiertes Phenyl bedeutet.

15. Verbindung nach Anspruch 14, worin R₄ eine gesättigte Kohlenwasserstoffkette mit 8 bis 18 Kohlenstoffatomen bedeutet.

16. Verbindung nach Anspruch 15, worin R₁ einen unsubstituierten oder substituierten Phenylrest bedeutet.

17. Verbindung nach Anspruch 16, bestehend aus
(±)-2-Dodecyl-α-phenyl-N-(2,4,6-trimethoxyphenyl)-2H-tetrazol-5-acetamid;
(±)-2-Dodecyl-N,α-diphenyl-2H-tetrazol-5-acetamid;
(±)-N-[2,6-Bis(1-methylethyl)phenyl]-2-dodecyl-α-phenyl-2H-tetrazol-5-acetamid;
(±)-N-(2,4-Difluorphenyl)-2-dodecyl-a-phenyl-2H-tetrazol-5-acetamid;
(±)-2-Octyl-α-phenyl-N-(2,4,6-trimethoxyphenyl)-2H-tetrazol-5-acetamid; oder
(±)-2-Hexadecyl-α-phenyl-N-(2,4,6-trimethoxyphenyl)-2H-tetrazol-5-acetamid.

18. Verbindung nach Anspruch 15, bestehend aus
(±)-N-(4,6-Dimethoxy-5-pyrimidinyl)-2-dodecyl-α-phenyl-2H-tetrazol-5-acetamid;
(±)-N-(5,7-Dimethyl-1,8-naphthyridin-2-yl)-2-dodecyl-α-phenyl-2H-tetrazol-5-acetamid;
(±)-2-Dodecyl-α-phenyl-N-(1,3,5-trimethyl-1H-pyrazol-4-yl)-2H-tetrazol-5-acetamid;
(±)-N-Cyclopropyl-2-dodecyl-α-phenyl-2H-tetrazol-5-acetamid;
(±)-2-Dodecyl-α-phenyl-N-2-pyridinyl-2H-tetrazol-5-acetamid;
(±)-2-Dodecyl-N-(3-methyl-2-pyridinyl)-α-phenyl-2H-tetrazol-5-acetamid;
(±)-2-Dodecyl-N-(3-methyl-2-pyridinyl)-2-phenyl-2H-tetrazol-5-acetamid, N-Oxid; oder
(±)-N-(1,1-Dimethylethyl)-2-dodecyl-α-phenyl-2H-tetrazol-5-acetamid.

19. Verbindung nach Anspruch 3, worin R₃ einen 5- oder 6-gliedrigen monocyclischen oder verschmolzenen bicyclischen heterocyclischen Rest mit mindestens 1 bis 4 Heteroatomen in mindestens einem Ring bedeutet, wobei dieses Heteroatom Stickstoff, Sauerstoff, Schwefel oder Kombinationen davon ist, wobei dieser heterocyclische Rest unsubstituiert oder mit einem Alkylrest mit 1 bis 4 Kohlenstoffatomen substituiert ist, sowie deren N-Oxide.

20. Verbindung nach Anspruch 19, worin R₃ 2-, 3- oder 4-Pyridyl bedeutet.

21. Verbindung nach Anspruch 20, bestehend aus
(±)-2-Dodecyl-α-(2-pyridyl)-N-(2,4,6-trimethoxyphenyl)-2H-tetrazol-5-acetamid; oder
(±)-N-[2,6-Bis(1-methylethyl)phenyl]-2-dodecyl-α-2-pyridinyl-2H-tetrazol-5-acetamid.

22. Verbindung nach Anspruch 3, worin von R₂ und R₃ ein jeder anderes als Wasserstoff bedeutet.

23. Verbindung nach Anspruch 22, bestehend aus
2-Dodecyl-α,α-dimethyl-N-(2,4,6-trimethoxyphenyl)-2H-tetrazol-5-acetamid;
2-Dodecyl-α,α-(2-propenyl)-N-(2,4,6-trimethoxyphenyl)-2H-tetrazol-5-acetamid;
1-(2-dodecyl-2H-tetrazol-5-yl)-N-(2,4,6-trimethoxyphenyl)-cyclopentancarboxamid; oder
2-Tridecyl-α,α-dimethyl-N-(2,4,6-trimethoxyphenyl)-2H-tetrazol-5-acetamid.

24. Verbindung nach Anspruch 2, worin n gleich Eins oder Zwei ist.

25. Verbindung nach Anspruch 24, worin R₁ einen unsubstituierten oder substituierten Phenylrest bedeutet.

26. Verbindung nach Anspruch 25, bestehend aus
2-Dodecyl-N-(2,4,6-trimethoxyphenyl)-2H-tetrazol-5-propanamid;
N-[2,6-Bis(1-methylethyl)phenyl]-2-dodecyl-2H-tetrazol-5-propanamid;
N-(2,4-Difluorphenyl)-2-dodecyl-2H-tetrazol-5-propanamid; oder
1-Dodecyl-N-(2,4,6-trimethoxyphenyl)-1H-tetrazol-5-propanamid.

27. Verbindung nach Anspruch 1, bestehend aus
(±)-N-(2,4-Difluorphenyl)-1-dodecyl-α-phenyl-1H-tetrazol-5-acetamid;
(±)-N-[2,6-Bis(1-methylethyl)phenyl]-1-dodecyl-α-phenyl-1H-tetrazol-5-acetamid.

28. Verbindung nach Anspruch 6, worin R₁ 2,6-(1-Methylethyl)phenyl oder 2,4,6-trimethoxyphenyl bedeutet; n gleich Null ist; R₂ und R₃ unabhängig voneinander Wasserstoff, Methyl, Fluor, Cyclohexyl, Phenyl oder substituiertes Phenyl, Phenylalkyl oder Naphthyl bedeuten; und R₄ sich in Position 2 befindet und 12 Kohlenstoffatome aufweist und die Seitenkette an das Kohlenstoffatom des Tetrazolringes gebunden ist.

29. Verbindung nach Anspruch 28, bestehend aus
(±)-2-Dodecyl-α-methyl-α-phenyl-N-(2,4,6-trimethoxyphenyl)-2H-tetrazol-5-acetamid;
(±)-2-Dodecyl-α-(4-fluorphenyl)-N-(2,4,6-trimethoxyphenyl)-2H-tetrazol-5-acetamid;
(±)-2-Dodecyl-α-2-naphthalenyl-N-(2,4,6-trimethoxyphenyl)-2H-tetrazol-5-acetamid;
(±)-α-([1,1'-Biphenyl]-4-yl)-2-Dodecyl-N-(2,4,6-trimethoxyphenyl)-2H-tetrazol-5-acetamid;
(±)-2-Dodecyl-α-methyl-N-(2,4,6-trimethoxyphenyl)-2H-tetrazol-5-acetamid;
(±)-2-Dodecyl-α-phenylmethyl-N-(2,4,6-trimethoxyphenyl)-2H-tetrazol-5-acetamid;
(±)-2-Dodecyl-α-cyclohexyl-N-(2,4,6-trimethoxyphenyl)-2H-tetrazol-5-acetamid;
(±)-2-Dodecyl-α-phenyl-N-(2,4,6-trimethoxyphenyl)-2H-tetrazol-5-acetamid [α]_{D}=-58° (1% in CH₃OH);
(±)-2-Dodecyl-α-phenyl-N-(2,4,6-trimethoxyphenyl)-2H-tetrazol-5-acetamid [α]_{D}=-55,1° (1% in CH₃OH);
(±)-N-[2,6-Bis(1-methylethyl)phenyl]-2-dodecyl-α-fluor-α-phenyl-2H-tetrazol-5-acetamid; oder
(±)-2-Dodecyl-α-fluor-α-phenyl-N-(2,4,6-trimethoxyphenyl)-2H-tetrazol-5-acetamid.

30. Verbindung nach Anspruch 1, worin R₄ an das Kohlenstoffatom des Tetrazolringes gebunden ist und sich die Seitenkette in Position 2 des Tetrazolringes befindet.

31. Verbindung nach Anspruch 30, bestehend aus
N-[2,6-Bis(1-methylethyl)phenyl]-5-decyl-2H-tetrazol-2-acetamid;
N-[2,6-Bis(1-methylethyl)phenyl]-5-dodecyl-2H-tetrazol-2-acetamid;
(±)-N-[2,6-Bis(1-methylethyl)phenyl]-5-dodecyl-α-phenyl-2H-tetrazol-2-acetamid;
(±)-N-[2,6-Bis(1-methylethyl)phenyl]-5-dodecyl-α-pentyl-2H-tetrazol-2-acetamid;
(±)-N-[2,6-Bis(1-methylethyl)phenyl]-5-(dodecylthio)-α-phenyl-2H-tetrazol-2-acetamid;
(±)-5-Decyl-α-phenyl-N-(2,4,6-trimethoxyphenyl)-2H-tetrazol-2-acetamid;
5-Dodecyl-N-(2,4,6-trimethoxyphenyl)-2H-tetrazol-2-acetamid;
(±)-5-Dodecyl-α-phenyl-N-(2,4,6-trimethoxyphenyl)-2H-tetrazol-2-acetamid;
(±)-5-Dodecyl-α-pentyl-N-(2,4,6-trimethoxyphenyl)-2H-tetrazol-2-acetamid;
(±)-N-(2,4-Difluorphenyl)-5-dodecyl-α-phenyl-2H-tetrazol-2-acetamid;
5-Dodecyl-α,α-dimethyl-N-(2,4,6-trimethoxyphenyl)-2H-tetrazol-2-acetamid;
(±)-5-(Dodecylthio)-α-phenyl-N-(2,4,6-trimethoxyphenyl)-2H-tetrazol-2-acetamid; oder
(±)-5-(Dodecylsulfinyl)-α-phenyl-N-(2,4,6-trimethoxyphenyl)-2H-tetrazol-2-acetamid.

32. Pharmazeutische Zusammensetzung, umfassend eine Verbindung nach einem der Ansprüche 1 bis 31 und einen pharmazeutisch annehmbaren, flüssigen oder festen Träger, Verdünner oder Zusatz.

33. Verwendung einer Verbindung nach einem der Ansprüche 1 bis 31 zur Herstellung von pharmazeutischen Zusammensetzungen mit Wirkung als Inhibitoren der Acyl-CoA:Cholesterin-Acyltransferase (ACAT), welche die Absorption von mit der Nahrung aufgenommenen Cholesterin senken und eine Therapie gegen Hypercholesterinämie ergeben.

34. Verfahren zur Herstellung einer Verbindung der Formel I 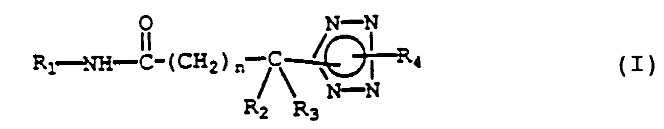 worin n gleich Null ist, R₂ und R₃ Wasserstoff bedeuten, und R₁ und R₄ gemäss Formel I in Anspruch 1 definiert sind, gekennzeichnet durch die nachstehenden Synthesewege:

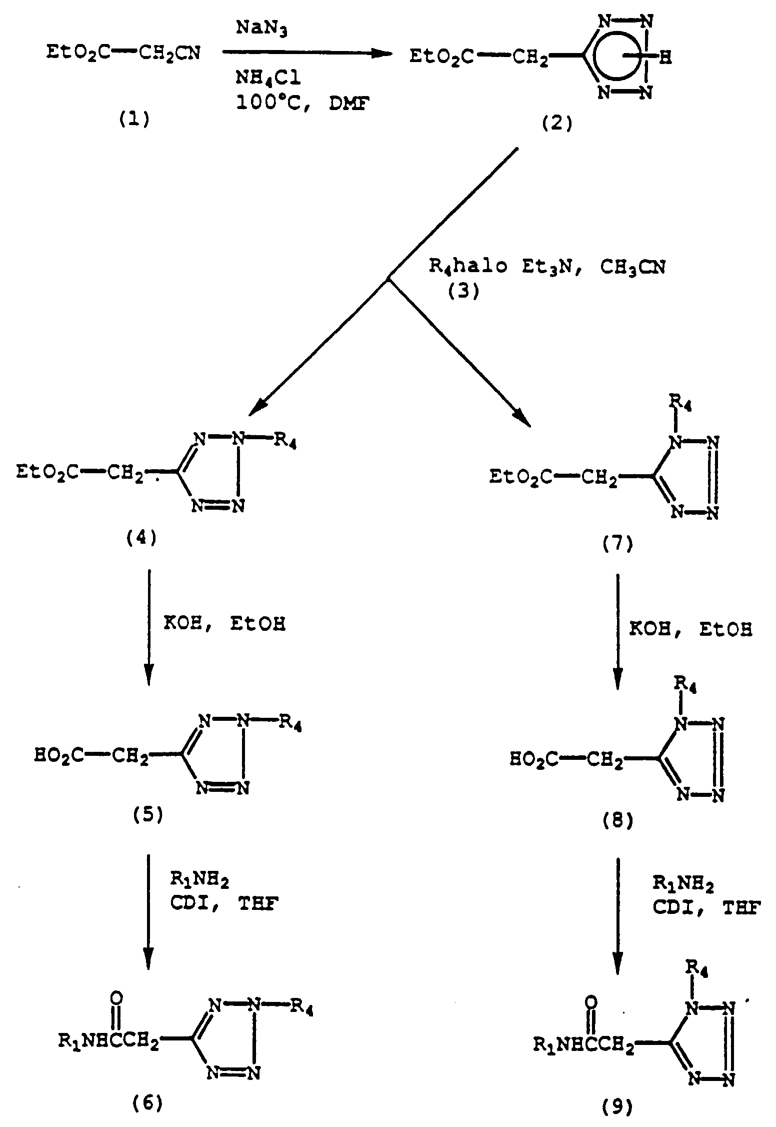

35. Verfahren zur Herstellung einer Verbindung der Formel I 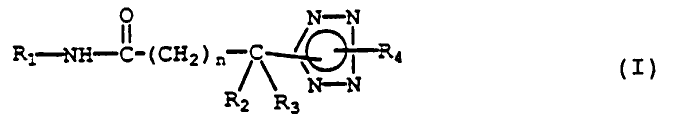 worin n gleich Null ist, R₁, R₂, R₃ und R₄ gemäss Formel I in Anspruch 1 definiert sind, gekennzeichnet durch die nachstehenden Synthesewege:

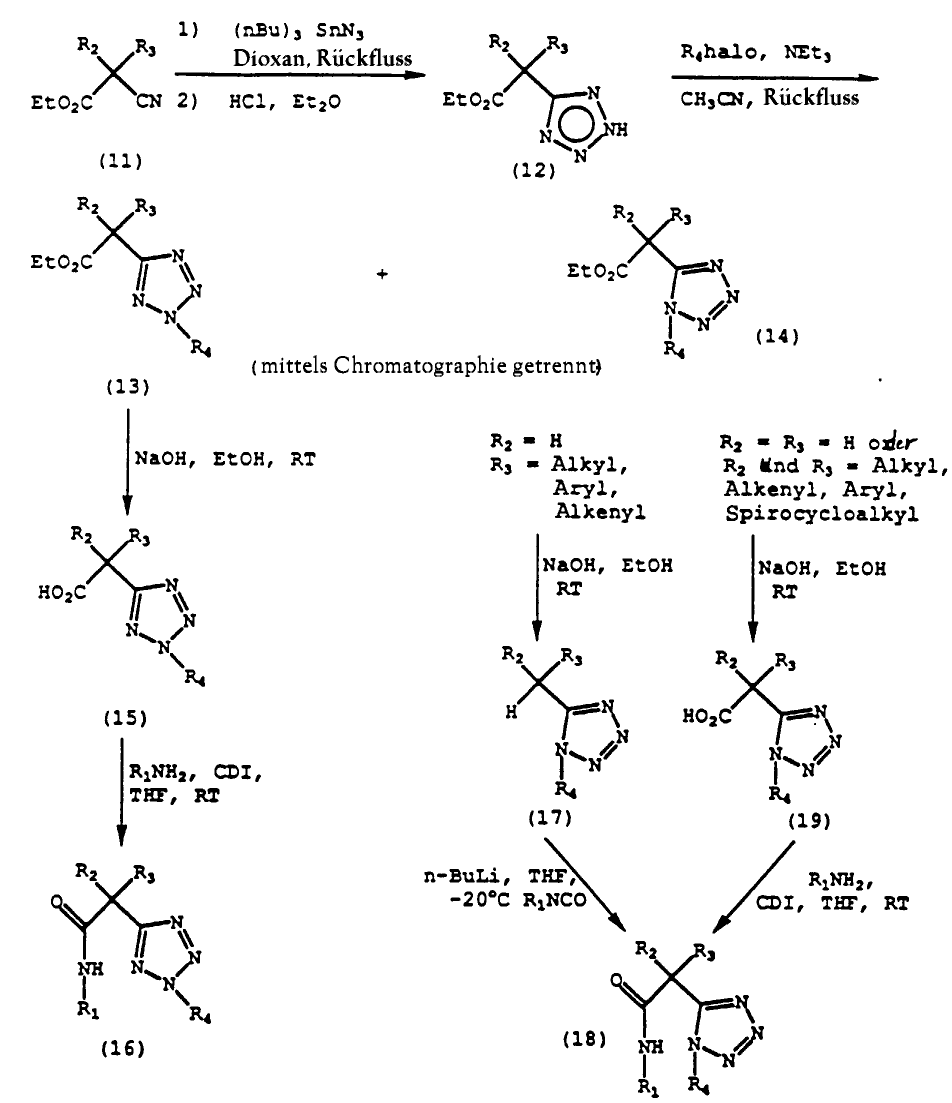

36. Verfahren zur Herstellung einer Verbindung der Formel I 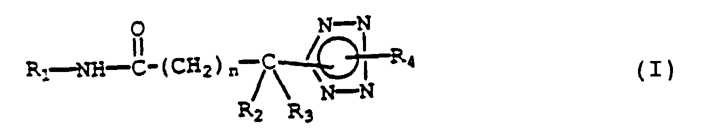 worin n gleich Eins oder Zwei ist, R₂ und R₃ Wasserstoff bedeuten, und R₁ und R₄ gemäss Formel I in Anspruch 1 definiert sind, gekennzeichnet durch die nachstehenden Synthesewege:

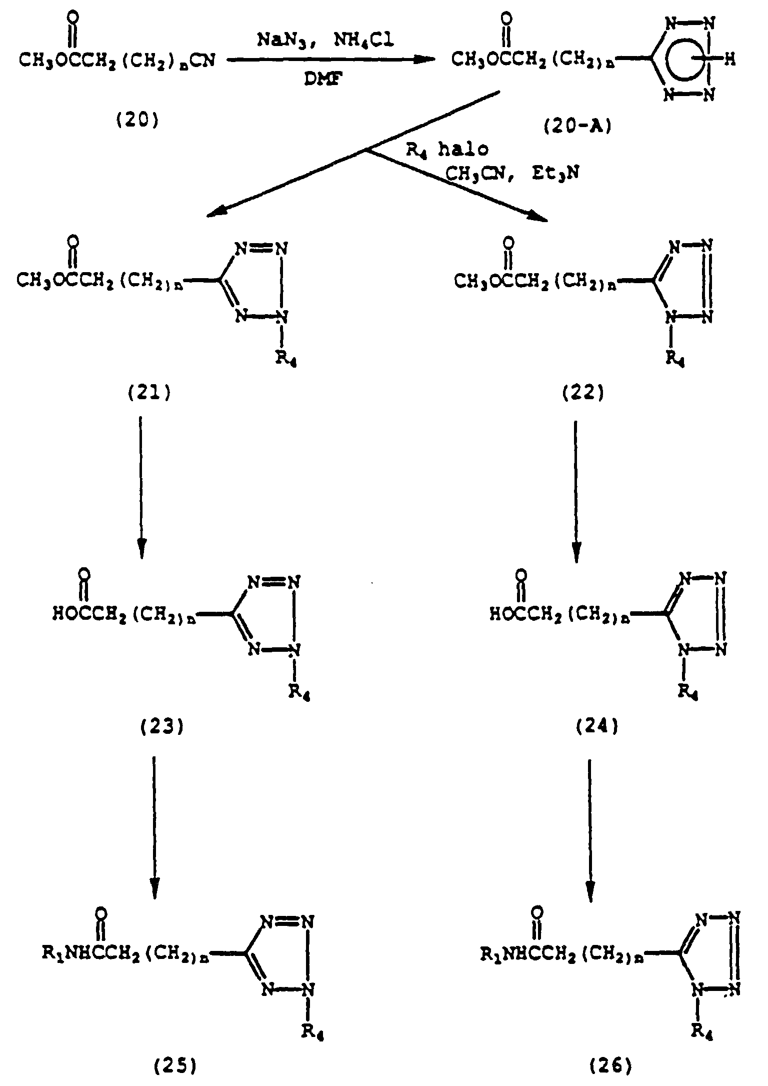

37. Verfahren zur Herstellung einer Verbindung der Formel I nach Anspruch 1, 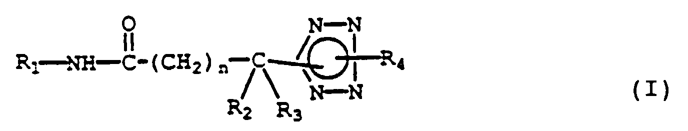 worin n gleich Eins ist, R₂ Wasserstoff und R₃ (X) Phenyl, substituiertes Phenyl, Alkyl, Alkenyl oder Heteroaryl bedeutet, gekennzeichnet durch die nachstehenden Synthesewege:

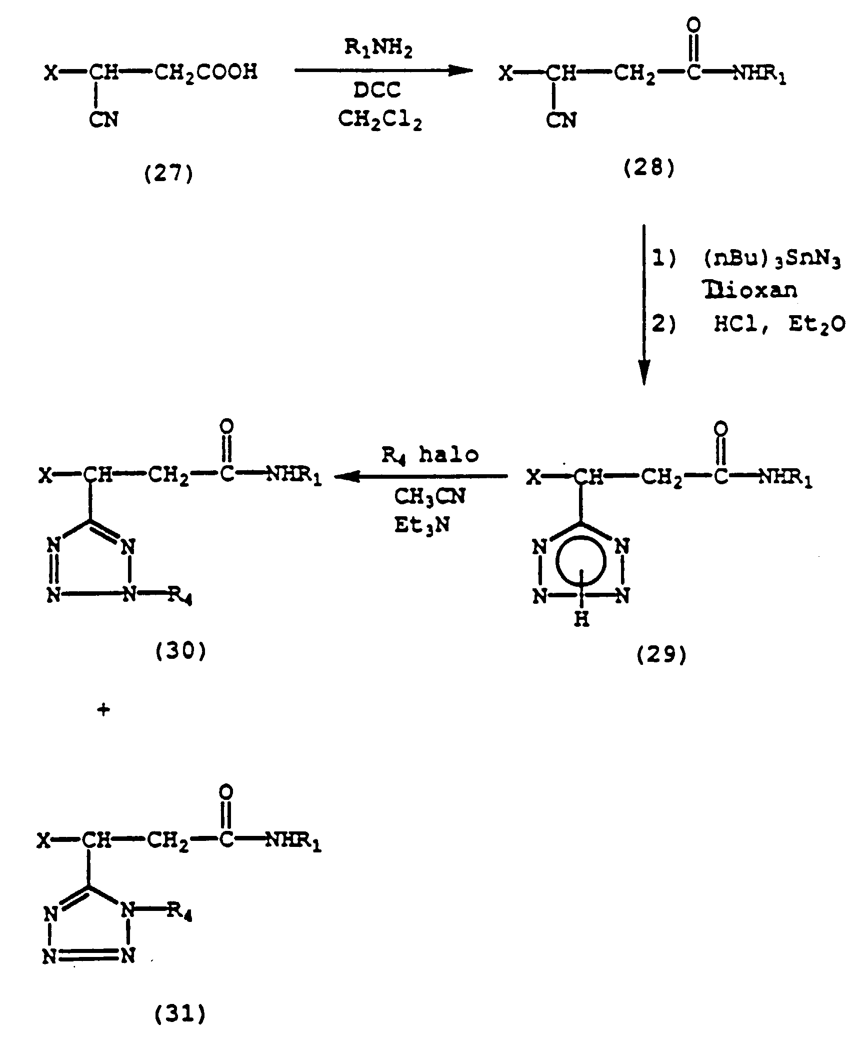

38. Verfahren zur Herstellung einer Verbindung der Formel I nach Anspruch 1, 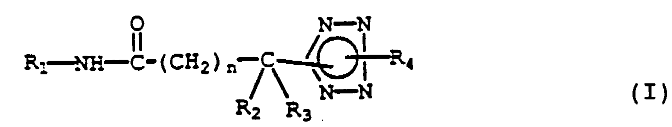 worin n gleich Null ist und R₂ und R₃ Alkyl oder Aryl bedeuten, gekennzeichnet durch die nachstehenden Synthesewege:

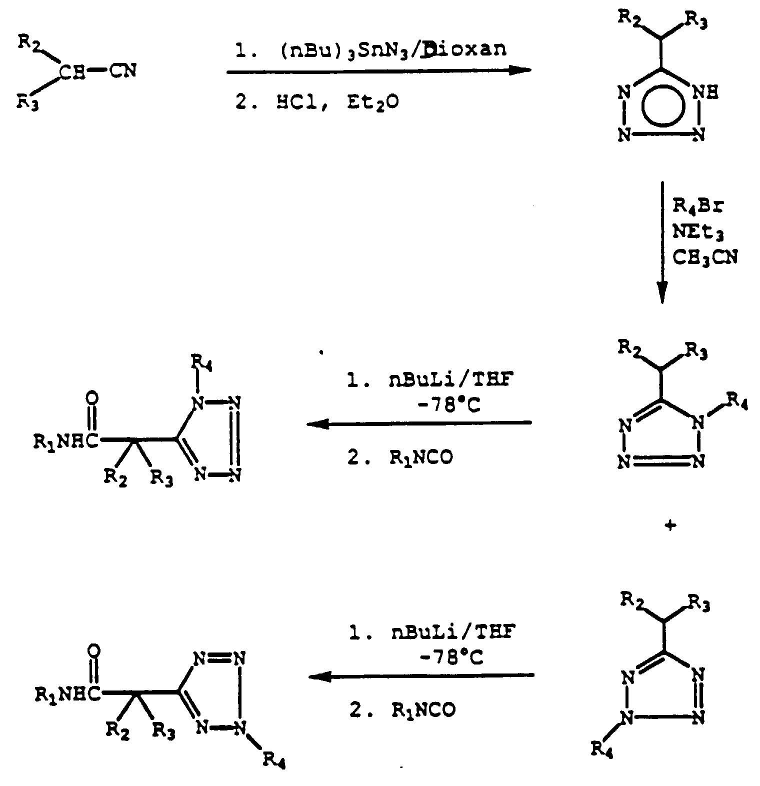

39. Verfahren zur Herstellung einer Verbindung der Formel I nach Anspruch 1, 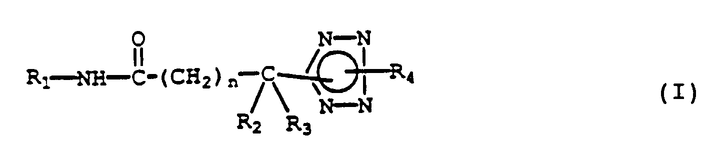 worin n gleich Zwei ist, R₂ Wasserstoff und R₃ Phenyl oder substituiertes Phenyl bedeutet, gekennzeichnet durch die nachstehenden Synthesewege:

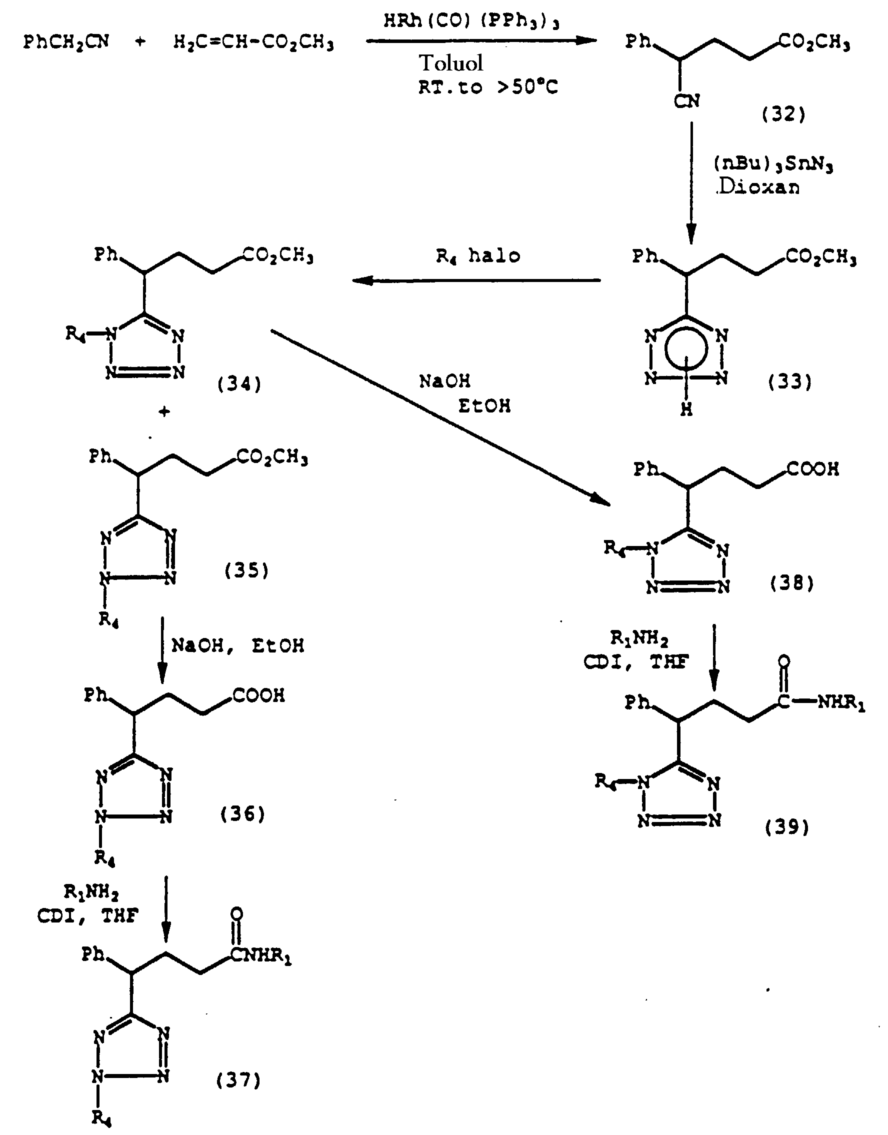

40. Verfahren zur Herstellung einer Verbindung der Formel I nach Anspruch 1, 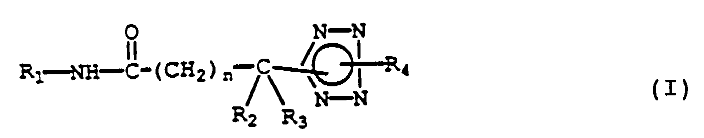 worin n gleich Null ist, R₂ Wasserstoff und R₃ Heteroaryl, 1- oder 2-Naphthyl oder substituiertes Phenyl bedeutet, gekennzeichnet durch die nachstehenden Synthesewege:

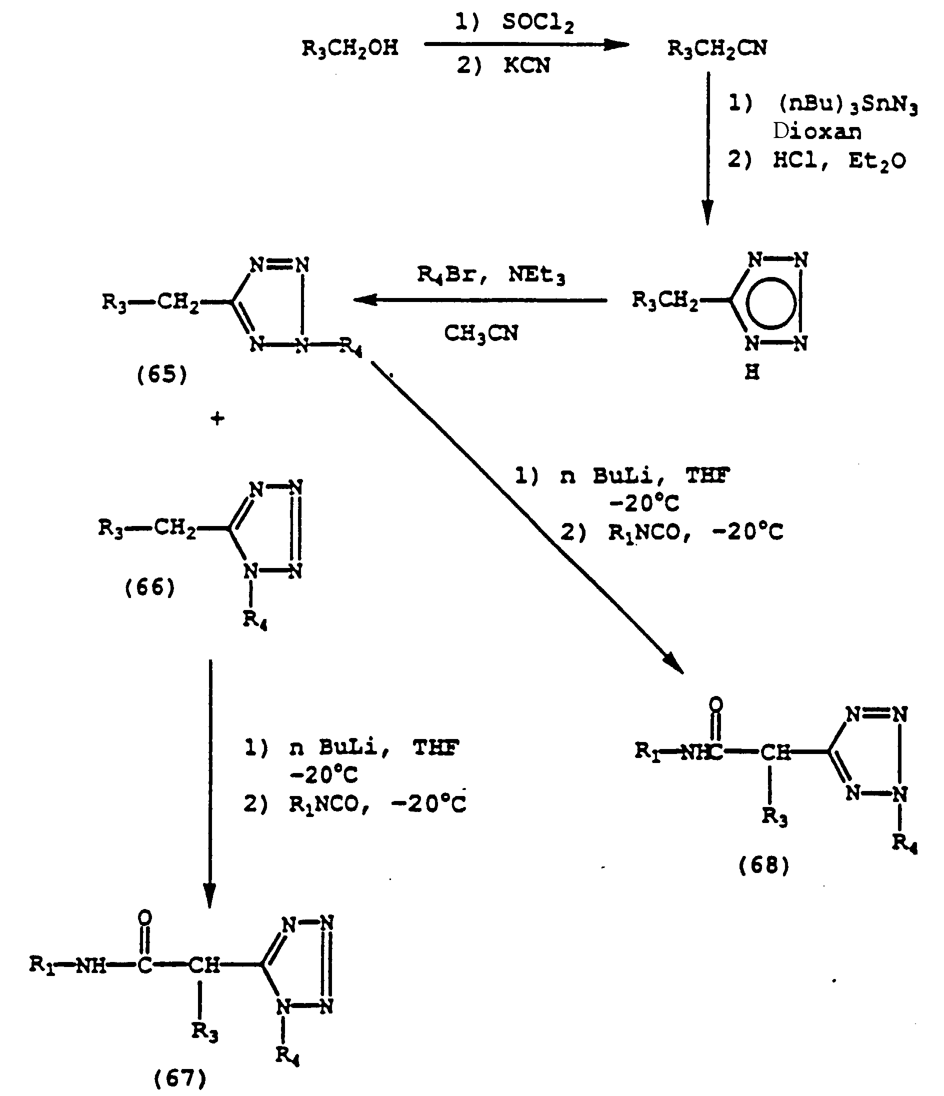

41. Verfahren zur Herstellung einer Verbindung der Formel I nach Anspruch 1, 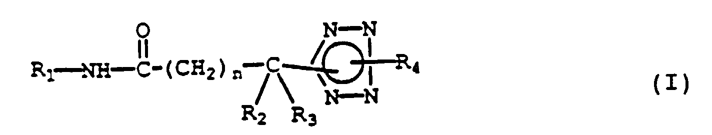 worin n gleich Eins ist, gekennzeichnet durch die nachstehenden Synthesewege:

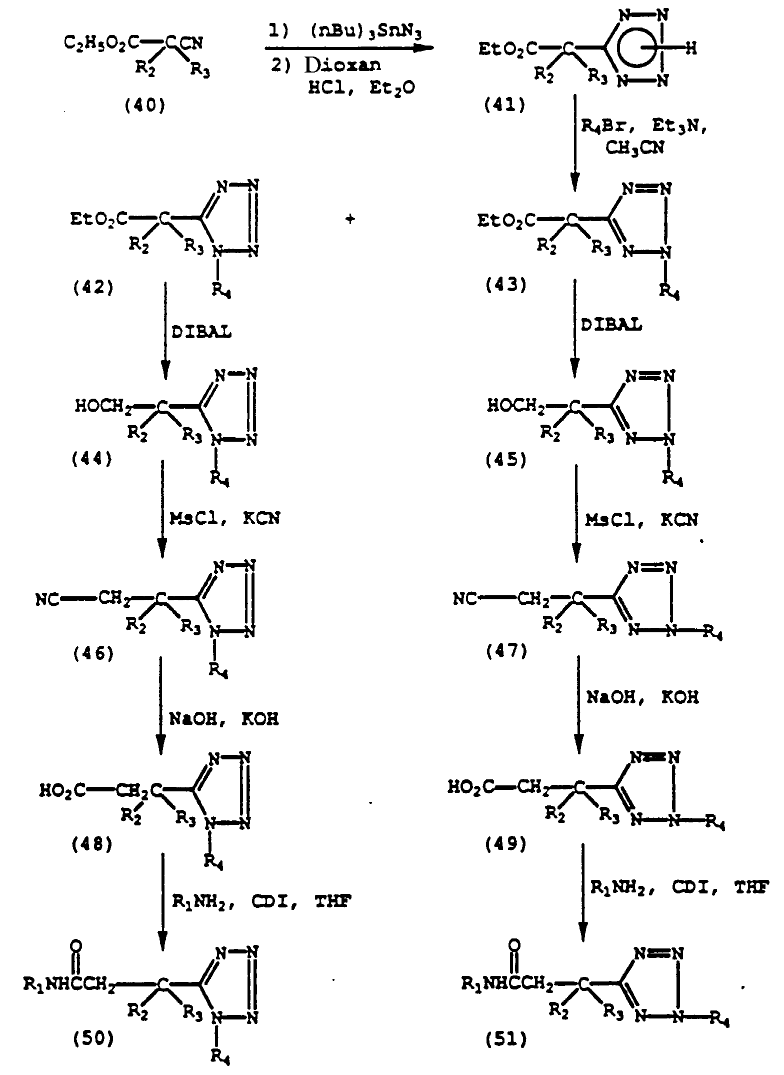

42. Verfahren zur Herstellung einer Verbindung der Formel I nach Anspruch 1, 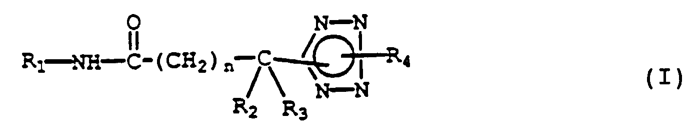 worin n gleich Eins ist und R₃ Heteroaryl bedeutet, gekennzeichnet durch die nachstehenden Synthesewege:

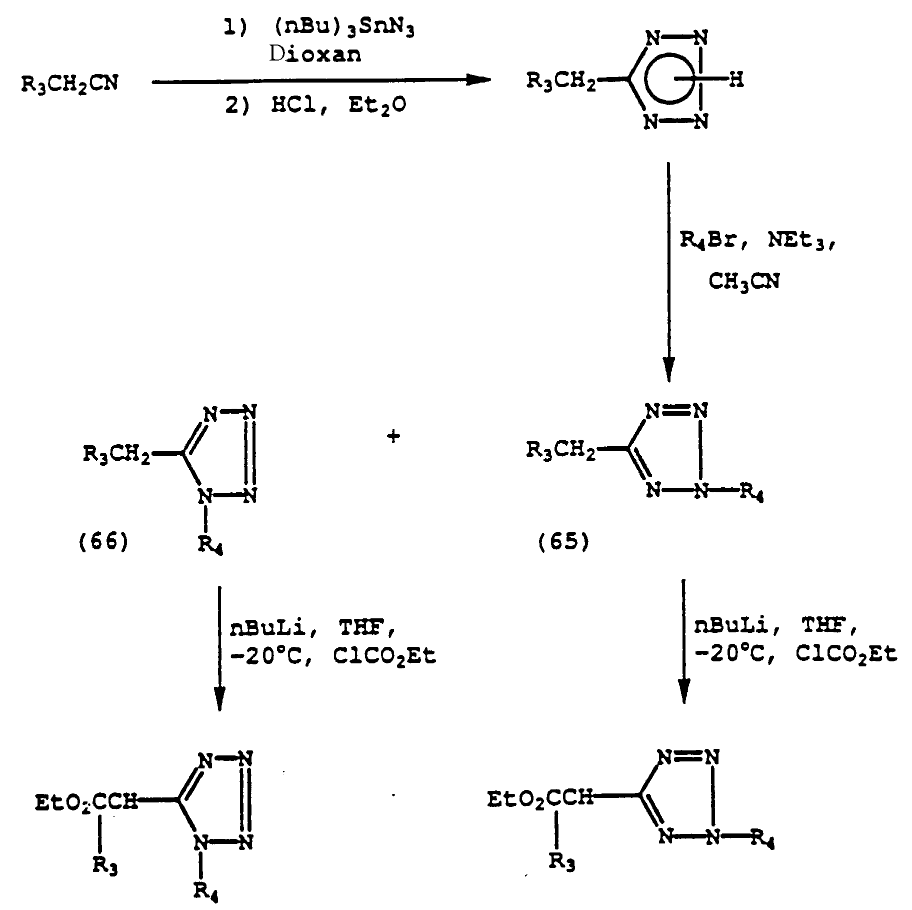

43. Verfahren zur Herstellung einer Verbindung der Formel I nach Anspruch 1, 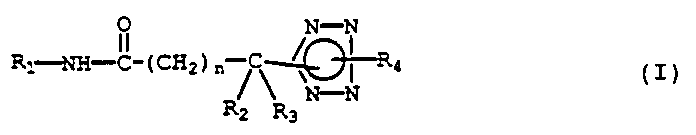 worin n gleich Zwei ist, mit der Massgabe, dass zumindest einer von R₂ und R₃ anderes als Wasserstoff bedeutet, gekennzeichnet durch die nachstehenden Synthesewege:

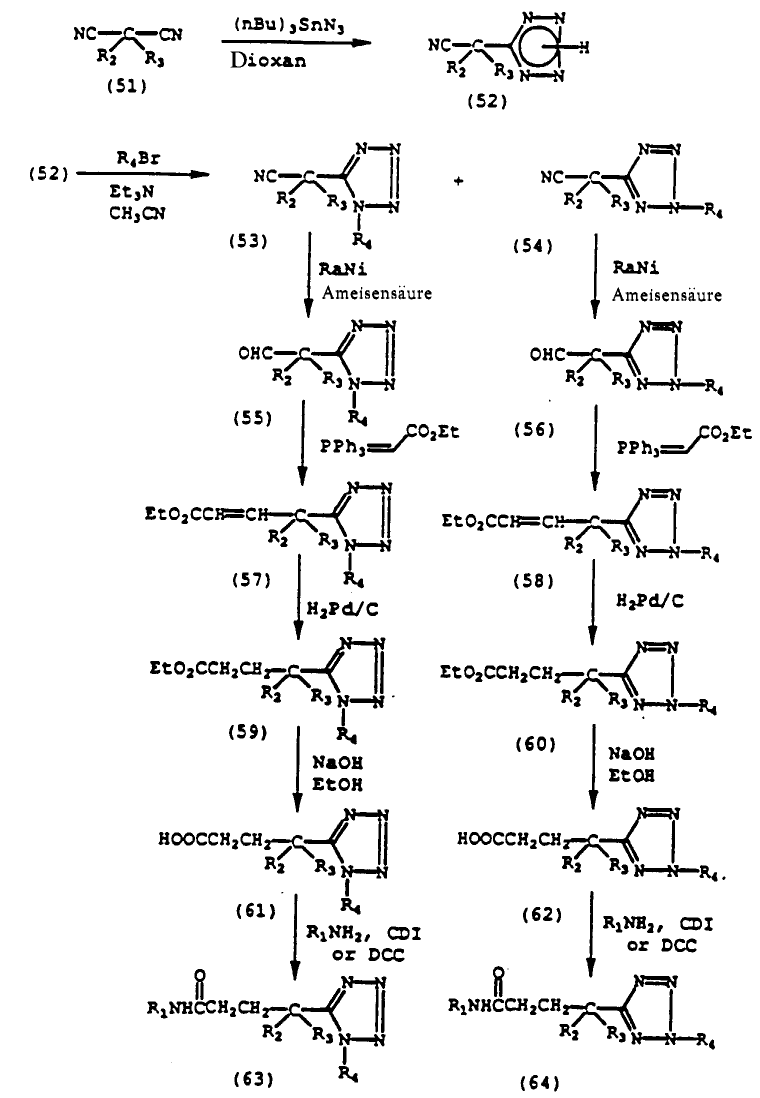

44. Verfahren zur Herstellung einer Verbindung der Formel I nach Anspruch 1, 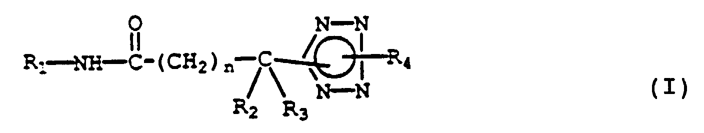 worin n gleich Null ist und R₃ F oder OH bedeutet, gekennzeichnet durch die nachstehenden Synthesewege:

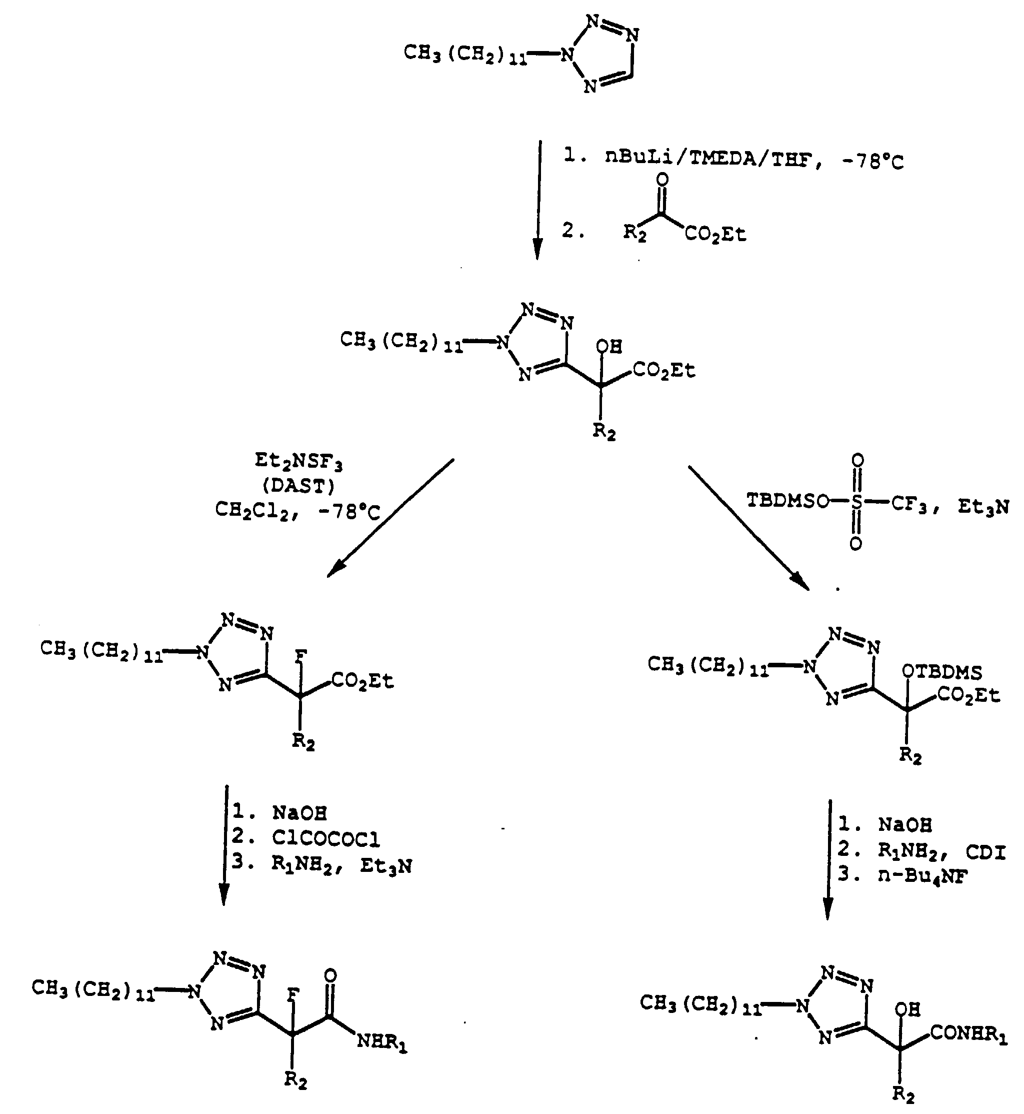

45. Verfahren zur Herstellung einer pharmazeutischen Zusammensetzung, gekennzeichnet durch die Kombination einer nach einem der Ansprüche 34 bis 43 hergestellten Verbindung mit einem pharmazeutisch annehmbaren, flüssigen oder festen Träger, Verdünner oder Zusatz.

## Revendications

1. Un composé représenté par la formule: 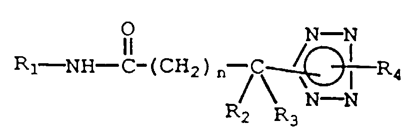 dans laquelle:
n est égal à zéro, un ou deux;
R₁ est sélectionné parmi:
(a) un radical phényl non substitué ou substitué par un à trois substituants sélectionnés parmi:
un radical alkyl comportant 1 à 4 atomes de carbone, linéaire ou ramifié, un radical alkoxy comportant 1 à 3 atomes de carbone, linéaire ou ramifié,
un radical alkylthio comportant 1 à 3 atomes de carbone, linéaire ou ramifié,
hydroxy,
phényl,
un atome de fluore, de chlore, de brome, un groupe nitro,
cyano, trifluorométhyl,
-COOH,
-COOalkyl dans lequel le radical alkyl comporte 1 à 4 atomes de carbone et est linéaire ou ramifié,
- (CH2)ₘNR₅R₆ où m est égal à zéro ou un et chacun des radicaux R₅ et R₆ représente un groupe hydrogène ou un radical alkyl linéaire ou ramifié comportant 1 à 4 atomes de carbone;
(b) un groupe 1- ou 2-naphtyl non substitué ou substitué par un à trois substituants sélectionnés parmi:
un radical alkyl linéaire ou ramifié comportant 1 à 4 atomes de carbone, un radical alkoxy linéaire ou ramifié commportant 1 à 3 atomes de carbone,
hydroxy,
un atome de fluore, de chlore, de brome, un groupe nitro, cyano, trifluorométhyl,
-COOH,
-COOalkyl où le radical alkyl est linéaire ou ramifié et comporte 1 à 4 atomes de carbone,
-(CH2)ₘNR₅R₆ où m, R₅ et R₆ sont tels que définis ci-dessus;
(c) le groupe: 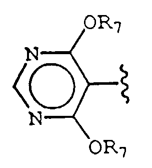 où:
R₇ est un radical alkyl inférieur comportant 1 à 3 atomes de carbone linéaire ou ramifié;
(d) le groupe: 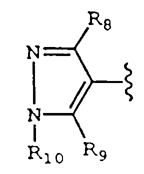 où:
R₈ et R₉ sont des radicaux alkyl linéaires ou ramifiés comportant 1 à 4 atomes de carbone ou un groupe phényl, et
R₁₀ est un groupe hydrocarboné, linéaire ou ramifié, comportant 1 à 18 atomes de carbone saturé ou insaturé, contenant une double liaison ou deux doubles liaisons non adjacentes; un groupe phényl; un groupe phényl substitué par un à trois substituants sélectionnés parmi des radicaux alkyl linéaires ou ramifiés comportant 1 à 4 atomes de carbone, des radicaux alkoxy linéaires ou ramifiés comportant 1 à 3 atomes de carbone, des groupes hydroxy, un atome de fluor, de chlore, de brome, un groupe nitro, cyano, trifluorométhyl, -COOH, -COOalkyl, le radical alkyl linéaire ou ramifié comportant 1 à 4 atomes de carbone ou un groupe (CH2)ₘNR₅R₆ où m, R₅ et R₆ sont tels que définis ci-dessus; ou un groupe hétérocyclique sélectionné parmi 2-, 3-, ou 4-pyridyl, 2-, 4-, ou 5-pyrimidinyl, 2- ou 3-pyrazinyl, 2-, 3-, 4-, 5-,6-, 7-, ou 8-quinolinyl, ou 3- ou 4-pyridazinyl et les N-oxides en dérivant;
(e) le groupe: 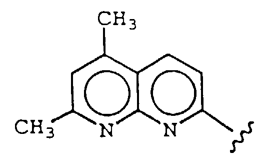
(f) le groupe: 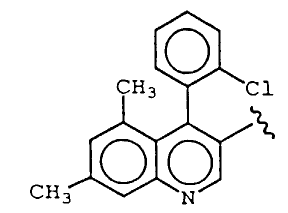
(g) un groupe hydrocarboné linéaire ou ramifié comportant 1 à 18 atomes de carbone qui est saturé ou insaturé contenant une double liaison ou deux doubles liaisons non-adjacentes;
(h) un groupe cycloalkyl comportant 3 à 8 atomes de carbone;
(i) un groupe hétéroaromatique choisi parmi 2-, 3-, ou 4-pyridyl qui est non substitué ou substitué par un groupe alkyl comportant 1 à 4 atomes de carbone ou 2-, 4- ou 5-pyrimidinyl, et les N-oxydes en dérivant;
(j) le groupe: 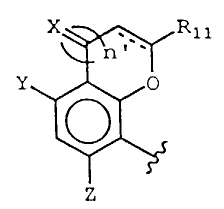 où:
--- signifie une liaison simple ou double;
Y et Z représentent chacun indépendamment l'un de l'autre un groupe hydrogène, un radical alkyl linéaire ou ramifié comportant 1 à 4 atomes de carbone, un groupe alkoxy comportant 1 à 3 atomes de carbone ou un groupe halo;
X représente un atome d'oxygène ou deux atomes d'hydrogène;
R¹¹ représentant un atome d'hydrogène ou un radical alkyl linéaiare ou ramifié comportant 1 à 4 atomes de carbone, et n'est égal à zéro ou un; ou
(k) est sélectionné parmi les groupes: 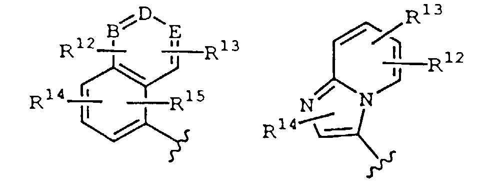 et 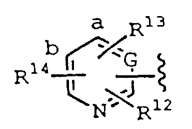 où
R¹², R¹³, R¹⁴ et R¹⁵ sont, sélectionnés indépendamment les uns des autres, parmi un atome d'hydrogène, un radical halo, un groupe alkyl linéaire ou ramifié comprenant 1 à 4 atomes de carbone, un groupe alkoxy comportant 1 à 3 atomes de carbone, et un groupe alkylthio comportant 1 à 3 atomes de carbone, un groupe cycloalkylthio comportant de 5 à 7 atomes de carbone, un groupe phénylalkylthio dans lequel le radical alkyl comprend 1 à 4 atomes de carbone, un groupe phénylthio substitué, hétéroarylthio, ou hétéroaryloxy; et B, D, E et G sont des atomes d'azote ou de carbone où un ou plusieurs des groupes B, D, et E correspondent à un atome d'azote; à la condition que lorsque G représente un atome d'azote, le groupe est lié à l'atome d'azote de la formule I à la position 4- ou 5- du cycle pyrimidine (a et b);
où R₂ et R₃ sont identiques ou différents l'un de l'autre et sont sélectionnés parmi:
(a) un groupe hydrogène, un groupe halo ou l'un des radicaux R₂ ou R₃ représente un groupe hydroxy;
(b) un radical alkyl linéaire ou ramifié comportant 1 à 12 atomes de carbone, ou un radical cycloalkyl comportant 3 à 8 atomes de carbone;
(c) un groupe phényl ou phénylalkyl où le radical akyl comporte 1 à 4 atomes de carbone et dans lequel le groupe phényl est non substitué ou substitué parmi 1 à 3 substituants sélectionnés parmi des radicaux alkyl linéaires ou ramifiés comportant 1 à 4 atomes de carbone, des radicaux alkoxy linéaires ou ramifiés comportant 1 à 4 atomes de carbone, les groupes alkylthio linéaires ou ramifiés comportant 1 à 4 atomes de carbone, hydroxy, un atome de fluore, de chlore, de brome, un groupe trifluorométhyl , cyano, nitro, phényl ou (CH2)ₘNR₅R₆ où m, R₅ et R₆ sont tels que définis ci-dessus;
(d) un groupe akenyl linéaire ou ramifié, comportant de 2 à 6 atomes de carbone; ou
(e) R₂ et R₃ forment ensemble avec l'atome de carbone auquel ils sont liés un groupe alkylidène comportant 1 à 4 atomes de carbone un goupe benzylidène ou un groupe spiroalkyl comportant 3 à 7 atomes de carbone; ou
(f) lorsque R₂ représente un atome d'hydrogène, F, un radical alkyl comportant 1 à 12 atomes de carbone, R₃ est un radical hétéroaryl sélectionné parmi les groupes hétérocycliques bicycliques fusionnés ou monocycliques à 5 ou 6 membres contenant au moins 1 à 4 hétéroatomes dans au moins 1 cycle, lesdits hétéroatomes étant sélectionnés parmi les atomes d'azote, d'oxygène, ou de soufre et leur combinaison, ledit groupe hétérocyclique étant non-substitué ou substitué par un groupe alkyl comportant 1 à 4 atomes de carbone et les N-oxydes en dérivant;
(g) le groupe 1- ou 2-naphtyl qui est non substitué ou substitué par un ou trois substituants sélectionnés parmi:
un radical alkyl linéaire ou ramifié comprenant 1 à 4 atomes de carbone;
un radical alkoxy linéaire ou ramifié comprenant 1 à 3 atomes de carbone,
où R₄ est une chaîne hydrocarbonée linéaire ou ramififée comportant 1 à 20 atomes de carbone et est saturé ou non-saturé et comporte une double liaison ou deux doubles liaisons non adjacentes, ou correspond à un alkylthio comprenant 1 à 20 atomes de carbone et est saturé; ou un sel pharmaceutiquement acceptable ou un isomère énantiomérique individuel en dérivant.

2. Un composé selon la revendication 1, dans lequel R₄ est en position 2 sur le cycle tétrazole et la chaîne latérale est liée à l'atome de carbone du cycle tétrazole.

3. Un composé selon la revendication 2, dans lequel n est égal à zéro.

4. Un composé selon la revendication 3, dans lequel chacun des radicaux R₂ et R₃ représente un atome d'hydrogène.

5. Un composé selon la revendication 4, dans lequel R₄ est une chaîne hydrocarbonée saturée et comporte de 8 à 18 atomes de carbone.

6. Un composé selon la revendication 5, dans lequel R₁ est un radical phényl ou un radical phényl substitué.

7. Un composé selon la revendication 6, qui correspond à:
N-[2,6-Bis(1-méthyléthyl)phényl]-2-dodécyl-2H-tétreazole-5-acétamide;
2-dodécyl-N-(2,4,6-triméthoxyphényl)-2H-tétrazole-5-acétamide;
N-(2,4-difluorophényl)-2-dodécyl-2H-tétrazole-5-acétamide;
2-tétradécyl-N-(2,4,6-tri-méthoxyphényl)-2H-tétrazole-5-acétamide.

8. Un composé selon la revendication 5, dans lequel R₁ présente un groupe: 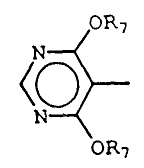 où:
R₇ est un radical alkyl inférieur comportant 1 à 3 atomes de carbone et est linéaire ou ramifié.

9. Un composé selon la revendication 8, correspondant à:
N-(4,6-diméthoxy-5-pyrimidinyl)-2-dodécyl-2H-tétrazole-5-acétamide; ou
N-(4,6-diméthoxy-5-pyrimidinyl)-2-dodécyl-1H-tétrazole-5-acétamide.

10. Un composé selon la revendication 5, dans lequel R₁ est un groupe hétéroaromatique sélectionné parmi 2-, 3-, ou 4-pyridyl qui est non-substitué ou substitué par un groupe alkyl comportant 1 à 4 atomes de carbone, ou 2-, 4- ou 5-pyrimidinyl et les N-oxydes en dérivant.

11. Un composé selon la revendication 10, qui correspond au:
2-dodécyl-N-(3-méthyl-2-pyridinyl)-2H-tétrazole-5-acétamide.

12. Un composé selon la revendication 5, dans lequel R₁ représente le groupe: 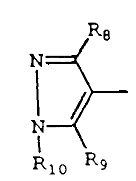 où:
R₈ et R₉ sont des radicaux alkyl linéaires ou ramifiés comportant 1 à 4 atomes de carbone ou un groupe phényl, et
R₁₀ est un groupe hydrocarboné, linéaire ou ramifié, comportant 1 à 18 atomes de carbone, et saturé ou insaturé, contenant une double liaison ou deux doubles liaisons non adjacentes; un groupe phényl; un groupe phényl substitué par un à trois substituants sélectionnés parmi des radicaux alkyl linéaires ou ramifiés comportant 1 à 4 atomes de carbone, des radicaux alkoxy linéaires ou ramifiés comportant 1 à 3 atomes de carbone, des groupes hydroxy, un atome de fluor, de chlore, de brome, un groupe nitro, cyano, trifluorométhyl, -COOH, -COO alkyl, le radical alkyl linéaire ou ramifié comportant 1 à 4 atomes de carbone ou un groupe (CH₂)ₘNR₅R₆ où m, R₅ et R₆ sont tels que définis ci-dessus; ou un groupe hétérocyclique sélectionné parmi 2-, 3-, ou 4-pyridyl, 2-, 4-, ou 5-pyrimidinyl, 2- ou 3-pyrazinyl, 2-, 3-, 4-, 5-,6-, 7-, ou 8-quinolinyl, ou 3- ou 4-pyridazinyl et les N-oxides en dérivant.

13. Un composé selon la revendication 12, correspondant au:
2-dodécyl-N-(1,3,5 -triméthyl-1H-pyrazol-4-yl)-2H-tétrazole-5-acétamide; ou
1-dodécyl-N-(1,3,5-triméthyl-1H-pyrazol-4-yl)-1H-tétrazole-5-actamide.

14. Un composé selon la revendication 3, dans lequel l'un des groupes R₂ et R₃ représente un atome d'hydrogène et l'autre représente un groupe phényl substitué ou non substitué.

15. Un composé selon la revendication 14, dans lequel R₄ est une chaîne hydrocarbonée saturée comportant 8 à 18 atomes de carbone.

16. Un composé selon la revendication 15, dans lequel R₁ est un radical phényl substitué ou non substitué.

17. Un composé selon la revendication 16, correspondant à:
(±) 2-dodécyl-α-phényl-N-(2,4,6-triméthoxyphényl)-2H-tétrazole-5-acétamide,
(±) 2-dodécyl-N,α-diphényl-2H-tétrazole-5-acétamide,
(±) -N-[2,6-bis(1-méthyléthyl)phényl]-2-dodécyl-α-phényl-2H-tétrazole-5-acétamide,
(±)-N-(2,4-difluorophényl)-2-dodécyl-α-phényl-2H-tétrazole-5-acétamide,
(±) -2-octyl-α-phényl-N-(2,4,6-triméthoxy-phényl)-2H-tétrazole-5-acétamide, ou
(±) -2-hexadécyl-α-phényl-N-(2,4,6-triméthoxyphényl)-2H-tétrazole-5-acétamide.

18. Un composé selon la revendication 15 correspondant à:
(±) -N-(4,6-diméthoxy-5-pyrimidinyl)s-2-dodécyl-α-phényl-2H-tétrazole-5-acétamide,
(±) -N-(5,7-diméthyl-1,8-naphtyridine-2-yl)-2-dodécyl-α-phényl-2H-tétrazole-5-acétamide,
(±)-2-dodécyl-α-phényl-N-(1,3,5-triméthyl-1H-pyrazol-4-yl)-2H-tétrazole-5-acétamide,
(±) -N-cyclopropyl-2-dodécyl-α-phényl-2H-tétrazole-5-acétamide,
(±)-2-dodécyl-α-phényl-N-2-pyridinyl-2H-tétrazole-5-acétamide,
(±)-2-dodécyl-N-(3-méthyl-2-pyridinyl)-α-phényl-2H-tétrazole-5-acétamide,
(±) -2-dodécyl-N-(3-méthyl-2-pyridinyl)-2-phényl-2H-tétrazole-5-acétamide, N-oxyde ou
(±)-N-(1,1-diméthyléthyl)-2-dodécyl-α-phényl-2H-tétrazole-5-acétamide.

19. La composition de la revendication 3, dans laquelle R₃ présente un groupe hétérocyclique bicyclique fusionné ou monocyclique à 5 ou 6 membres contenant au moins 1 à 4 hétéroatomes dans au moins un cycle, ledit hétéroatome étant sélectionné parmi l'atome d'azote, d'oxygène, ou de soufre, et leurs combinaisons, ledit groupe hétérocyclique étant non-substitué ou substitué par un groupe alkyl comportant 1 à 4 atomes de carbone, et les N-oxydes en dérivant.

20. Un composé selon la revendication 19, dans lequel R₃ représente un groupe 2-, 3-, ou 4-pyridyl.

21. Un composé selon la revendication 20 qui correspond au:
(±)-2-dodécyl-α-(2-pyridyl)-N-(2,4,6-triméthoxyphényl)-2H-tétrazole-5-acétamide, ou
(±)-N-[2,6-bis(1-méthyléthyl)phényl]-2-dodécyl-α-2-pyridinyl-2H-tétrazole-5-acétamide.

22. Un composé selon la revendication 3, dans lequel chacun des groupes R₂ et R₃ ne représentent pas un atome d'hydrogène.

23. Un composé selon la revendication 22, qui correspond au:
2-dodécyl-α,α-diméthyl-N-(2,4,6-triméthoxyphényl)-2H-tétrazole-5-acétamide,
2-dodécyl-α,α'-(2-propényl)-N-(2,4,6-triméthoxyphényl)-2H-tétrazole-5-acétamide,
1-(2-dodécyl-2H-tétrazol-5-yl)-N-(2,4,6-triméthoxyphényl)cyclopentanecarboxamide, ou
2-tridécyl-α,α-diméthyl-N-(2,4,6-triméthoxyphényl)-2H-tétrazole-5-acétamide.

24. Un composé selon la revendication 2, dans lequel n est égal à un ou deux.

25. Un composé selon la revendication 24, dans lequel R₁ est un radical phényl non substitué ou substitué.

26. Un composé selon la revendication 25, qui correspond au:
2-dodécyl-N-(2,4,6-triméthoxyphényl)-2H-tétrazole-5-propanamide,
- N-(2,6-bis(1-méthyléthyl)phényl)-2-dodécyl-2H-tétrazole-5-propanamide,
- N-(2,4-difluorophényl)-2-dodécyl-2H-tétrazole-5-propanamide, ou
- 1-dodécyl-N-(2,4,6-triméthoxyphényl)-1H-tétrazole-5-propanamide.

27. Un composé selon la revendication 1, qui correspond au:
(±) -n-(2,4-difluorophényl)-1-dodécyl-α-phényl-1H-tétrazole-5-acétamide,
(±)-N-[2,6-bis(1-méthyléthyl)phényl]-1-dodécyl-α-phényl-1H-tétrazole-5-acétamide.

28. Un composé selon la revendication 6, dans lequel R1 représente 2,6-[1-méthyléthyl)phényl ou 2,4,6-triméthoxyphényl; n est égal à zéro; R2 et R3 sont chacun indépendamment l'un de l'autre un groupe hydrogène, un groupe méthyl, un groupe fluoro, cyclohéxyl, phényl, ou un groupe phényl substitué, phénylalkyl, ou naphthyl, et R₄ est en position 2 et comporte pour 12 atomes de carbone du cycle et est lié à l'atome de carbone du cycle tétrazole.

29. Un composé selon la revendication 28, qui correspond au:
(±)-2-dodécyl-α-méthyl-α-phényl-N-(2,4,6-triméthoxyphényl)-2H-tétrazole-5-acétamide,
(±) -2-dodécyl-α-(4-fluorophényl)-N-(2,4,6-triméthoxyphényl)-2H-tétrazole-5-acétamide,
(±) -2-dodécyl-α-naphtalényl-N-(2,4,6-triméthoxyphényl)-2H-tétrazole-5-acétamide,
(±) -α-([1,1'-biphényl]-4-yl)-2-dodécyl-N-(2,4,6-triméthoxy-phényl)-2H-tétrazole-5-acétamide,
(±) -2-dodécyl-α-méthyl-N-(2,4,6-triméthoxy-phényl)-2H-tétrazole-5-acétamide,
(±) -2-dodécyl-α-phénylméthyl-N-(2,4,6-triméthoxyphényl)-2H-tétrazole-5-acétamide,
(±) -2-dodécyl-α-cyclohexyl-N-(2,4,6-triméthoxy-phényl)-2H-tétrazole-5-acétamide,
(-) -2-dodécyl-α-phényl-N-(2,4,6-triméthoxyphényl)-2H-tétrazole-5-actamide [α]_{D} = -58° (1% dans CH₃OH),
(±) -2-2-dodécyl-α-phényl-N-(2,4,6-triméthoxyphényl)-2H-tétrazole-5-acétamide [α]_{D}= +55,1° (1% dans CH₃OH),
(±)-N-[2,6-bis(1-méthyléthyl)phényl]-2-dodécyl-α-fluoro-α-phényl-2H-tétrazole-5-acétamide, ou
(±) -2-dodécyl-α-fluoro-α-phényl-N-(2,4,6-triméthoxy phényl)-2H-tétrazole-5-acétamide.

30. Un composé selon la revendication 1, dans lequel R₄ est lié à l'atome de carbone du cycle tétrazole et la chaîne latérale est en position 2 sur le cycle trétrazole.

31. Un composé selon la revendication 30, qui correspond au:
N-[2,6-bis(1-méthmyléthyl)phényl]-5-décyl-2H-tétrazole-2-acétamide;
N-[2,6-bis(1-méthyléthyl)phényl-5-dodécyl-2H-tétrazole-2-acétamide;
(±)-N-[2,6-bis(1-méthyléthyl)phényl]-5-dodécyl-α-phényl-2H-tétrazole-2-acétamide;
(±)-N-[2,6-bis(1-méthyléthyl)phényl]-5-dodécyl-α-pentyl-2H-tétrazole-5-acétamide;
(±)-N-[2,6-bis(1-méthyléthyl)phényl-5-(dodécylthio)-α-phényl-2H-tétrazole-2-acétamide;
(±)-5-décyl-α-phényl-N-(2,4,6-triméthoxyphényl-2H-tétrazole-2-acétamide; 5-dodécyl-N-(2,4,6-triméthoxy-phényl)-2H-tétrazole-2-acétamide;
(±)-5-dodécyl-α-phényl-N-(2,4,6-triméthoxyphényl)-2H-tétrazole-2-acétamide;
(±)-5-dodécyl-α-pentyl-N-(2,4,6-triméthoxyphényl)-2H-tétrazole-2-acétamide;
(±)-N-(2,4-difluorophényl-5-dodécyl-α-phényl-2H-tétrazole-2-acétamide;
5-dodécyl-α,α-diméthyl-N-(2,4,6-triméthoxyphényl)-2H-tétrazole-2-acétamide;
(±)-5-(dodécylthio)-α-phényl-N-(2,4,6-triméthoxyphényl)-2H-tétrazole-2-acétamide; ou
(±)-5-(dodécylsulfinyl)-α-phényl-N-(2,4,6-triméthoxyphényl)-2H-tétrazole-2-acétamide.

32. Une composition pharmaceutique comprenant un composé selon l'une quelconque des revendications 1 à 31 et un adjuvant, diluant ou support solide ou liquide pharmaceutiquement acceptable.

33. Utilisation d'un composé selon l'une quelconque des revendications 1 à 31 pour la préparation de compositions pharmarceutiques agissant en tant qu'inhibiteurs de l'acy-CoA: chlolesterol acyltransférase (ACAT) diminuant l'absorption du cholestérol alimentaire et fournissant une thérapie pour l'hypercholestérolémie.

34. Un procédé de préparation d'un composé de formule I: 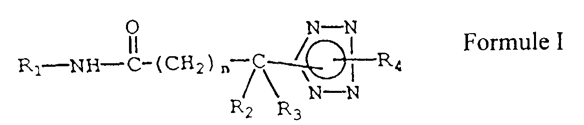 dans laquelle:
n est égal à 0, et
R₂ et R₃ représentent des atomes d'hydrogène et
R₁ et R₄ sont tels que définis selon la formule I de la revendication 1, caractérisé par les voies de synthèse suivantes:

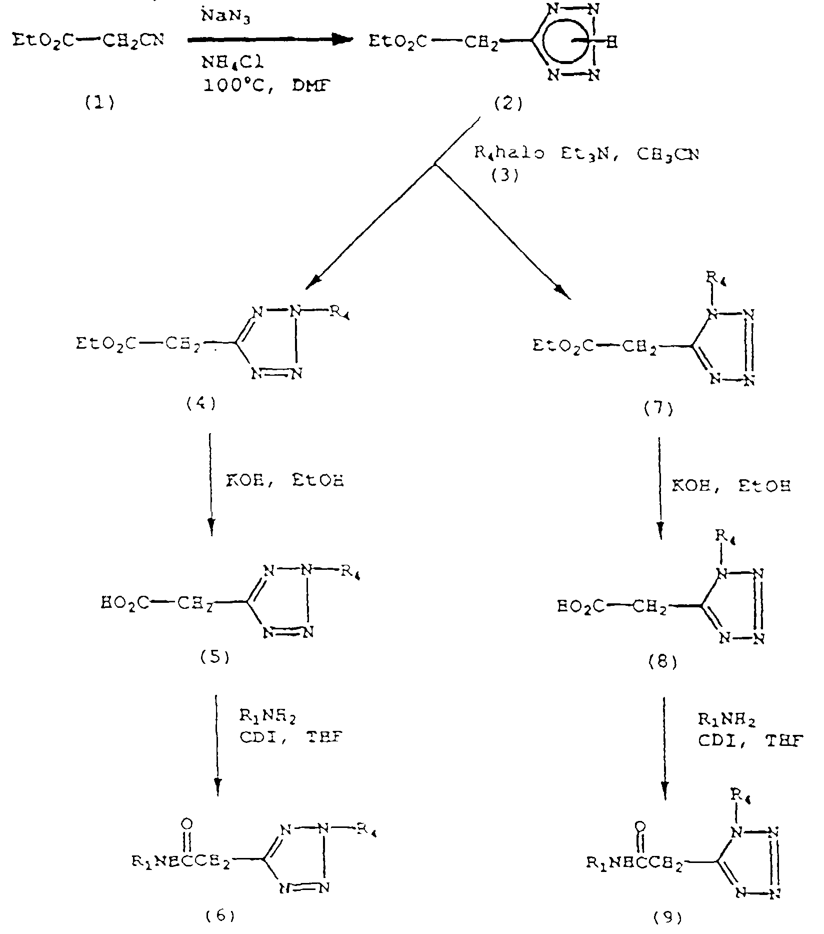

35. Un procédé pour la préparation d'un composé de formule I: 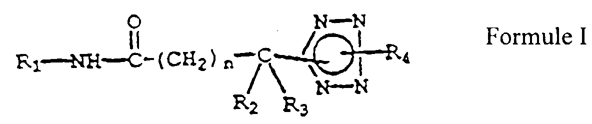 dans laquelle:
n est égal à zéro,
R₁, R₂, R₃ et R₄ sont tels que définis dans la formule I de la revendication 1, caractérisé par les voies de synthèse suivantes:

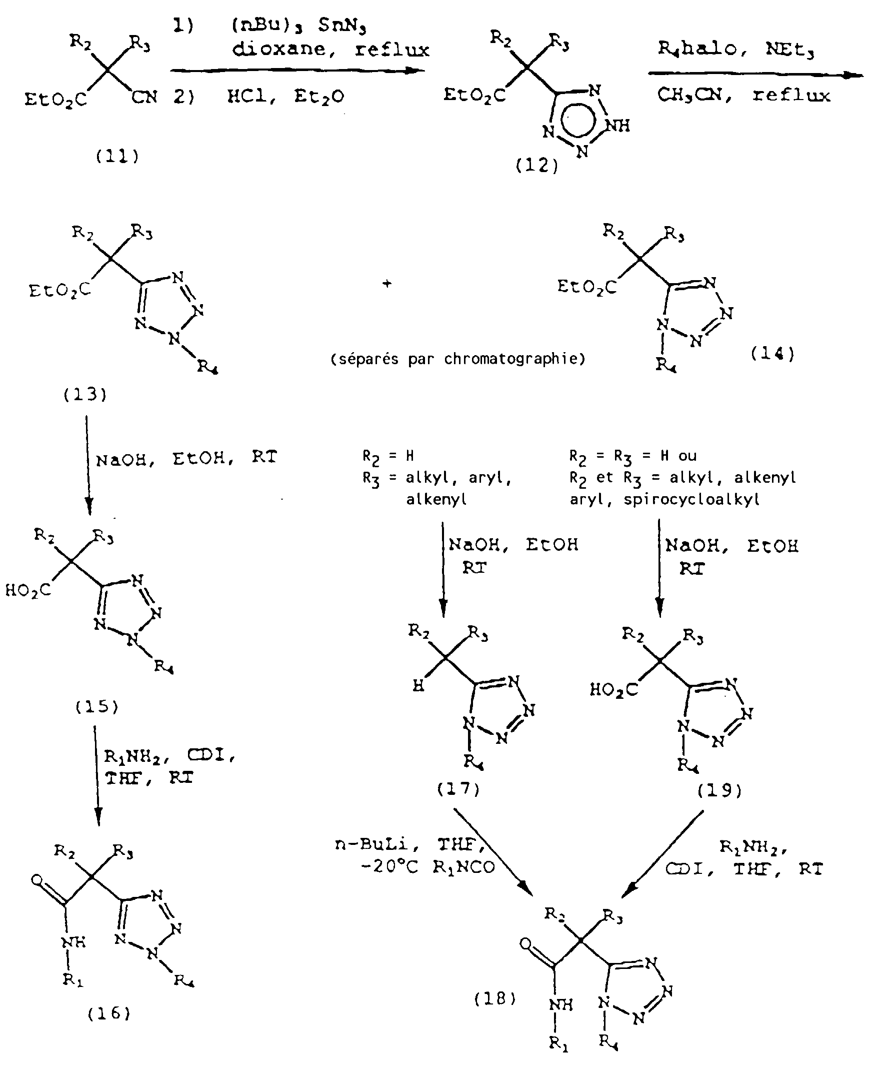

36. Un procédé pour la préparation d'un composé de formule I: 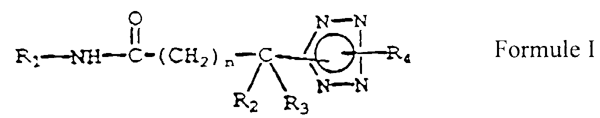 dans laquelle:
n est égal à un ou deux,
R₂ et R₃ représentent des atomes hydrogènes, et
R₁ et R₄ sont tels que définis à la formule I de la revendication 1, caractérisé par les voies de synthèse suivantes:

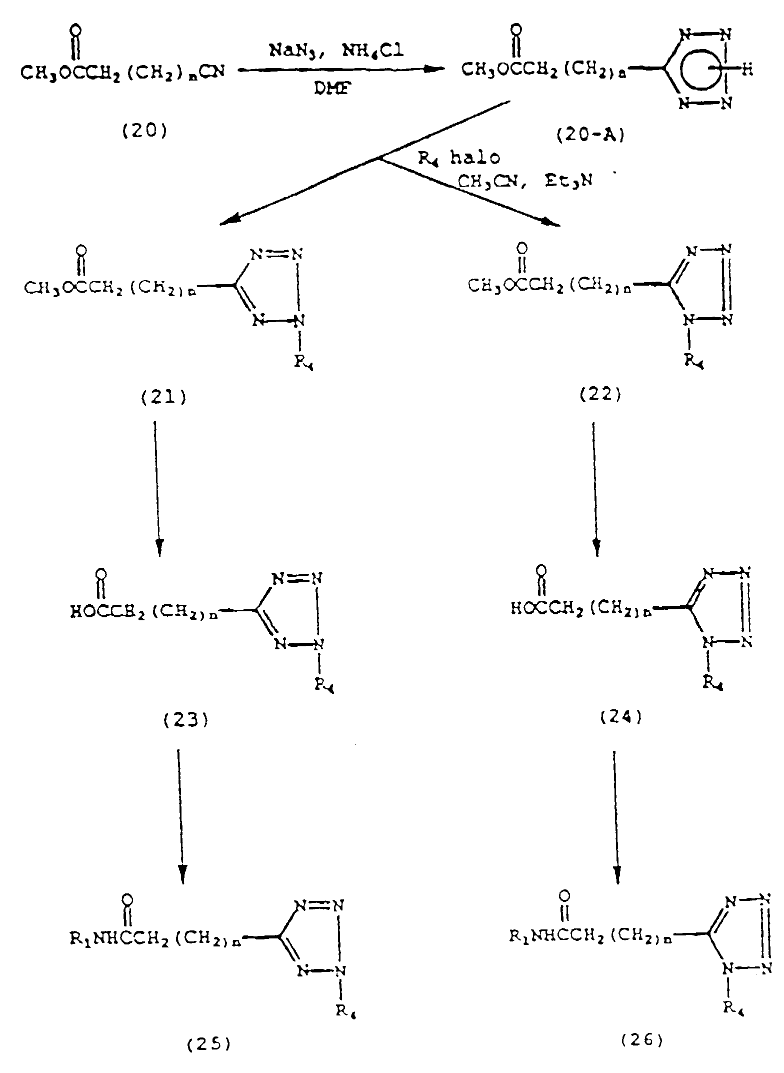

37. Un procédé de préparation d'un composé de formule I, selon la revendication 1: 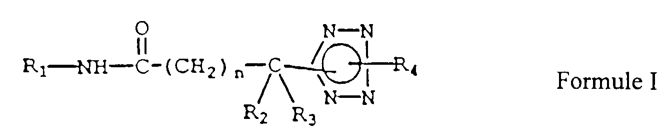 dans lequel:
n est égal à 1,
R₂ est un atome d'hydrogène et R3(x) est un radical phényl, phényl substitué, alkyl, alkényl ou hétéroaryl, caractérisé par les voies de synthèse suivantes:

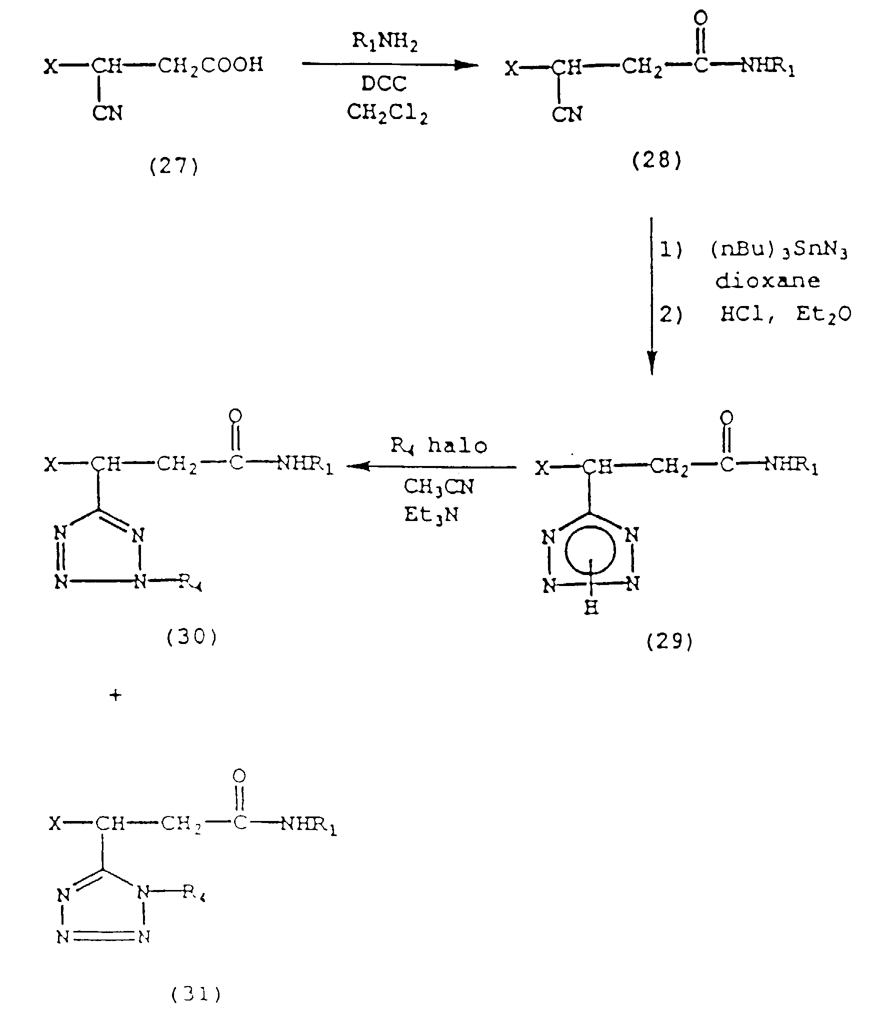

38. Un procédé de préparation d'un composé de formule I, selon la revendication 1: 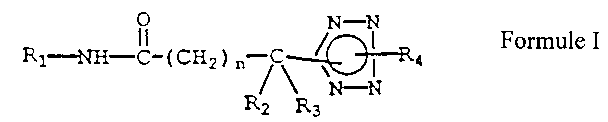 dans laquelle:
n est zéro, et
R₂ et R₃ représentent des radicaux alkyl ou aryl, caractérisé par les voies de synthèse suivantes:

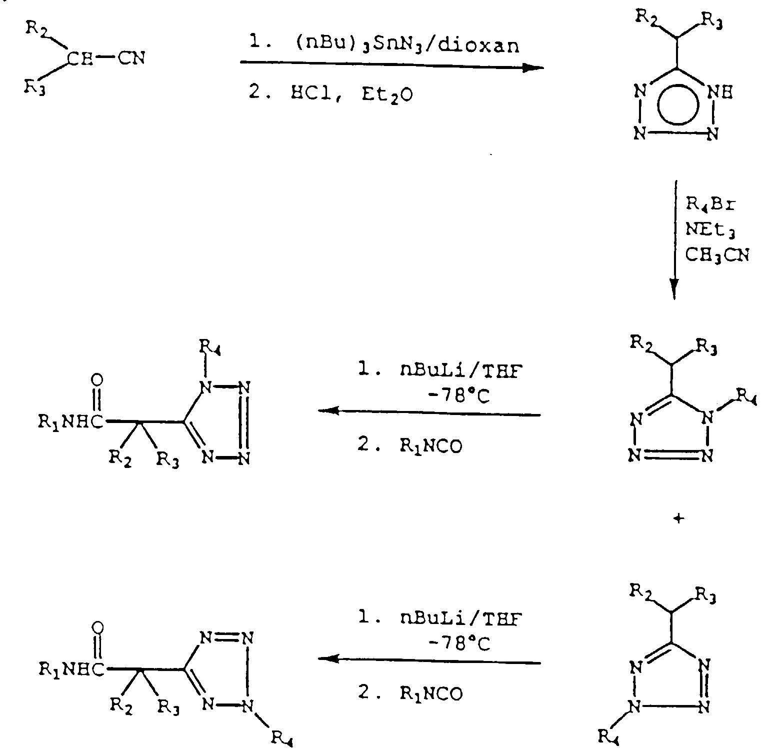

39. Un procédé pour la préparation d'un composé de formule I, selon la revendication 1: 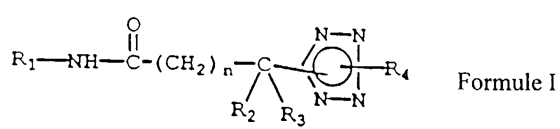 dans laquelle:
n est égal à 2,
R₂ est un atome d'hydrogène. et
R3 représente un radical phényl ou phényl substitué, caractérisé par les voies de synthèse suivantes:

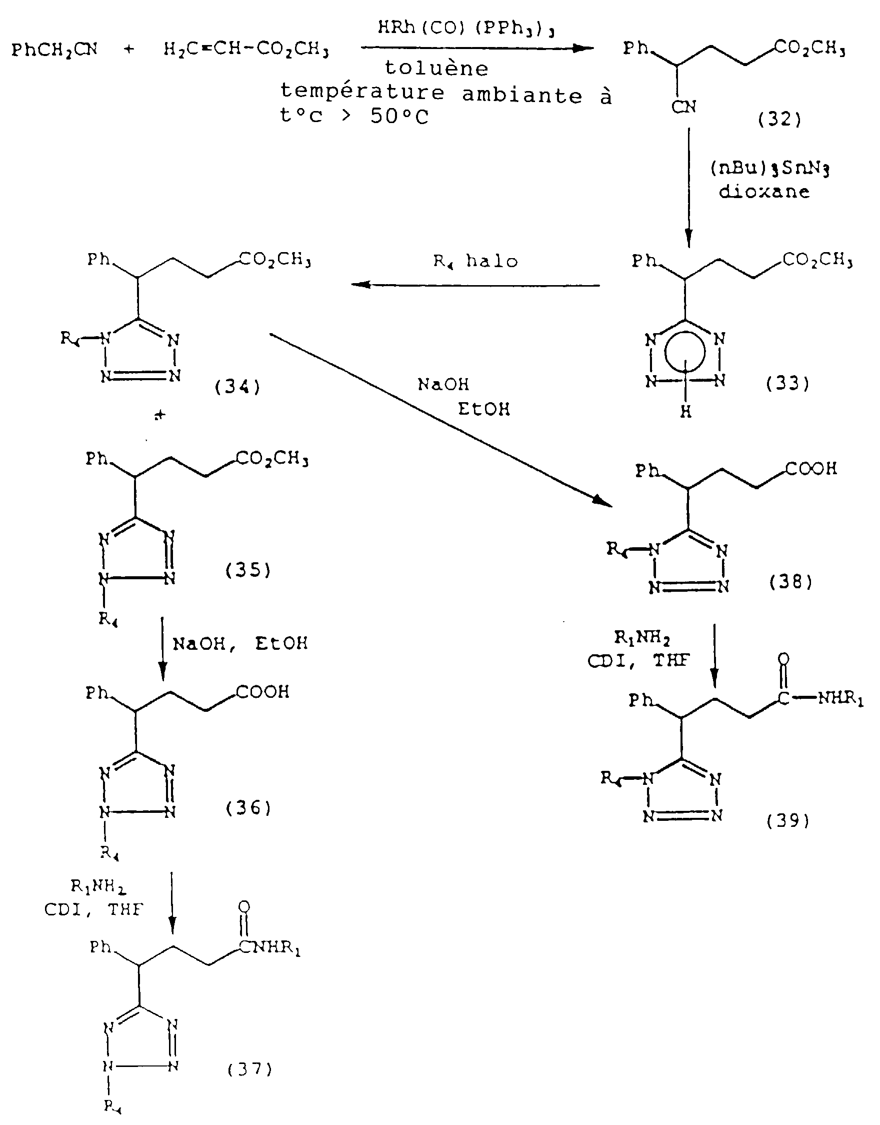

40. Un procédé pour la préparation d'un composé de formule I, selon la revendication 1: 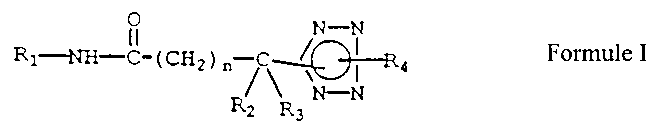 dans laquelle:
n est égal à zéro,
R₂ représente un atome d'hydrogène, et
R₃ est hétéroarayl, 1- ou 2-naphthyl ou phényl substitué, caractérisé par les voies de synthèse suivantes:

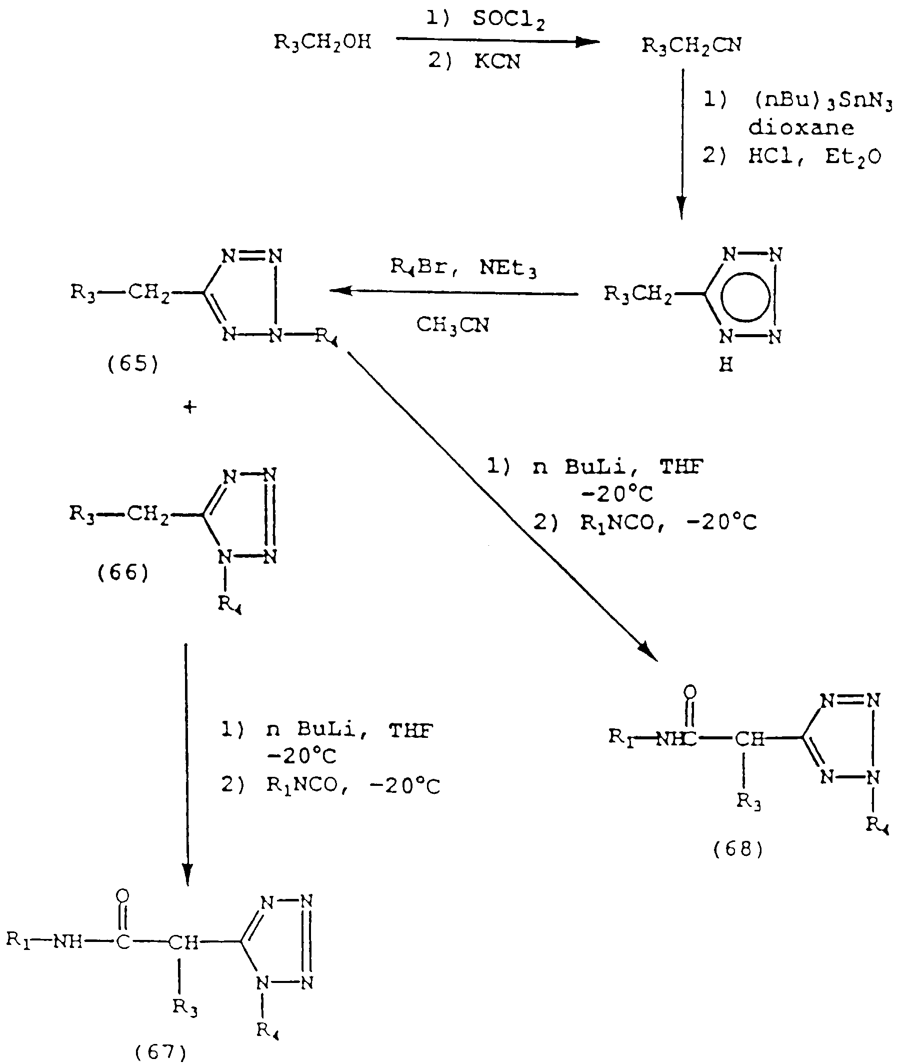

41. Un procédé de préparation d'un composé de formule I, selon la revendication 1: 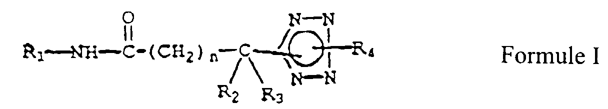 dans laquelle:
n est égal à un, caractérisé par les voies de synthèse suivantes:

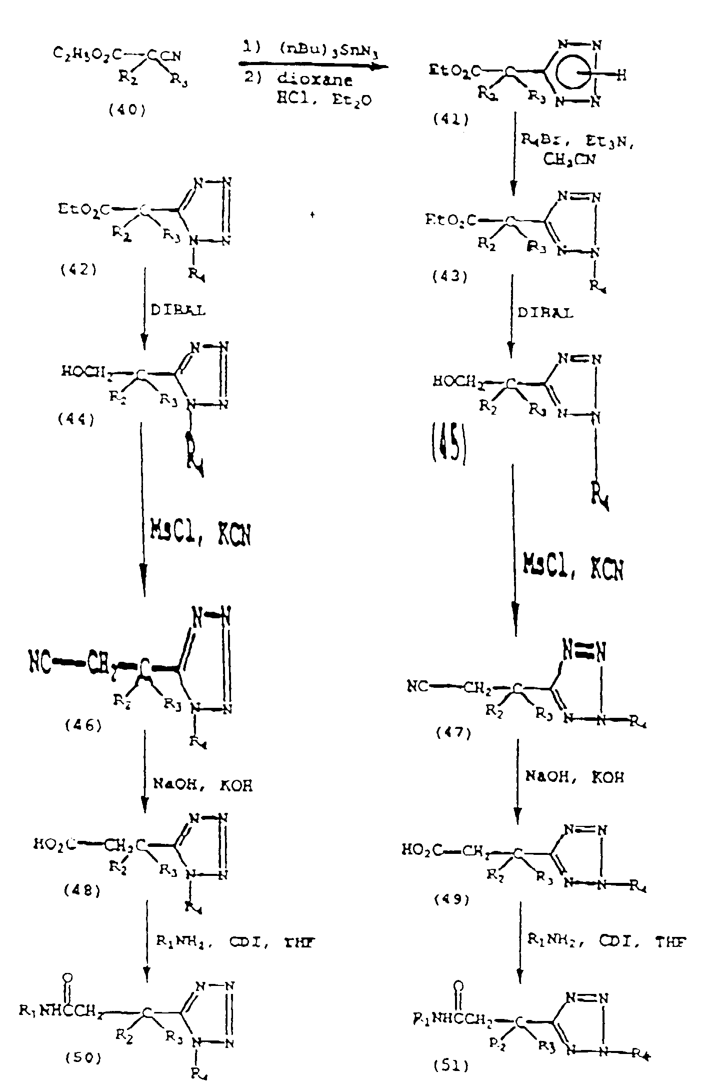

42. Un procédé pour la préparation d'un composé de formule I, selon la revendication 1: 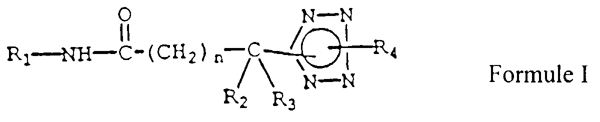 dans laquelle:
n est égal à un, et
R₃ est un groupe hétéroaryl, caractérisé par les voies de synthèse suivantes:

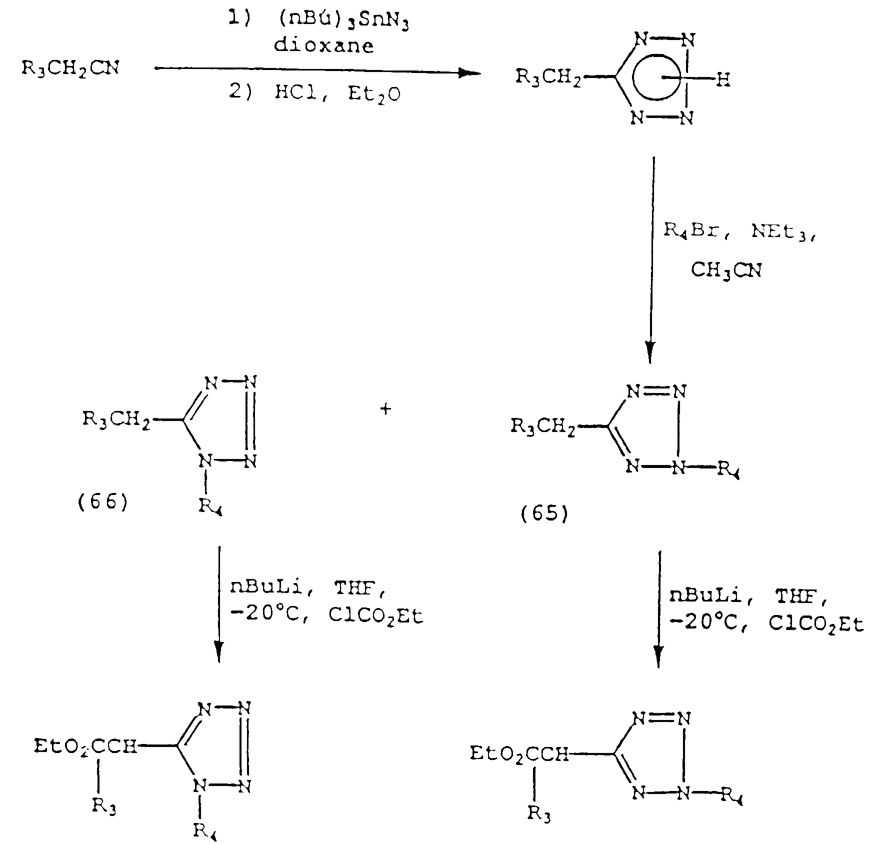

43. Un procédé pour la préparation d'un composé de formule I, selon la revendication 1: 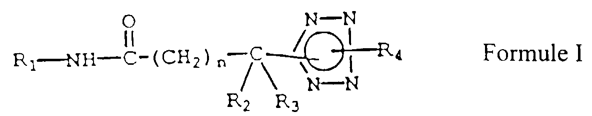 dans laquelle:
n est égal à deux, à condition qu'au moins l'un des substituants R₂ ou R₃ soit différent d'un atome d'hydrogène, caractérisé par les voies de synthèse suiantes:

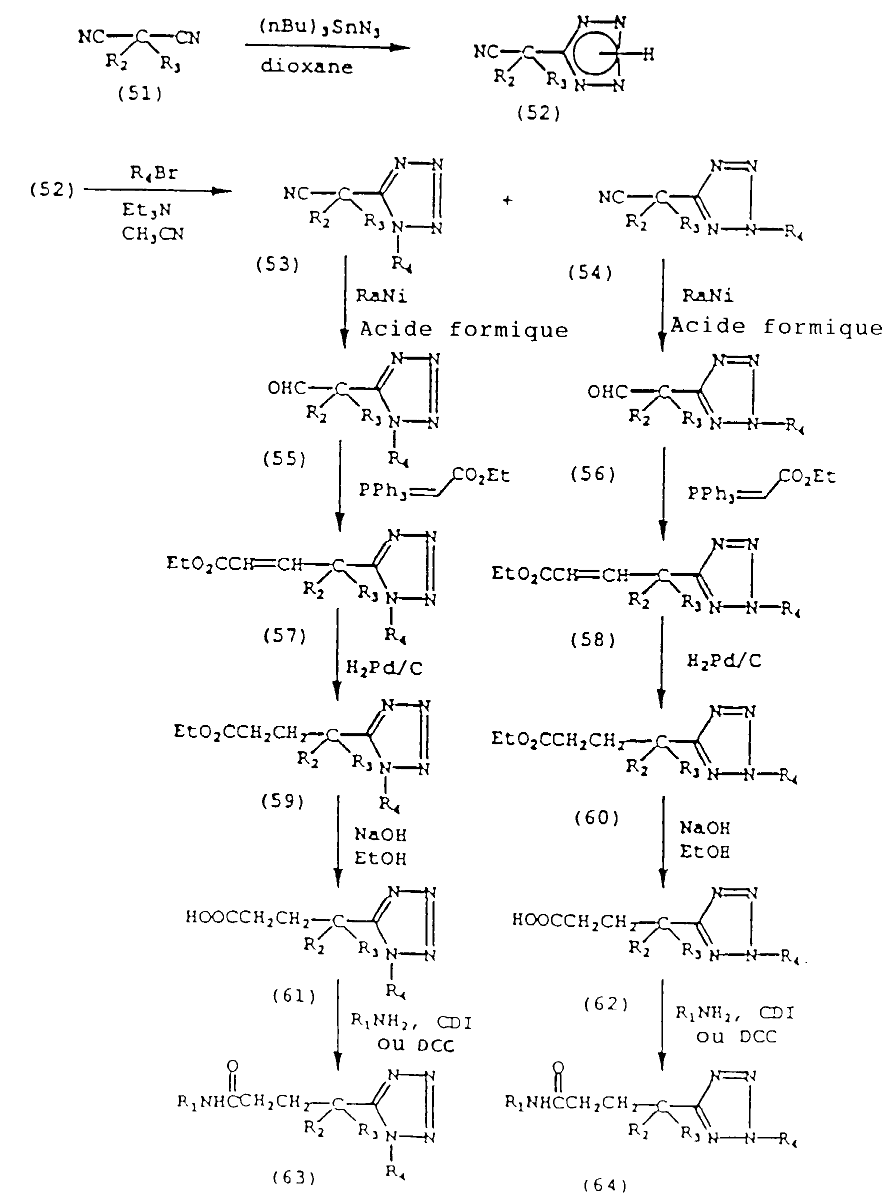

44. Un procédé pour la préparation d'un composé de formule I: 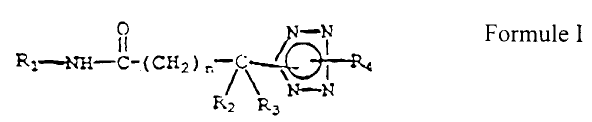 dans laquelle:
n est égal à zéro,
R₃ est F ou OH, caractérisé par les voies de synthèse suivantes:

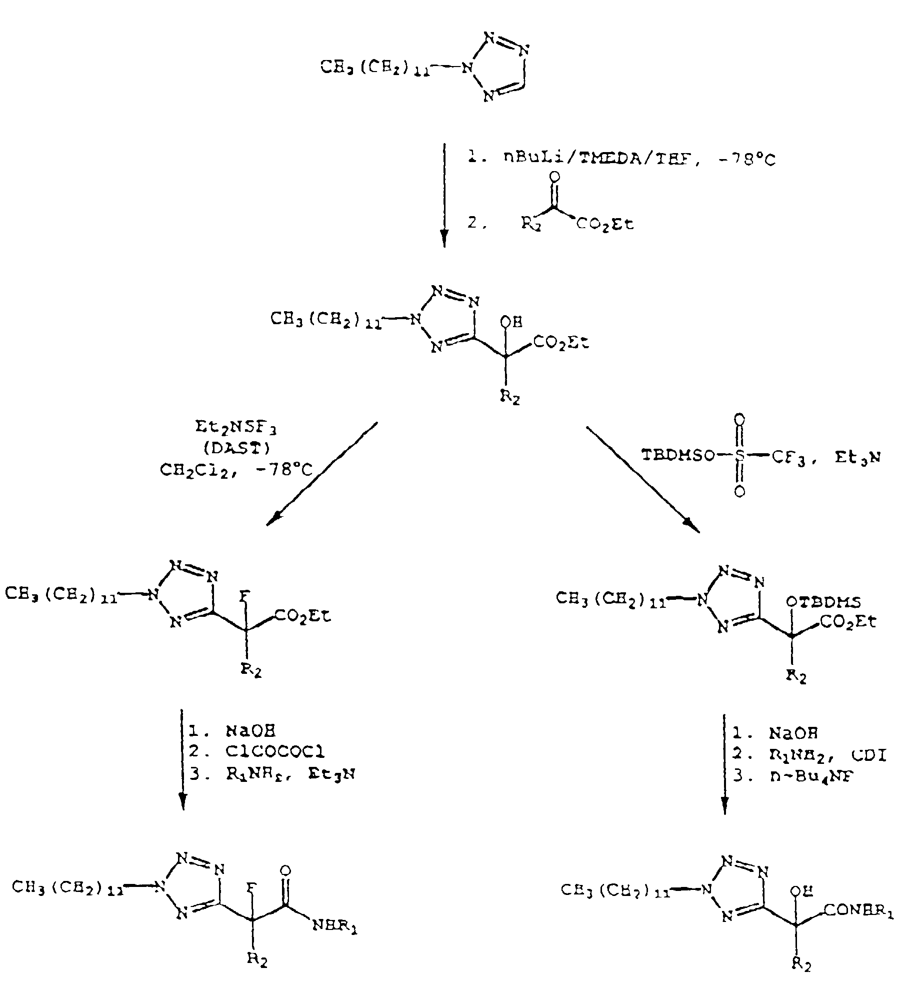

45. Un procédé pour la préparation d'une composition pharmaceutique caractérisé par la combinaison d'un composé fabriqué selon l'une quelconque des revendications 34 à 43, avec un adjuvant, diluant ou support solide ou liquide pharmaceutiquement acceptable.
